# EUROPEAN PATENT APPLICATION

(11) **EP 2 093 292 A2**
(43) Date of publication of application: **26.08.2009**
(21) Application number: 09151315.0
(22) Date of filing: 26.03.2001
(51) Int. Cl.: C12N 15/11, A61K 31/7125, C07H 21/04, C12Q 1/44, A61P 35/00

(54) **Inhibition of specific histone deacetylase isoforms**

(30) Priority: 24.03.2000 US 192157 P
(62) Divisional of application: 01274226.8
(71) Applicant: Methylgene, Inc., Saint-Laurent, Quebec H4S 2A1 (CA)
(72) Inventor: Li, Zuomei, Kirkland H9H 3X3 (CA); Bonfils, Claire, Montréal Québec (CA); Besterman, Jeffrey, Baie d'Urfe H9X 3V3 (CA)
(74) Representative: Fiorucci, Hélène

(57) **Abstract**

This invention relates to the inhibition of histone deacetylase expression and enzymatic activity. The invention provides methods and reagents for inhibiting specific histone deacetylase (HDAC) isoforms by inhibiting expression at the nucleic acid level or enzymatic activity at the protein level.

SEQUENCE LISTING

<110> Li, Zuomei
      Bonfils, Claire
      Besterman, Jeffrey

<120> Inhibition of Specific Histone Deacetylase Isoforms

<130> 9351-62PCT FC/ntb

<140> US 09/817,913
<141> 2001-03-26

<150> US 60/192,157
<151> 2000-03-24

<160> 33

<170> PatentIn version 3.1

<210> 1
<211> 481
<212> PRT
<213> Human

<400> 1

Met Ala Gln Thr Gln Gly Thr Arg Arg Lys Val Cys Tyr Tyr Tyr Asp
1               5                   10                  15

Gly Asp Val Gly Asn Tyr Tyr Tyr Gly Gln Gly His Pro Met Lys Pro
            20                  25                  30

His Arg Ile Arg Met Thr His Asn Leu Leu Leu Asn Tyr Gly Leu Tyr
        35                  40                  45

Arg Lys Met Glu Ile Tyr Arg Pro His Lys Ala Asn Ala Glu Glu Met
    50                  55                  60

Thr Lys Tyr His Ser Asp Asp Tyr Ile Lys Phe Leu Arg Ser Ile Arg
65                  70                  75                  80

Pro Asp Asn Met Ser Glu Tyr Ser Lys Gln Met Gln Arg Phe Asn Val
                85                  90                  95

Gly Glu Asp Cys Pro Val Phe Asp Gly Leu Phe Glu Phe Cys Gln Leu
            100                 105                 110

Ser Thr Gly Gly Ser Val Ala Ser Ala Val Lys Leu Asn Lys Gln Gln
        115                 120                 125

Thr Asp Ile Ala Val Asn Trp Ala Gly Gly Leu His His Ala Lys Lys
    130                 135                 140

Ser Glu Ala Ser Gly Phe Cys Tyr Val Asn Asp Ile Val Leu Ala Ile
145                 150                 155                 160

Leu Glu Leu Leu Lys Tyr His Gln Arg Val Leu Tyr Ile Asp Ile Asp
                165                 170                 175

Ile His His Gly Asp Gly Val Glu Glu Ala Phe Tyr Thr Thr Asp Arg
            180                 185                 190

Val Met Thr Val Ser Phe His Lys Tyr Gly Glu Tyr Phe Pro Gly Thr
        195                 200                 205

Gly Asp Leu Arg Asp Ile Gly Ala Gly Lys Gly Lys Tyr Tyr Ala Val
    210                 215                 220

Tyr Pro Leu Arg Asp Gly Ile Asp Asp Glu Ser Tyr Glu Ala Ile Phe
225                 230                 235                 240

Lys Pro Val Met Ser Lys Val Met Glu Met Phe Gln Pro Ser Ala Val
                245                 250                 255

Val Leu Gln Cys Gly Ser Asp Ser Leu Ser Gly Asp Arg Leu Gly Cys
            260                 265                 270

Phe Asn Leu Thr Ile Lys Gly His Ala Lys Cys Val Glu Phe Val Lys
        275                 280                 285

Ser Phe Asn Leu Pro Met Leu Met Leu Gly Gly Gly Gly Tyr Thr Ile
    290                 295                 300

Arg Asn Val Ala Arg Cys Trp Thr Tyr Glu Thr Ala Val Ala Leu Asp
305                 310                 315                 320

Thr Glu Ile Pro Asn Glu Leu Pro Tyr Asn Asp Tyr Phe Glu Tyr Phe
                325                 330                 335

Gly Pro Asp Phe Lys Leu His Ile Ser Pro Ser Asn Met Thr Asn Gln
            340                 345                 350

Asn Thr Asn Glu Tyr Leu Glu Lys Ile Lys Gln Arg Leu Phe Glu Asn
        355                 360                 365

Leu Arg Met Leu Pro His Ala Pro Gly Val Gln Met Gln Ala Ile Pro
    370                 375                 380

Glu Asp Ala Ile Pro Glu Glu Ser Gly Asp Glu Asp Glu Asp Asp Pro
385                 390                 395                 400

Asp Lys Arg Ile Ser Ile Cys Ser Ser Asp Lys Arg Ile Ala Cys Glu
                405                 410                 415

Glu Glu Phe Ser Asp Ser Glu Glu Glu Gly Glu Gly Gly Arg Lys Asn
            420                 425                 430

Ser Ser Asn Phe Lys Lys Ala Lys Arg Val Lys Thr Glu Asp Glu Lys
        435                 440                 445

Glu Lys Asp Pro Glu Glu Lys Lys Glu Val Thr Glu Glu Glu Lys Thr
    450                 455                 460

Lys Glu Glu Lys Pro Glu Ala Lys Gly Val Lys Glu Glu Val Lys Leu
465                 470                 475                 480

Ala

<210>  2
<211>  1611
<212>  DNA
<213>  Human

<400>  2
atgtctgggg tctctgcccg ctggtgctgc tgtctcccac tcggtcatcc tgagaacaca      60

gcctgagcgr ctctgtcact cggggtagac cacgcgggga ggcgagcaag atggcgcaga     120

cgcagggcac ccggaggaaa gtctgttact actacgacgg ggatgttgga aattactatt     180

atggacaagg ccacccaatg aagcctcacc gaatccgcat gactcataat ttgctgctca     240

actatggtct ctaccgaaaa atggaaatct atcgccctca caaagccaat gctgaggaga     300

tgaccaagta ccacagcgat gactacatta aattcttgcg ctccatccgt ccagataaca 360

tgtcggagta cagcaagcag atgcagagat tcaacgttgg tgaggactgt ccagtattcg 420

atggcctgtt tgagttctgt cagttgtcta ctggtggttc tgtggcaagt gctgtgaaac 480

ttaataagca gcagacggac atcgccgtga attgggctgg gggcctgcac catgcaaaga 540

agtccgaggc atctggcttc tgttacgtca atgatatcgt cttggccatc ctggaactgc 600

taaagtatca ccagagggtg ctgtacattg acattgatat tcaccatggt gacggcgtgg 660

aagaggcctt ctacaccacg gaccgggtca tgactgtgtc ctttcataag tatggagagt 720

acttcccagg aactggggac ctacgggata ccggggctgg caaagacaag tattatgctg 780

ttaactaccc gctccgagac gggattgatg acgagtccta tgaggccatt ttcaagccgg 840

tcatgtccaa agtaatggag atgttccagc ctagtgcggt ggtcttacag tgtggctcag 900

actccctatc tggggatcgg ttaggttgct tcaatctatc tatcaaagga cacgccaagt 960

gtgtggaatt tgtcaagagc tttaacctgc ctatgctgat gctgggaggc ggtggttaca 1020

ccattcgtaa cgttgcccgg tgctggacat atgagacagc tgtggccctg gatacggaga 1080

tccctaatga gcttccatac aatgactact ttgaatactt tggaccagat ttcaagctcc 1140

acatcagtcc ttccaatatg actaaccaga acacgaatga gtacctggag aagatcaaac 1200

agcgactgtt tgagaacctt agaatgctgc cgcacgcacc tggggtccaa acgcaggcga 1260

ttcctgagga cgccatccct gaggagagtg gcgatgagga cgaagacgac cctgacaagc 1320

gcatctcgat ctgctcctct gacaaacgaa ttgcctgtga ggaagagttc tccgattctg 1380

aagaggaggg agaggggggc cgcaagaact cttccaactt caaaaaagcc aagagagtca 1440

aaacagagga tgaaaaagag aaagacccag aggagaagaa aggaatcacc gaagaggaga 1500

aaaccaagga ggagaagcca gaagccaaag gggtcaagga ggaggccaag ttggcctgaa 1560

tggacctctc cagctctggc ttcctgctga gtccctcacg tttctttccc c 1611

<210> 3
<211> 489
<212> PRT
<213> Human

<400> 3

Met Ala Tyr Ser Gln Gly Gly Gly Lys Lys Lys Cys Lys Val Cys Tyr
1               5                   10                  15

Tyr Tyr Asp Gly Asp Ile Gly Asn Tyr Tyr Tyr Gly Gln Gly His Pro
            20                  25                  30

Met Lys Pro His Arg Ile Arg Met Thr His Asn Leu Leu Leu Asn Tyr
        35                  40                  45

Gly Leu Tyr Arg Lys Met Glu Ile Tyr Arg Pro His Lys Ala Thr Ala
    50                  55                  60

Glu Glu Met Thr Lys Tyr His Ser Asp Glu Tyr Ile Lys Phe Leu Arg
65                  70                  75                  80

Ser Ile Arg Pro Asp Asn Met Ser Glu Tyr Ser Lys Gln Met His Ile
                85                  90                  95

Pro Phe Asn Val Gly Glu Asp Cys Pro Ala Phe Asp Gly Leu Phe Glu
            100                 105                 110

Phe Cys Gln Leu Ser Thr Gly Gly Ser Val Ala Gly Ala Val Lys Leu
        115                 120                 125

Asn Arg Gln Gln Thr Asp Met Ala Val Asn Trp Ala Gly Gly Leu His
    130                 135                 140

His Ala Lys Lys Tyr Glu Ala Ser Gly Phe Cys Tyr Val Asn Asp Ile
145                 150                 155                 160

Val Leu Ala Ile Leu Glu Leu Leu Lys Tyr His Gln Arg Val Leu Tyr
                165                 170                 175

Ile Asp Ile Asp Ile His His Arg Gly Asp Gly Val Glu Glu Ala Phe
            180                 185                 190

Tyr Thr Thr Asp Arg Val Met Thr Val Ser Phe Tyr Gly Glu Tyr Phe
        195                 200                 205

Pro Gly Thr Gly Asp Leu Arg Asp Ile Gly Ala Gly Lys Gly Lys Tyr
    210                 215                 220

Tyr Ala Val Asn Phe Pro Met Cys Asp Gly Ile Asp Asp Glu Ser Tyr
225                 230                 235                 240

Gly Gln Ile Phe Lys Pro Ile Ile Ser Lys Val Met Glu Met Tyr Gln
                245                 250                 255

Pro Ser Ala Val Val Leu Gln Cys Gly Ala Asp Ser Leu Ser Gly Asp
            260                 265                 270

Arg Leu Gly Cys Phe Asn Leu Thr Val Lys Gly His Ala Lys Cys Val
        275                 280                 285

Glu Val Val Lys Thr Phe Asn Leu Pro Leu Leu Met Leu Gly Gly Gly
    290                 295                 300

Gly Tyr Thr Ile Leu Arg Asn Val Ala Arg Cys Trp Thr Tyr Glu Thr
305                 310                 315                 320

Ala Val Ala Leu Asp Cys Glu Ile Pro Asn Glu Leu Pro Tyr Asn Asp
                325                 330                 335

Tyr Phe Glu Tyr Phe Gly Pro Asp Phe Lys Leu His Ile Ser Pro Ser
            340                 345                 350

Asn Met Thr Asn Gln Asn Thr Pro Glu Tyr Met Glu Lys Ile Lys Gln
        355                 360                 365

Arg Leu Phe Glu Asn Leu Arg Met Leu Pro His Ala Pro Gly Val Gln
    370                 375                 380

Met Gln Ala Ile Pro Glu Asp Ala Val His Glu Asp Ser Gly Asp Glu
385                 390                 395                 400

Asp Gly Glu Asp Pro Asp Lys Arg Ile Ser Ile Arg Ala Ser Asp Lys
                405                 410                 415

Arg Ile Ala Cys Asp Glu Glu Phe Ser Asp Ser Glu Asp Glu Gly Glu
            420                 425                 430

Gly Gly Arg Asn Val Ala Asp His Lys Lys Gly Ala Lys Ala Arg Ile
        435                 440                 445

Glu Glu Asp Lys Lys Glu Thr Glu Asp Lys Lys Thr Asp Val Lys Glu
    450                 455                 460

Glu Asp Lys Ser Lys Asp Asn Ser Gly Glu Lys Thr Asp Thr Lys Gly
465                 470                 475                 480

Thr Lys Ser Glu Gln Leu Ser Asn Pro
                485

<210> 4
<211> 1985
<212> DNA
<213> Human

<400> 4
cgccgagctt tcggcacctc tgccgggtgg taccgagcct tcccggcgcc ccctcctctc      60

ctcccaccgg cctgcccttc cccgcgggac tatcgccccc acgtttccct cagccctttt     120

ctctcccggc cgagccgcgg cggcagcagc agcagcagca gcagcaggag gaggagcccg     180

gtggcggcgg tggccgggga gcccatggcg tacagtcaag gaggcggcaa aaaaaaagtc     240

tgctactact acgacggtga tattggaaat tattattatg gacagggtca tcccatgaag     300

cctcatagaa tccgcatgac ccataacttg ctgttaaatt atggcttaca cagaaaaatg     360

gaaatatata ggccccataa agccactgcc gaagaaatga caaaatatca cagtgatgag     420

tatatcaaat ttctacggtc aataagacca gataacatgt ctgagtatag taagcagatg     480

catatattta atgttggaga agattgtcca gcgtttgatg gactctttga gttttgtcag     540

ctctcaactg gcggttcagt tgctggagct gtgaagttaa accgacaaca gactgatatg     600

gctgttaatt gggctggagg attacatcat gctaagaaat acgaagcatc aggatcctgt     660

tacgttaatg atattgtgct tgccatcctt gaattactaa agtatcatca gagagtctta     720

tatatcgata tagatattca tcatggtgat ggtgtcgaag aagcttttta tacaacagat     780

cgtgtaatga cggtatcatt ccataaatat ggggaatact ttcctggcac aggagacttg     840

agggatattg gtgctggaaa aggcaaatac tatgctgtca attttccaat gtgtgatggt     900

atagacgatg agtcatatgg gcagatattt aagcctatta tctcaaaggt gatggagatg     960

tatcaaccta gtgctgtggt attacagtgt ggtgcagact cattatctgg tgatagactg    1020

ggttgtttca atctaacagt caaaggtcat gctaaatgtg tagaagttgt aaaaactttt    1080

aacttaccat tactgatgct tggaggaggt ggctacacaa tccgtaatgt tgctcgatgt    1140

tggacatatg agactgcagt tgcccttgat tgtgagattc ccaacgagtt gccatataat    1200

gattactttg agtattttgg accagacttc aaactgcata ttagtccttc aaacatgaca    1260

aaccagaaca ctccagaata tacggaaaag ataaaacagc gtttgtttga aaatttgcgc    1320

atgttacctc atgcacctgg tgtccagatg caagctattc cagaagatgc tgttcatgaa 1380

gacagtggag atgaagatgg agaagatcca gacaagagaa tttctattcg agcatcagac 1440

aagcggatag cttgtgatga agaattctca gattctgagg atgaaggaga aggaggtcga 1500

agaaatgtgg ctgatcataa gaaaggagca aagaaagcta gaattgaaga agataagaaa 1560

gaaacagagg acaaaaaaac agacgttaag gaagaagata aatccaagga caacagtggt 1620

gaaaaaacag ataccaaagg aaccaaatca gaacagctca gcaacccctg aatctgacag 1680

tctcaccaat ttcagaaaat cattaaaaag aaaatattga aaggaaaatg ttttcttttt 1740

gaagacttct ggcttcattt tatactactt tggcatggac tgtatttatt ttcaaatggg 1800

actttttcgt ttttgttttt ctgggcaagt tttattgtga gattttctaa ttatgaagca 1860

aaatttcttt tctccaccat gctttatgtg atagtattta aaattgatgt gagttattat 1920

gtcaaaaaaa ctgatctatt aaagaagtaa ttggcctttc tgagctgaaa aaaaaaaaaa 1980

aaaag 1985

<210> 5
<211> 428
<212> PRT
<213> Human

<400> 5

Met Ala Lys Thr Val Ala Tyr Phe Tyr Asp Pro Asp Val Gly Asn Phe
1               5                   10                  15

His Tyr Gly Ala Gly His Pro Met Lys Pro His Arg Leu Ala Leu Thr
            20                  25                  30

His Ser Leu Val Leu His Tyr Gly Leu Tyr Lys Lys Met Ile Val Phe
        35                  40                  45

Lys Pro Tyr Gln Ala Ser Gln His Asp Met Cys Arg Phe His Ser Glu
    50                  55                  60

Asp Tyr Ile Asp Phe Leu Gln Arg Val Ser Pro Thr Asn Met Gln Gly
65                  70                  75                  80

Phe Thr Lys Ser Leu Asn Ala Pro Asn Val Gly Asp Asp Cys Pro Val
                85                  90                  95

Phe Pro Gly Leu Phe Glu Phe Cys Ser Arg Tyr Thr Gly Ala Ser Leu
            100                 105                 110

Gln Gly Ala Thr Gln Leu Asn Asn Lys Ile Cys Asp Ile Ala Asn Trp
        115                 120                 125

Ala Gly Gly Leu His His Ala Lys Lys Phe Glu Ala Ser Gly Phe Cys
    130                 135                 140

Tyr Val Asn Asp Ile Val Ile Gly Ile Leu Glu Leu Leu Leu Lys Tyr
145                 150                 155                 160

His Pro Arg Val Leu Tyr Ile Asp Ile Asp Ile His His Gly Asp Gly
                165                 170                 175

Val Gln Glu Ala Phe Tyr Leu Thr Asp Arg Val Met Thr Val Ser Phe
            180                 185                 190

His Lys Tyr Gly Asn Tyr Phe Phe Pro Gly Thr Gly Asp Met Tyr Glu
        195                 200                 205

Val Gly Ala Glu Ser Gly Arg Tyr Tyr Cys Leu Asn Val Pro Leu Arg
    210                 215                 220

Asp Gly Ile Asp Asp Gln Ser Tyr Lys His Leu Phe Gln Pro Val Ile
225                 230                 235                 240

Asn Gln Val Val Asp Phe Tyr Gln Pro Thr Cys Ile Val Leu Gln Cys
                245                 250                 255

Gly Ala Asp Ser Leu Gly Cys Asp Arg Leu Gly Cys Phe Asn Leu Ser
            260                 265                 270

Ile Arg Gly His Cys Glu Cys Val Glu Tyr Val Lys Ser Phe Asn Ile
        275                 280                 285

Pro Pro Leu Leu Val Leu Gly Gly Gly Gly Tyr Thr Val Arg Asn Val
    290                 295                 300

Ala Arg Cys Trp Thr Tyr Glu Thr Ser Leu Leu Val Glu Glu Ala Ile
305                 310                 315                 320

Ser Glu Glu Leu Pro Tyr Ser Glu Tyr Phe Glu Tyr Phe Ala Pro Asp
                325                 330                 335

Phe Thr Leu His Pro Asp Val Ser Thr Arg Ile Glu Asn Gln Ser Arg
            340                 345                 350

Gln Tyr Leu Asp Gln Ile Arg Gln Thr Ile Phe Glu Asn Leu Lys Met
        355                 360                 365

Leu Asn His Ala Pro Ser Val Gln Ile His Asp Val Pro Ala Asp Leu
    370                 375                 380

Leu Thr Tyr Asp Arg Thr Asp Glu Ala Asp Ala Glu Glu Arg Gly Pro
385                 390                 395                 400

Glu Glu Asn Tyr Ser Arg Pro Glu Ala Pro Asn Glu Phe Tyr Asp Gly
                405                 410                 415

Asp His Asp Asn Asp Lys Glu Ser Asp Val Glu Ile
            420                 425

<210>  6
<211>  1954
<212>  DNA
<213>  Human

<400>  6
ggaattcgcg gccgcggcgg gcgcgggagg tgcggggcct gctcccgccg gcaccatggc      60

caagaccgtg gcctatttct acgaccccga cgtgggcaac ttccactacg gagctggaca     120

ccctatgaag ccccatcgcc tggcattgac ccatagcctg gtcctgcatt acggtctcta     180

taagaagatg atcgtcctca agccatacca ggcctcccaa catgacatgt gccgcttcca     240

ctccgaggac tacattgact tcctgcagag agtcagcccc accaatatgc aaggcttcac     300

caagagtctt aatgccttca acgtaggcga tgactgccca gtgtttcccg ggctctttga     360

gttctgctcg cgttacacag gcgcatctct gcaaggagca acccagctga acaacaagat     420

ctgtgatatt gccattaact gggctggtgg tctgcaccat gccaagaagt ttgaggcctc     480

tggcttctgc tatgtcaacg acattgtgat tggcatcctg gagctgctca agtaccaccc     540

tcgggtgctc tacattgaca ttgacatcca ccatggtgac ggggttcaag aagctttcta     600

cctcactgac cgggtcatga cggtgtcctt ccacaaatac ggaaattact tcttccctgg     660

cacaggtgac atgtatgaag tcggggcaga gagtggccgc tactactgtc tgaacgtgcc 720

cctgcgggat ggcattgatg accagagtta caagcacctt ttccagccgg ttatcaacca 780

ggtagtggac ttctaccaac ccacgtgcat tgtgctccag tgtggagctg actctctggg 840

ctgtgatcga ttgggctgct ttaacctcag catccgaggg catggggaat gcgttgaata 900

tgtcaagagc ttcaatatcc ctctactcgt gctgggtggt ggtggttata ctgtccgaaa 960

tgttgcccgc tgctggacat atgagacatc gctgctggca gaagaggcca ttagtgagga 1020

gcttccctat agtgaatact tcgagtactt tgccccagac ttcacacttc atccagatgt 1080

cagcacccgc atcgagaatc agaactcacg ccagtatctg gaccagatcc gccagacaat 1140

ctttgaaaac ctgaagatgc tgaaccatgc acctagtgtc cagattcatg acgtgcctgc 1200

agacctcctg acctacgaca ggaccgatga ggccgatgca gaggagaggg gtcctgagga 1260

gaactatagc aggccagagg catccaatga gttctatgat ggagaccatg acaatgacaa 1320

ggaaagcgat gtggagattt aagagtggct tgggatgctg tgtcccaagg aatttctttt 1380

cacctcttgg aagggctgga gggaaaagga gtggctccta gagtcctggg ggtcacccca 1440

ggggcttttg ctgactctgg gaaagagtct ggagaccaca tttggttctc gaaccatcta 1500

cctgcttttc ctctctctcc caaggactga caatggtacc tattagggat gagatacaga 1560

caaggatagc tatctgggac attattggca gtgggccctg gaggcagtcc ctagcccccc 1620

ttgcccctta tttcttccct gcttccctcg aacccagaga tttttgaggg atgaacgggt 1680

agacaaggac tgagattgcc tctgacttcc tcctcccctg ggttctgacc ttcttcctcc 1740

ccttgcttcc agggaagatg aagagagaga gatttggaag ggctctggc tccctaacac 1800

ctgaatccca gatgatggga agtatgtttt caagtgtggg gaggatatga aaatgttctg 1860

ctctcacttt tggctttatg tccattttac cactgttttt atccaataaa ctaagtcggt 1920

atttttgta cctttgatgg tttagcggcc gcgc 1954

<210> 7
<211> 967
<212> PRT
<213> Human

<400> 7

Met Leu Ala Met Lys His Gln Gln Glu Leu Leu Glu His Gln Arg Lys
1               5                   10                  15

Leu Glu Arg His Arg Gln Glu Gln Glu Leu Glu Lys Gln His Arg Glu
            20                  25                  30

Gln Lys Leu Gln Gln Leu Lys Asn Lys Glu Lys Gly Lys Glu Ser Ala
        35                  40                  45

Val Ala Ser Thr Glu Val Lys Met Lys Leu Gln Glu Phe Val Leu Asn
    50                  55                  60

Lys Lys Lys Ala Leu Ala His Pro Asn Leu Asn His Cys Ile Ser Ser
65                  70                  75                  80

Cys Pro Arg Tyr Trp Tyr Gly Lys Thr Gln His Ser Ser Leu Asp Gln
                85                  90                  95

Ser Ser Pro Pro Gln Ser Gly Val Ser Thr Ser Tyr Asn His Pro Val
            100                 105                 110

Leu Gly Met Tyr Asp Ala Lys Asp Asp Phe Pro Leu Arg Lys Thr Ala
        115                 120                 125

Ser Glu Pro Asn Leu Lys Leu Arg Ser Arg Leu Lys Gln Lys Val Ala
    130                 135                 140

Glu Arg Arg Ser Ser Pro Leu Leu Arg Arg Lys Asp Gly Pro Val Val
145                 150                 155                 160

Thr Ala Leu Lys Lys Arg Pro Leu Asp Val Thr Asp Ser Ala Cys Ser
                165                 170                 175

Ser Ala Pro Gly Ser Gly Pro Ser Ser Pro Asn Asn Ser Ser Gly Ser
            180                 185                 190

Val Ser Ala Glu Asn Gly Ile Ala Pro Ala Val Pro Ser Ile Pro Ala
        195                 200                 205

Glu Thr Ser Leu Ala His Arg Leu Val Ala Arg Glu Gly Ser Ala Ala
    210                 215                 220

Pro Leu Pro Leu Tyr Thr Ser Pro Ser Leu Pro Asn Ile Thr Leu Gly
225                 230                 235                 240

Leu Pro Ala Thr Gly Pro Ser Ala Gly Thr Ala Gly Gln Gln Asp Thr
                245                 250                 255

Glu Arg Leu Thr Leu Pro Ala Leu Gln Gln Arg Leu Ser Leu Phe Pro
            260                 265                 270

Gly Thr His Leu Thr Pro Tyr Leu Ser Thr Ser Pro Leu Glu Arg Asp
        275                 280                 285

Gly Gly Ala Ala His Ser Pro Leu Leu Gln His Met Val Leu Leu Glu
    290                 295                 300

Gln Pro Pro Ala Gln Ala Pro Leu Val Thr Gly Leu Gly Ala Leu Pro
305                 310                 315                 320

Leu His Ala Gln Ser Leu Val Gly Ala Asp Arg Val Ser Pro Ser Ile
                325                 330                 335

His Lys Leu Arg Gln His Arg Pro Leu Gly Arg Thr Gln Ser Ala Pro
            340                 345                 350

Leu Pro Gln Asn Ala Gln Ala Leu Gln His Leu Val Ile Gln Gln Gln
        355                 360                 365

His Gln Gln Phe Leu Glu Lys His Lys Gln Gln Phe Gln Gln Gln Gln
    370                 375                 380

Leu Gln Met Asn Lys Ile Ile Pro Lys Pro Ser Glu Pro Ala Arg Gln
385                 390                 395                 400

Pro Glu Ser His Pro Glu Glu Thr Glu Glu Glu Leu Arg Glu His Gln
                405                 410                 415

Ala Leu Leu Asp Glu Pro Tyr Leu Asp Arg Leu Pro Gly Gln Lys Glu
            420                 425                 430

Ala His Ala Gln Ala Gly Val Gln Val Lys Gln Glu Pro Ile Glu Ser
        435                 440                 445

Asp Glu Glu Glu Ala Glu Pro Pro Arg Glu Val Glu Pro Gly Gln Arg
    450                 455                 460

Gln Pro Ser Glu Gln Glu Leu Leu Phe Arg Gln Gln Ala Leu Leu Leu
465                 470                 475                 480

Glu Gln Gln Arg Ile His Gln Leu Arg Asn Tyr Gln Ala Ser Met Glu
                485                 490                 495

Ala Ala Gly Ile Pro Val Ser Phe Gly Gly His Arg Pro Leu Ser Arg
            500                 505                 510

Ala Gln Ser Ser Pro Ala Ser Ala Thr Phe Pro Val Ser Val Gln Glu
        515                 520                 525

Pro Pro Thr Lys Pro Arg Phe Thr Thr Gly Leu Val Tyr Asp Thr Leu
    530                 535                 540

Met Leu Lys His Gln Cys Thr Cys Gly Ser Ser Ser Ser His Pro Glu
545                 550                 555                 560

His Ala Gly Arg Ile Gln Ser Ile Trp Ser Arg Leu Gln Glu Thr Gly
                565                 570                 575

Leu Arg Gly Lys Cys Glu Cys Ile Arg Gly Arg Lys Ala Thr Leu Glu
            580                 585                 590

Glu Leu Gln Thr Val His Ser Glu Ala His Thr Leu Leu Tyr Gly Thr
        595                 600                 605

Asn Pro Leu Asn Arg Gln Lys Leu Asp Ser Lys Lys Leu Leu Gly Ser
    610                 615                 620

Leu Ala Ser Val Phe Val Arg Leu Pro Cys Gly Gly Val Gly Val Asp
625                 630                 635                 640

Ser Asp Thr Ile Trp Asn Glu Val His Ser Ala Gly Ala Ala Arg Leu
                645                 650                 655

Ala Val Gly Cys Val Val Glu Leu Val Phe Lys Val Ala Thr Gly Glu
            660                 665                 670

Leu Lys Asn Gly Phe Ala Val Val Arg Pro Pro Gly His His Ala Glu
        675                 680                 685

Glu Ser Thr Pro Met Gly Phe Cys Tyr Phe Asn Ser Val Ala Val Ala
    690                 695                 700

Ala Lys Leu Leu Gln Gln Arg Leu Ser Val Ser Lys Ile Leu Ile Val
705                 710                 715                 720

Asp Trp Asp Val His His Gly Asn Gly Thr Gln Gln Ala Phe Tyr Ser
                725                 730                 735

Asp Pro Ser Val Leu Tyr Met Ser Leu His Arg Tyr Asp Asp Gly Asn
            740                 745                 750

Phe Phe Pro Gly Ser Gly Ala Pro Asp Glu Val Gly Thr Gly Pro Gly
        755                 760                 765

Val Gly Phe Asn Val Asn Met Ala Phe Thr Gly Gly Leu Asp Pro Pro
    770                 775                 780

Met Gly Asp Ala Glu Tyr Leu Ala Ala Phe Arg Thr Val Val Met Pro
785                 790                 795                 800

Ile Ala Ser Glu Phe Ala Pro Asp Val Val Leu Ala Ser Ser Gly Phe
                805                 810                 815

Asp Ala Val Glu Gly His Pro Thr Pro Leu Gly Gly Tyr Asn Leu Ser
            820                 825                 830

Ala Arg Cys Phe Gly Tyr Leu Thr Lys Gln Leu Met Gly Leu Ala Gly
        835                 840                 845

Gly Arg Ile Val Leu Ala Leu Glu Gly Gly His Asp Leu Thr Ala Ile
    850                 855                 860

Cys Asp Ala Ser Glu Ala Cys Val Ser Ala Leu Leu Gly Asn Glu Leu
865                 870                 875                 880

Asp Pro Leu Pro Glu Lys Val Leu Gln Gln Arg Pro Asn Ala Asn Ala
                885                 890                 895

Val Arg Ser Met Glu Lys Val Met Glu Ile His Ser Lys Tyr Trp Arg
            900                 905                 910

Cys Leu Gln Arg Thr Thr Ser Thr Ala Gly Arg Ser Leu Ile Glu Ala
        915                 920                 925

Gln Thr Cys Glu Asn Glu Glu Ala Glu Thr Val Thr Ala Met Ala Ser
    930                 935                 940

Leu Ser Val Gly Val Lys Pro Ala Glu Lys Arg Pro Asp Glu Glu Pro
945                 950                 955                 960

Met Glu Glu Glu Pro Pro Leu
                965

<210>  8
<211>  8459
<212>  DNA
<213>  Human

<400>  8
ggaggttgtg gggccgccgc cgcggagcac cgtccccgcc gccgcccgag cccgagcccg      60

agcccgcgca cccgcccgcg ccgccgccgc cgccgcccga acagcctccc agcctgggcc     120

cccggcggcg ccgtggccgc gtcccggctg tcgccgcccg agcccgagcc cgcgcgccgg     180

cgggtggcgg cgcaggctga ggagatgcgg cgcggagcgc cggagcaggg ctagagccgg     240

ccgccgccgc ccgccgcggt aagcgcagcc ccggcccggc gcccgcgggc cattgtccgc     300

cgcccgcccc gcgccccgcg cagcctgcag gccttggagc ccgcggcagg tggacgccgc     360

cggtccacac ccgccccgcg cgcggccgtg ggaggcgggg gccagcgctg gccgcgcgcc     420

gtgggacccg ccggtcccca gggccgcccg gccccttctg gacctttcca cccgcgccgc     480

gaggcggctt cgcccgccgg ggcgggggcg cgggggtggg cacggcaggc agcggcgccg     540

tctcccggtg cggggcccgc gccccccgag caggttcatc tgcagaagcc agcggacgcc     600

tctgttcaac ttgtgggtta cctggctcat gagaccttgc cggcgaggct cggcgcttga     660

acgtctgtga cccagccctc accgtcccgg tacttgtatg tgttggcggg agtttggagc     720

tcgttggagc tatcgtttcc gtggaaattt tgagccattt cgaatcactt aaaggagtgg     780

acattgctag caatgagctc ccaaagccat ccagatggac tttctggccg agaccagcca     840

gtggagctgc tgaatcccgc ccgcgtgaac cacatgccca gcacggtgga tgtggccacg     900

gcgctgcctc tgcaagtggc ccccccggca gtgcccatgg acccgcgcct ggaccaccag     960

ttctcactgc ctgtggcaga gccggccctg cgggagcagc agctgcagca ggagctcctg    1020

gcgctcaagc agaagcagca gatccagagg cagatcctca tcgccgagtt ccagaggcag 1080
cacgagcagc tctcccggca gcacgaggcg cagctccacg agcacatcaa gcaataacag 1140
gagatgctgg ccatgaagca ccagcaggag ctgctggaac accagcggaa gctggagagg 1200
caccgccagg agcaggagct ggagaagcag caccgggagc agaagctgca gcagctcaag 1260
aacaaggaga agggcaaaga gagtgccgtg gccagcacag aagtgaagat gaagttacaa 1320
gaatttgtcc tcaataaaaa gaaggcgctg gcccaccgga atctgaacca ctgcacttcc 1380
agagaccctc gctactggta cgggaaaacg cagcacagtt cccttgacca gagttctcca 1440
ccccagagcg gagtgtcgac ctcctataac cacccggtcc tgggaatgta cgacgccaaa 1500
gatgacttcc ctcttaggaa aacagcttct gaaccgaatc tgaaatcacg gtccaggcta 1560
aagcagaaag tggccgaaag acggagcagc cccctgttac gcaggaaaga cgggccagtg 1620
gtcactgctc taaaaaagcg tccgttggat gtcacagact ccgcgtgcag cagcgcccca 1680
ggctccggac ccagctcacc caacaacagc tccgggagcg tcgcgtggag gaacggtatc 1740
gcgcccgccg tccccagcat cccggcggag acgagtttgg cgcacagact tgtggcacga 1800
gaaggctcgg ccgctccact tcccctctac acatcgccat ccttgcccaa catcacgctg 1860
ggcctgcctg ccaccggccc ctctgcgggc acggcgggcc agcaggacac cgagagactc 1920
acccttcccg ccctccagca gaggctctcc cttttccccg gcacccacct cactccctac 1980
ctgagcacct cgcccttgga gcgggacgga ggggcagcgc acagccctct tctgcagcac 2040
atggtcttac tggagcagcc accggcacaa gcacccctcg tcacaggcct gggagtactg 2100
cccctccacg cacagtcctt ggttggtgca gaccgggtgt ccccctccat ccacaagctg 2160
cggcagcacc gcccactggg gcggacccag tcggccccgc tgccccagaa cgcccaggct 2220
ctgcagcacc tggtcatcca gcagcagcat cagcagtttc tggagaaaca caagcagcag 2280
ttccagcagc agcaactgca gatgaacaag atcatcccca agccaagcga gccagcccgg 2340
cagccggaga gccacccgga ggagacggag gaggagctcc gtgagcacca ggctctgctg 2400
gacgagccct acctggaccg gctgccgggg cagaaggagg cgcacgcaca ggccggcgtg 2460
caggtgaagc aggagcccat tgagagcgat gaggaagagg cagagccccc acgggaggtg 2520
gagccgggcc agcgccagcc cagtgagcag gagctgctct tcagacagca agccctcctg 2580
ctggagcagc agcggatcca ccagctgagg aactaccagg cgtccatgga ggccgccggc 2640
atccccgtgt ccttcggcgg ccacaggcct ctgtcccggg cgcagtcctc acccgcgtct 2700
gccaccttcc ccgtgtccgt gcaggagccc cccaccaagc cgaggttcac gacaggcctc 2760

gtgtatgaca cgctgatgct gaagcaccag tgcacctgcg ggagtagcag cagccacccc 2820

gagcacgccg ggaggatcca gagcatctgg tcccgcctgc agaagacggg cctccggggc 2880

aaatgcgagt gcatccgcgg acgcaaggcc accctggaag agctacagac ggtgcactcg 2940

gaagcccaca ccctcctgta tggcacgaac cccctcaacc ggcagaaact ggacagtaag 3000

aaacttctag gctcgctcgc ctccgtgttc gtccggctcc cttgcggtgg tgttggggtg 3060

gacagtgaca ccatatggaa cgaggtgcac tcggcggggg cagcccgcct ggctgtgggc 3120

tgcgtggtag agctggtctt caaggtggcc acaggggagc tgaaaaatgg ctttgctgtg 3180

gtccgccccc ctggacacca tgcggaggag agcacgccca tgggcttttg ctacttcaac 3240

tccgcggccg tggcagccaa gcttctgcag cagaggttga gcgtgagcaa gatcctcatc 3300

gtggactggg acgtgcacca tggaaacggg acccagcagg ctttctacag cgaccctagc 3360

gtcctgtaca tgtccctcca ccgctacgac gatgggaact tcttcccagg cagcggggct 3420

cctgatgagg tgggcacagg gcccggcgtg ggtttcaacg tcaacacggc tttcaccggc 3480

ggcctggacc cccccatggg agacgctgag tacttggcgg ccttcagaac ggtggtaatg 3540

ccgatcgcca gcgagtttgc cccggatgtg gtgctggtgt catcaggctt cgatgccgtg 3600

gagggccacc ccacccctct tgggggctac aacctctccg ccagatgctt cgggtacctg 3660

acgaagcagc tgatgggcct ggctggcggc cggattgtcc tggccctcga gggaggccac 3720

gacctgaccg ccatttgcga cgcctcggaa gcatgtgttt ctgccttgct gggaaacgag 3780

cttgatcctc tcccagaaaa ggttttacag caaagaccca atgcaaacgc tgtccgttcc 3840

atggagaaag tcatggagat ccacagcaag tactggcgct gcctgcagcg cacaacctcc 3900

acagcggggc gttctctgat cgaggctcag acttgcgaga acgaagaagc cgagacggtc 3960

accgccatgg cctcgctgtc cgtggacgtg aagcccgccg aaaagagacc agatgaggag 4020

cccatggaag aggagccgcc cctgtagcac tccctcgaag ctgctgttct cttgtctgtc 4080

tgtctctgtc ttgaagctca gccaagaaac tttcccgtgt cacgcctgcg tcccaccgtg 4140

gggctctctt ggagcaccca gggacaccca gcgtgcaaca gccacgggaa gcctttctgc 4200

cgcccaggcc cacaggtctc gagacgcaca tgcacgcctg ggcgtggcag cctcacaggg 4260

aacacgggac agacgccggc gacgcgcaga cacacggaca cgcggaagcc aagcacactc 4320

tggcgggtcc cgcaagggac gccgtggaag aaaggagcct gtggcaacag gcggccgagc 4380

tgccgaattc agttgacacg aggcacagaa aacaaatatc aaagatctaa taatacaaaa 4440

caaacttgat taaaactggt gcttaaagtt tattacccac aactccacag tctctgtgta 4500
aaccactcga ctcatcttgt agcttatttt ttttttaaag aggacgtttt ctacggctgt 4560
ggcccgcctc tgtgaaccat agcggtgtgc ggcggggggt ctgcacccgg gtgggggaca 4620
gagggacctt taaagaaaac aaaactggac agaaacagga atgtgagctg ggggagctgg 4680
cttgagtttc tcaaaagcca tcggaagatg cgagtttgtg cctttttttt tattgctctg 4740
tcacttggtc actgggctgc tgatggtcag ctctgagaca gtggtttgag agcaggcaga 4800
gtggattttt gtggctgggt tttctgaagt ctgaggaaca atgccttaag aaaaaacaaa 4860
cagcaggaat cggtgggaca gtttcctgtg gccagccgag cctggcagtg ctggcaccgc 4920
gagctggcct gacgcctcaa gcacgggcac cagccgtcat ctccggggcc aggggctgca 4980
gcccggcggt ccctgttttg ctttattgct gtttaagaaa aatggaggta gttccaaaaa 5040
agtggcaaat cccgttggag gttttgaagt ccaacaaatt ttaaacgaat ccaaagtgtt 5100
ctcacacgtc acatacgatt gagcatctcc atctggtcgt gaagcatgtg gtaggcacac 5160
ttgcagtgtt acgatcggaa tgctttttat taaaagcaag tagcatgaag tattgcttaa 5220
attttaggta taaataaata tatatatgta taatatatat tccaatgtat tccaagctaa 5280
gaaacttact tgattcttat gaaatcttga taaaatattt ataatgcatt tatagaaaaa 5340
gtatatatat atatataaaa tgaatgcaga ttgcgaaggt ccctgcaaat ggatggcttg 5400
tgaatttgct ctcaaggtgc ttatggaaag ggatcctgat tgattgaaat tcatgttttc 5460
tcaagctcca gattggctag atttcagatc gccaacacat tcgccactgg gcaactaccc 5520
tacaagtttg tactttcatt ttaattattt tctaacagaa ccgctcccgt ctccaagcct 5580
tcatgcacat atgtacctaa tgagttttta tagcaaagaa tataaatttg ctgttgattt 5640
ttgtatgaat tttttcacaa aaagatcctg aataagcatt gttttatgaa ttttacattt 5700
ttcctcacca tttagcaatt ttccgaatgg taataatgtc taaatctttt tcctttctga 5760
attcttgctt gtacattttt ttttaccttt caaaggtttt taattatttt tgtttttatt 5820
tttgtacgat gagttttctg cagcgtacag aattgttgct gtcagattct attttcagaa 5880
agtgagagga gggaccgtag gtcttttcgg agtgacacca acgattgtgt ctttcctggt 5940
ctgtcctagg agctgtataa agaagcccag gggctctttt taactttcaa cactagtagt 6000
attacgaggg gtggtgtgtt tttcccctcc gtggcaaggg cagggagggt tgcttaggat 6060
gcccggccac cctgggaggc ttgccagatg ccgggggcag tcagcattaa tgaaactcat 6120
gtttaaactt ctctgaccac atcgtcagga tagaattcta acttgagttt tccaaacacc 6180

ttttgagcat gtcagcaatg catggggcac acgtggggct ctttacccac ttgggttttt 6240

ccactgcagc cacgtggcca gccctggatt ttggagcctg tggctgcaag gaacccaggg 6300

acccttgttg cctggtgaac ctgcagggag ggtatgattg cctgaccagg acagccagtc 6360

tttactcttt ttctcttcaa cagtaactga cagtcacgtt ttactggtaa cttattttcc 6420

agcacatgaa gccaccagtt tcattccaaa gtgtatattg ggttcagact tgggggcaga 6480

agttcagaca caccgtgctc aggagggacc cagagccgag tttcggagtt tggtaaagtt 6540

tacagggtag cttctgaaat taactcaaac ttttgaccaa atgagtgcag attcttggat 6600

acggtcttgg gacttgtttg actttcccct ccctggtggc cactctttgc tctgaagccc 6660

agattggcaa gaggagctgg tccattcccc attcatggca cagaacagtg gcagggccca 6720

gctagcaggc tcttctggcc tccttggcct cattctctgc atagccctct ggggatcctg 6780

ccacctgccc tcttaccccg ccgtggctta tggggaggaa tgcatcatct cacttttttt 6840

ttttaagcag atgatgggat aacatggact gctcagtggc caggttatca gtggggggac 6900

ttaattctaa tctcattcaa atggagacga cctctgcaaa ggcctggcag ggggaggcaa 6960

gtttcatctg tcagctcact ccagcttcac aaatgtgctg agagcattac tgtgtagcct 7020

tttctttgaa gacacactcg gctcttctcc acagcaagcg tccagggcag atggcagagg 7080

atctgcctcg gcgtctgcag gcgggaccac gtcagggagg gttccttcat gtgttctccc 7140

tgtgggtcct tggaccttta gcctttttct tcctttgcaa aggccttggg ggcactggct 7200

gggagtcagc aagcgagcac tttatatccc tttgagggaa accctgatga cgccactggg 7260

cctcttggcg tctgacctgc cctcgccgct tcccgccgtg ccgcagcgtg cccacgtgcc 7320

cacgccccac cagcaggcgg ctgccccgga ggccgtggcc cgctgggact ggccgcccct 7380

ccccagcgtc ccagggctct ggttctggag ggccactttg tcaaggtgtt tcagtttttc 7440

tttacttctt ttgaaaatct gtttgcaagg ggaaggacca tttcgtaatg gtctgacaca 7500

aaagcaagtt tgatttttgc agcactagca atggactttg ttgcttttct ttttgatcag 7560

aacattcctt ctttactggt cacagccacg tgctcattcc attcttcttt ttgtagactt 7620

tgggcccacg tgttttatgg gcattgatac atatataaat atatagatat aaatatatat 7680

gaatacattt ttttaagttt cctacacctg gaggttgcat ggactgtacg accggcatga 7740

ctttatattg tatacagatt ttgcacgcca aactcggcag ctttggggaa gaagaaaaat 7800

gcctttctgt tcccctctca tgacatttgc agatacaaaa gatggaaatt tttctgtaaa 7860

acaaaacctt gaaggagagg agggcgggga agtttgcgtc ttattgaact tattcttaag 7920

aaattgtact ttttattgta agaaaaataa aaaggactac ttaaacattt gtcatattaa 7980

gaaaaaaagt ttatctagca cttgtgacat accaataata gagtttattg tatttatgtg 8040

gaaacagtgt tttagggaaa ctactcagaa ttcacagtga actgcctgtc tctctcgagt 8100

tgatttggag gaattttgtt ttgttttgtt ttgtttgttt ccttttatct ccttccacgg 8160

gccaggcgag cgccgcccgc cctcactggc cttgtgacgg tttattctga ttgagaactg 8220

ggcggactcg aaagagtccc cttttccgca cagctgtgtt gactttttaa ttacttttag 8280

gtgatgtatg gctaagattt cactttaagc agtcgtgaac tgtgcgagca ctgtggttta 8340

caattatact ttgcatcgaa aggaaaccat ttcttcattg taacgaagct gagcgtgttc 8400

ttagctcggc ctcactttgt ctctggcatt gattaaaagt ctgctattga aagaaaaag 8459

<210> 9
<211> 717
<212> PRT
<213> Human

<400> 9

Leu Arg Gln Gly Gly Thr Leu Thr Gly Lys Phe Met Ser Thr Ser Ser
1               5                   10                  15

Ile Pro Gly Cys Leu Leu Gly Val Ala Leu Glu Gly Asp Gly Ser Pro
            20                  25                  30

His Gly His Ala Ser Leu Leu Gln His Val Leu Leu Leu Glu Gln Ala
        35                  40                  45

Arg Gln Gln Ser Thr Leu Ile Ala Val Pro Leu His Gly Gln Ser Pro
    50                  55                  60

Leu Val Thr Gly Glu Arg Val Ala Thr Ser Met Arg Thr Val Gly Lys
65                  70                  75                  80

Leu Pro Arg His Arg Pro Leu Ser Arg Thr Gln Ser Ser Pro Leu Pro
                85                  90                  95

Gln Ser Pro Gln Ala Leu Gln Gln Leu Val Met Gln Gln Gln His Gln
            100                 105                 110

Gln Phe Leu Glu Lys Gln Lys Gln Gln Gln Leu Gln Leu Gly Lys Ile
        115                 120                 125

Leu Thr Lys Thr Gly Glu Leu Pro Arg Gln Pro Thr Thr His Pro Glu
    130                 135                 140

Glu Thr Glu Glu Glu Leu Thr Glu Gln Gln Glu Val Leu Leu Gly Glu
145                 150                 155                 160

Gly Ala Leu Thr Met Pro Arg Glu Gly Ser Thr Glu Ser Glu Ser Thr
                165                 170                 175

Gln Glu Asp Leu Glu Glu Glu Asp Glu Glu Glu Asp Gly Glu Glu Glu
            180                 185                 190

Asp Cys Ile Gln Val Lys Asp Glu Glu Gly Glu Ser Gly Ala Glu Glu
        195                 200                 205

Gly Pro Asp Leu Glu Glu Pro Gly Ala Gly Tyr Lys Lys Leu Phe Ser
    210                 215                 220

Asp Ala Gln Pro Leu Gln Pro Leu Gln Val Tyr Gln Ala Pro Leu Ser
225                 230                 235                 240

Leu Ala Thr Val Pro His Gln Ala Leu Gly Arg Thr Gln Ser Ser Pro
                245                 250                 255

Ala Ala Pro Gly Gly Met Lys Ser Pro Pro Asp Gln Pro Val Lys His
            260                 265                 270

Leu Phe Thr Thr Gly Val Val Tyr Asp Thr Phe Met Leu Lys His Gln
        275                 280                 285

Cys Met Cys Gly Asn Thr His Val His Pro Glu His Ala Gly Arg Ile
    290                 295                 300

Gln Ser Ile Trp Ser Arg Leu Gln Glu Thr Gly Leu Leu Ser Lys Cys
305                 310                 315                 320

Glu Arg Ile Arg Gly Arg Lys Ala Thr Leu Asp Glu Ile Gln Thr Val
                325                 330                 335

His Ser Glu Tyr Ile His Thr Leu Leu Tyr Gly Thr Ser Pro Leu Asn
            340                 345                 350

Arg Gln Lys Leu Asp Ser Lys Lys Leu Leu Gly Pro Ile Ser Gln Lys
        355                 360                 365

Met Tyr Ala Val Leu Pro Cys Gly Gly Ile Gly Val Asp Ser Asp Thr
    370                 375                 380

Val Trp Asn Glu Met His Ser Ser Ser Ala Val Arg Met Ala Val Gly
385                 390                 395                 400

Cys Leu Leu Glu Leu Ala Phe Lys Val Ala Ala Gly Glu Leu Lys Asn
                405                 410                 415

Gly Phe Ala Ile Ile Arg Pro Pro Gly His His Ala Glu Glu Ser Thr
            420                 425                 430

Ala Met Gly Phe Cys Phe Phe Asn Ser Val Ala Ile Thr Ala Lys Leu
        435                 440                 445

Leu Gln Gln Lys Leu Asn Val Gly Lys Val Leu Ile Val Asp Trp Asp
    450                 455                 460

Ile His His Gly Asn Gly Thr Gln Gln Ala Phe Tyr Asn Asp Pro Ser
465                 470                 475                 480

Val Leu Tyr Ile Ser Leu His Arg Tyr Asp Asn Gly Asn Phe Phe Pro
                485                 490                 495

Gly Ser Gly Ala Pro Glu Glu Val Gly Gly Gly Pro Gly Val Gly Tyr
            500                 505                 510

Asn Val Asn Val Ala Trp Thr Gly Gly Val Asp Pro Pro Ile Gly Asp
        515                 520                 525

Val Glu Tyr Leu Thr Ala Phe Arg Thr Val Val Met Pro Ile Ala His
    530                 535                 540

Glu Phe Ser Pro Asp Val Val Thr Leu Val Ser Ala Gly Phe Asp Ala
545                 550                 555                 560

Val Glu Gly His Leu Ser Pro Leu Gly Gly Tyr Ser Val Thr Ala Arg
                565                 570                 575

Cys Phe Gly His Leu Thr Arg Gln Leu Met Thr Leu Ala Gly Gly Arg
            580                 585                 590

Val Val Leu Ala Leu Glu Gly Gly His Asp Leu Thr Ala Ile Cys Asp
        595                 600                 605

Ala Ser Glu Ala Cys Val Ser Ala Leu Leu Ser Val Glu Leu Gln Pro
    610                 615                 620

Leu Asp Glu Leu Val Leu Gln Gln Lys Pro Asn Ile Asn Ala Val Ala
625                 630                 635                 640

Thr Leu Glu Lys Val Ile Glu Thr Gln Ser Lys His Trp Ser Cys Val
                645                 650                 655

Gln Lys Phe Ala Ala Gly Leu Gly Arg Ser Leu Arg Glu Ala Gln Ala
            660                 665                 670

Gly Glu Thr Glu Glu Ala Glu Thr Val Ser Ala Met Ala Leu Leu Ser
        675                 680                 685

Val Gly Ala Glu Gln Ala Gln Ala Ala Ala Ala Arg Glu His Ser Pro
    690                 695                 700

Arg Pro Ala Glu Glu Pro Met Glu Gln Glu Pro Ala Leu
705                 710                 715

<210>  10
<211>  2233
<212>  DNA
<213>  Human

<400>  10
ccctgcggca gggtggcacg ctgaccggca agttcatgag cacatcctct attcctggct      60

gcctgctggg cgtggcactg gagggcgacg ggagccccca cgggcatgcc tccctgctgc     120

agcatgtgct gttgctggag caggcccggc agcagagcac cctcattgct gtgccactcc     180

acgggcagtc cccactagtg acgggtgaac gtgtggccac cagcatgcgg acggtaggca     240

agctcccgcg gcatcggccc ctgagccgca ctcagtcctc accgctgccg cagagtcccc     300

aggccctgca gcagctggtc atgcaacaac agcaccagca gttcctggag aagcagaagc 360
agcagcagct acagctgggc aagatcctca ccaagacagg ggagctgccc aggcagccca 420
ccacccaccc tgaggagaca gaggaggagc tgacggagca gcaggaggtc ttgctggggg 480
agggagccct gaccatgccc cgggagggct ccacagagag tgagagcaca caggaagacc 540
tggaggagga ggacgaggaa gaggatgggg aggaggagga ggattgcatc caggttaagg 600
acgaggaggg cgagagtggt gctgaggagg ggcccgactt ggaggagcct ggtgctggat 660
acaaaaaact gttctcagat gcccagccgc tgcagccttt gcaggtgtac caggcgcccc 720
tcagcctggc cactgtgccc caccaggccc tgggccgtac ccagtcctcc cctgctgccc 780
ctgggggcat gaagagcccc ccagaccagc ccgtcaagca cctcttcacc acaggtgtgg 840
tctacgacac gttcatgcta aagcaccagt gcatgtgcgg gaacacacac gtgcaccctg 900
agcatgctgg ccggatccag agcatctggt cccggctgca ggagacaggc ctgcttagca 960
agtgcgagcg gatccgaggt cgcaaagcca cgctagatga gatccagaca gtgcactctg 1020
aataccacac cctgctctac gggaccagtc ccctcaaccg gcagaagcta gacagcaaga 1080
agttgctcgg ccccatcagc cagaagatgt atgctgtgct gccttgtggg ggcatcgggg 1140
tggacagtga caccgtgtgg aatgagatgc actcctccag tgctgtgcgt atggcagtgg 1200
gctgcctgct ggagctggcc ttcaaggtgg ctgcaggaga gctcaagaat ggatttgcca 1260
tcatccggcc cccaggacac cacgccgagg aatccacagc cacgggattc tgcttcttca 1320
actctgtagc catcaccgca aaactcctac agcagaagtt gaacgtgggc aaggtcctca 1380
tcgtggactg ggacattcac catggcaatg gcacccagca ggcgttctat aatgacccct 1440
ctgtgctcta catctctctg catcgctatg acaacgggaa cttctttcca ggctctgggg 1500
ctcctgaaga ggttggtgga ggaccaggcg tggggtacaa tgtgaacgtg gcatggacag 1560
gaggtgtgga cccccccatt ggagacgtgg agtaccttac agccttcagg acagtggtga 1620
tgcccattgc ccacgagttc tcacctgatg tggtcctagt ctccgccggg tttgatgctg 1680
ttgaaggaca tctgtctcct ctgggtggct actctgtcac cgccagatgt tttggccact 1740
tgaccaggca gctgatgacc ctggcagggg gccgggtggt gctggccctg gagggaggcc 1800
atgacttgac cgccatctgt gatgcctctg aggcttgtgt ctcggctctg ctcagtgtag 1860
agctgcagcc cttggatgag gcagtcttgc agcaaaagcc caacatcaac gcagtggcca 1920
cgctagagaa agtcatcgag atccagagca aacactggag ctgtgtgcag aagttcgccg 1980
ctggtctggg ccggtccctg cgagaggccc aagcaggtga ggccgaggag gccgagactg 2040

tgagcgccat ggccttgctg tcggtggggg ccgagcaggc ccaggctgcg gcagcccggg 2100

aacacagccc caggccggca gaggagccca tggagcagga gcctgccctg tgacgccccg 2160

gcccccatcc ctctcggctt caccattgtg attttgttta ttttttctat taaaaacaaa 2220

aagtcacaca ttc 2233

<210> 11
<211> 1215
<212> PRT
<213> Human

<400> 11

Met Thr Ser Thr Gly Gln Asp Ser Thr Thr Thr Arg Gln Arg Arg Ser
1               5                   10                  15

Arg Gln Asn Pro Gln Ser Pro Pro Gln Asp Ser Ser Val Thr Ser Lys
            20                  25                  30

Arg Asn Ile Lys Lys Gly Ala Val Pro Arg Ser Ile Pro Asn Leu Ala
        35                  40                  45

Glu Val Lys Lys Lys Gly Lys Met Lys Lys Leu Gly Gln Ala Met Glu
    50                  55                  60

Glu Asp Leu Ile Val Gly Leu Gln Gly Met Asp Leu Asn Leu Glu Ala
65                  70                  75                  80

Glu Ala Leu Ala Gly Thr Gly Leu Val Leu Asp Glu Gln Leu Asn Glu
                85                  90                  95

Phe His Cys Leu Trp Asp Asp Ser Phe Pro Glu Gly Pro Glu Arg Leu
            100                 105                 110

His Ala Ile Lys Glu Gln Leu Ile Gln Glu Gly Leu Leu Asp Arg Cys
        115                 120                 125

Val Ser Phe Gln Ala Arg Phe Ala Glu Lys Glu Glu Leu Met Leu Val
    130                 135                 140

His Ser Leu Glu Tyr Ile Asp Leu Met Glu Thr Thr Gln Tyr Met Asn
145                 150                 155                 160

Glu Gly Glu Leu Arg Val Leu Ala Asp Thr Tyr Asp Ser Val Tyr Leu
                165                 170                 175

His Pro Asn Ser Tyr Ser Cys Ala Cys Leu Ala Ser Gly Ser Val Leu
            180                 185                 190

Arg Leu Val Asp Ala Val Leu Gly Ala Glu Ile Arg Asn Gly Met Ala
        195                 200                 205

Ile Ile Arg Pro Pro Gly His His Ala Gln His Ser Leu Met Asp Gly
    210                 215                 220

Tyr Cys Met Phe Asn His Val Ala Val Ala Ala Arg Tyr Ala Gln Gln
225                 230                 235                 240

Lys His Arg Ile Arg Arg Val Leu Ile Val Asp Trp Asp Val His His
                245                 250                 255

Gly Gln Gly Thr Gln Phe Thr Phe Asp Gln Asp Pro Ser Val Leu Tyr
            260                 265                 270

Phe Ser Ile His Arg Tyr Glu Gln Gly Arg Phe Trp Pro His Leu Lys
        275                 280                 285

Ala Ser Asn Trp Ser Thr Thr Gly Phe Gly Gln Gly Gln Gly Tyr Thr
    290                 295                 300

Ile Asn Val Pro Trp Asn Gln Val Gly Met Arg Asp Ala Asp Tyr Ile
305                 310                 315                 320

Ala Ala Phe Leu His Val Leu Leu Pro Val Ala Leu Glu Phe Gln Pro
                325                 330                 335

Gln Leu Val Leu Val Ala Ala Gly Phe Asp Ala Leu Gln Gly Asp Pro
            340                 345                 350

Lys Gly Glu Met Ala Ala Thr Pro Ala Gly Phe Ala Gln Leu Thr His
        355                 360                 365

Leu Leu Met Gly Leu Ala Gly Gly Lys Leu Ile Leu Ser Leu Glu Gly
    370                 375                 380

Gly Tyr Asn Ile Arg Ala Leu Ala Glu Gly Val Ser Ala Ser Leu His
385                 390                 395                 400

Thr Leu Leu Gly Asp Pro Cys Pro Met Leu Glu Ser Pro Gly Ala Pro
                405                 410                 415

Cys Arg Ser Ala Gln Ala Ser Val Ser Cys Ala Leu Glu Ala Leu Glu
            420                 425                 430

Pro Phe Trp Glu Val Leu Val Arg Ser Thr Glu Thr Val Glu Arg Asp
        435                 440                 445

Asn Met Glu Glu Asp Asn Val Glu Glu Ser Glu Glu Glu Gly Pro Trp
    450                 455                 460

Glu Pro Pro Val Leu Pro Ile Leu Thr Trp Pro Val Leu Gln Ser Arg
465                 470                 475                 480

Thr Gly Leu Val Tyr Asp Gln Asn Met Met Asn His Cys Asn Leu Trp
                485                 490                 495

Asp Ser His His Pro Glu Val Pro Gln Arg Ile Leu Arg Ile Met Cys
            500                 505                 510

Arg Leu Glu Glu Leu Gly Ile Ala Gly Arg Cys Leu Thr Ile Thr Pro
        515                 520                 525

Arg Pro Ala Thr Glu Ala Glu Leu Leu Thr Cys His Ser Ala Glu Tyr
    530                 535                 540

Val Gly His Leu Arg Ala Thr Glu Lys Met Lys Thr Arg Glu Leu His
545                 550                 555                 560

Arg Glu Ser Ser Asn Phe Asp Ser Ile Tyr Ile Cys Pro Ser Thr Phe
                565                 570                 575

Ala Cys Ala Gln Ile Ala Thr Gly Ala Ala Cys Arg Leu Val Glu Ala
            580                 585                 590

Val Ile Ser Gly Glu Val Ile Asn Gly Ala Ala Val Val Arg Pro Pro
        595                 600                 605

Gly His His Ala Glu Gln Asp Ala Ala Cys Gly Phe Cys Phe Phe Asn
    610                 615                 620

Ser Val Ala Val Ala Ala Arg His Ala Gln Thr Ile Ser Gly His Ala
625                 630                 635                 640

Leu Arg Ile Leu Ile Val Asp Trp Asp Val His His Gly Asn Gly Thr
                645                 650                 655

Gln His Met Phe Glu Asp Asp Pro Ser Val Leu Tyr Val Ser Leu His
            660                 665                 670

Arg Tyr Asp His Gly Thr Phe Phe Pro Met Gly Asp Glu Gly Ala Ser
        675                 680                 685

Ser Gln Ile Gly Arg Ala Ala Gly Thr Gly Phe Thr Val Asn Val Ala
    690                 695                 700

Trp Asn Gly Pro Arg Met Gly Asp Ala Asp Tyr Leu Ala Ala Trp His
705                 710                 715                 720

Arg Leu Val Leu Pro Ile Ala Tyr Glu Phe Asn Pro Glu Leu Val Leu
                725                 730                 735

Val Ser Ala Gly Phe Asp Ala Ala Arg Gly Asp Pro Leu Gly Gly Cys
            740                 745                 750

Gln Val Ser Pro Glu Gly Tyr Ala His Leu Thr His Leu Leu Met Gly
        755                 760                 765

Leu Ala Ser Gly Arg Ile Ile Leu Ile Leu Glu Gly Gly Tyr Asn Leu
    770                 775                 780

Thr Ser Ile Ser Glu Ser Met Ala Ala Cys Thr Arg Ser Ile Leu Gly
785                 790                 795                 800

Asp Pro Pro Pro Leu Leu Thr Leu Pro Arg Pro Pro Leu Ser Gly Ala
                805                 810                 815

Leu Ala Ser Ile Thr Glu Thr Ile Gln Val His Arg Arg Tyr Trp Arg
            820                 825                 830

Ser Leu Arg Val Met Lys Val Glu Asp Arg Glu Gly Pro Ser Ser Ser
        835                 840                 845

Lys Leu Val Thr Lys Lys Ala Pro Gln Pro Ala Lys Pro Arg Leu Ala
    850                 855                 860

Glu Arg Met Thr Thr Arg Glu Lys Lys Val Leu Glu Ala Gly Met Gly
865                 870                 875                 880

Lys Val Thr Ser Ala Ser Phe Gly Glu Glu Ser Thr Pro Gly Gln Thr
                885                 890                 895

Asn Ser Glu Thr Ala Val Val Ala Leu Cys Gln Asp Gln Pro Ser Glu
            900                 905                 910

Ala Ala Thr Gly Gly Ala Thr Leu Ala Gln Thr Ile Ser Glu Ala Ala
        915                 920                 925

Ile Gly Gly Ala Met Leu Gly Gln Thr Thr Ser Glu Glu Ala Val Gly
    930                 935                 940

Gly Ala Thr Pro Asp Gln Thr Thr Ser Glu Glu Thr Val Gly Gly Ala
945                 950                 955                 960

Ile Leu Asp Gln Thr Thr Ser Glu Asp Ala Val Gly Gly Ala Thr Ile
                965                 970                 975

Gly Gln Thr Thr Ser Glu Glu Ala Val Gly Gly Ala Thr Leu Ala Gln
            980                 985                 990

Thr Ile Ser Glu Ala Ala Met Glu Gly Ala Thr Leu Asp Gln Thr Thr
        995                 1000                1005

Ser Glu  Glu Ala Pro Gly Gly  Thr Glu Leu Ile Gln  Thr Pro Leu
    1010                 1015                 1020

Ala Ser  Ser Thr Asp His Gln  Thr Pro Pro Thr Ser  Pro Val Gln
    1025                 1030                 1035

Gly Thr  Thr Pro Gln Ile Ser  Pro Ser Thr Leu Ile  Gly Ser Leu
    1040                 1045                 1050

Arg Thr Leu Glu Leu Gly Ser Glu Ser Gln Gly Ala Ser Glu Ser
    1055                1060                1065

Gln Ala Pro Gly Glu Glu Asn Leu Leu Gly Glu Ala Ala Gly Gly
    1070                1075                1080

Gln Asp Met Ala Asp Ser Met Leu Met Gln Gly Ser Arg Gly Leu
    1085                1090                1095

Thr Asp Gln Ala Ile Phe Tyr Ala Val Thr Pro Leu Pro Trp Cys
    1100                1105                1110

Pro His Leu Val Ala Val Cys Pro Ile Pro Ala Ala Gly Leu Asp
    1115                1120                1125

Val Thr Gln Pro Cys Gly Asp Cys Gly Thr Ile Gln Glu Asn Trp
    1130                1135                1140

Val Cys Leu Ser Cys Tyr Gln Val Tyr Cys Gly Arg Tyr Ile Asn
    1145                1150                1155

Gly His Met Leu Gln His His Gly Asn Ser Gly His Pro Leu Val
    1160                1165                1170

Leu Ser Tyr Ile Asp Leu Ser Ala Trp Cys Tyr Tyr Cys Gln Ala
    1175                1180                1185

Tyr Val His His Gln Ala Leu Leu Asp Val Lys Asn Ile Ala His
    1190                1195                1200

Gln Asn Lys Phe Gly Glu Asp Met Pro His Pro His
    1205                1210                1215

<210> 12
<211> 4099
<212> DNA
<213> Human

<400> 12
gggcagtccc ctgaggagcg gggctggttg aaacgctagg ggcgggatct ggcggagtgg 60

aagaaccgcg gcaggggcca agcctcctca actatgacct caaccggcca ggattccacc 120

acaaccaggc agcgaagaag taggcagaac ccccagtcgc cccctcagga ctccagtgtc 180

acttcgaagc gaaatattaa aaagggagcc gttccccgct ctatccccaa tctagcggag 240
gtaaagaaga aaggcaaaat gaagaagctc ggccaagcaa tggaagaaga cctaatcgtg 300
ggactgcaag ggatggatct gaacctcgag gctgaagcac tggctggcac tggcttggtg 360
ttggatgagc agttaaatga attccattgc ctctgggatg acagcttccc ggaaggccct 420
gagcggctcc atgccatcaa ggagcaactg atccaggagg gcctcctaga tcgctgcgtg 480
tcctttcagg cccggtttgc tgaaaaggaa gagctgatgt tggttcacag cctagaatat 540
attgacctga tggaaacaac ccagtacatg aatgagggag aactccgtgt cctagcagac 600
acccacgact cagtttatct gcatccgaac tcatactcct gtgcctgcct ggcctcaggc 660
tctgtcctca ggctggtgga tgcggtcctg ggggctgaga tccggaacgg catggccatc 720
attaggcctc ctggacatca cgcccagcac agtcttatgg atggctattg catgttcaac 780
cacgtggctg tggcagcccg ctatgctcaa cagaaacacc gcacccggag ggtccttatc 840
gtagattggg atgtgcacca cggtcaagga acacagttca ccttcgacca ggaccccagt 900
gtcctctatt tctccatcca ccgctacgag cagggtaggt tctggcccca cctgaaggcc 960
tctaactggt ccaccacagg tttcggccaa ggccaaggat ataccatcaa tgtgccttgg 1020
aaccaggtgg ggatgcggga tgctgactac attgctgctt tcctgcacgt cctgctgcca 1080
gtcgccctcg agctccagcc tcagctggtc ctggtggccg ctggatttga tgccctgcaa 1140
ggggacccca agggcgagat ggccgccact ccggcagggt tcgcccagct aacccacctg 1200
ctcatgggtc tggcaggagg caagctgatc ctgtctctgg agggtggcta caacctccgc 1260
gccctggctg aaggcgtcag tgcttcgctc cacacccttc tgggagaccc ttgccccatg 1320
ccggagtcac ctggtgcccc ctgccggagc gcccaggctt cagtttcctg tgctctggaa 1380
gcccttgagc ccttctggga ggttcttgtg agatcaactg agaccgtgga gagggacaac 1440
atggaggagg acaatgtaga ggagagcgag gaggaaggac cctgggagcc ccctgtgctc 1500
ccaatcctga catggccagt gctacagtct cgcacagggc tggtctatga ccaaaatatg 1560
atgaatcact gcaacttgtg ggacagccac caccctgagg taccccagcg catcttgcgg 1620
atcatgtgcc gtctggagga gctgggcctt gccgggcgct gcctcaccct gacaccgcgc 1680
cctgccacag aggctgagct gctcacctgt cacagtgctg agtacgtggg tcatctccgg 1740
gccacagaga aaatgaaaac ccgggagctg caccgtgaga gttccaactt tgactccatc 1800
tatatctgcc ccagtacctt cgcctgtgca cagcttgcca ctggcgctgc ctgccgcctg 1860
gtggaggctg tgctctcagg agaggtcctg aatggtgctg ctgtggtgcg tcccccagga 1920

caccacgcag agcaggatgc agcttgcggt ttttgctttt tcaactctgt ggctgtggct 1980
gctcgccatg cccagactat cagtgggcat gccctacgga tcctgattgt ggattgggat 2040
gtccaccacg gtaatggaac tcagcacatg tttgaggatg accccagtgt gctatatgtg 2100
tccctgcacc gctatgatca tggcaccttc ttccccatgg gggatgaggg tgccagcagc 2160
cagatcggcc gggccgcggg cacaggcttc accgtcaacg tggcatggaa cgggccccgc 2220
atgggtgatg ctgactacct agctgcctgg catcgcctgg tgcttcccat tgcctacgag 2280
tttaacccag aactggtgct ggtctcagct ggctttgatg ctgcacgggg ggatccgctg 2340
gggggctgcc aggtgtcacc tgagggttat gcccacctca cccacctgct gatgggcctt 2400
gccagtggcc gcattatcct tatcctagag ggtggctata acctgacatc catctcagag 2460
tccatggctg cctgcactcg ctccctcctt ggagacccac cacccctgct gaccctgcca 2520
cggcccccac tatcaggggc cctggcctca atcactgaga ccatccaagt ccatcgcaga 2580
tactggcgca gcttacgggt catgaaggca gaagacagag aaggaccctc cagttctaag 2640
ttggtcacca agaaggcacc ccaaccagcc aaacctaggt tagctgagcg gatgaccaca 2700
cgagaaaaga aggttctgga agcaggcatg gggaaagtca cctcggcatc atttggggaa 2760
gagtccactc caggccagac taactcagag acagctgtgg tggccctcac tcaggaccag 2820
ccctcagagg cagccacagg gggagccact ctggcccaga ccatttctga ggcagccatt 2880
gggggagcca tgctgggcca gaccacctca gaggaggctg tcgggggagc cactccggac 2940
cagaccacct cagaggagac tgtgggagga gccattctgg accagaccac ctcagaggat 3000
gctgttgggg gagccacgct gggccagact acctcagagg aggctgtagg aggagctaca 3060
ctggcccaga ccatctcgga ggcagccatg gagggagcca cactggacca gactacgtca 3120
gaggaggctc cagggggcac cgagctgatc caaactcctc tagcctcgag cacagaccac 3180
cagacccccc caacctcacc tgtgcaggga actacacccc agatatctcc cagtacactg 3240
attgggagtc tcaggacctt ggagctaggc agcgaacctc agggggcctc agaatctcag 3300
gccccaggag aggagaacct accaggagag gcagctggag gtcaggacat ggctgattcg 3360
atgctgacgc agggatctag gggcctcact gatcaggcca tattttatgc tgtgacacca 3420
ctgccctggt gtccccattc ggtggcagta tgccccatac ctgcagcagg cctagacgtg 3480
acccaacctt gtggggactg tggaacaatc caagagaact gggtgtgtct ctcttgctat 3540
caggtctacc gtggtcgtta catcaatggc cacatgctcc aacaccatgg aaattctgga 3600

cacccgctgg tcctcagcca catcgacctg tcagcctggc gttactactg tcaggcctat 3660

gtccaccacc aggctctcct agatgtgaag aacatcgccc accagaacaa gtttggggag 3720

gatatgcccc acccacacta agccccagaa tacggtccct cttcaccttc tgaggcccac 3780

gatagaccag ttccagcctg ttccaggctg taccttggat gaggggtagc ctcccactgc 3840

atcccatcct gaatatcctt tgcaactccc caagagtgct tatttaagtg ttaatacttt 3900

taagagaact gcgacgatta attgtggatc tccccctgcc catcgcccgc ttgaggggca 3960

ccactactcc agcccagaag gaaagggggg cagctcagtg gccccaagag ggagccgata 4020

tcatgaggat aacattggcg ggaggggagt taactggcag gcatggcaag gttgcatatg 4080

taataaagta caagctgtt 4099

<210> 13
<211> 855
<212> PRT
<213> Human

<400> 13
   Met Asp Leu Arg Val Gly Gln Arg Pro Pro Val Glu Pro Pro Pro Glu
   1 5 10 15
   Pro Thr Leu Leu Ala Leu Gln Arg Pro Gln Arg Leu His His His Leu
   20 25 30
   Phe Leu Ala Gly Leu Gln Gln Gln Arg Ser Val Glu Pro Met Arg Leu
   35 40 45
   Ser Met Asp Thr Pro Met Pro Glu Leu Gln Val Gly Pro Gln Glu Gln
   50 55 60
   Glu Leu Arg Gln Leu Leu His Lys Asp Lys Ser Lys Arg Ser Ala Val
   65 70 75 80
   Ala Ser Ser Val Val Lys Gln Lys Leu Ala Glu Val Ile Leu Lys Lys
   85 90 95
   Gln Gln Ala Ala Leu Glu Arg Thr Val His Pro Asn Ser Pro Gly Ile
   100 105 110
   Pro Tyr Arg Thr Leu Glu Pro Ile Glu Thr Glu Gly Ala Thr Arg Ser
   115 120 125
   Met Leu Ser Ser Phe Leu Pro Pro Val Pro Ser Ile Pro Ser Asp Pro
       130                 135                 140
   Pro Glu His Phe Pro Leu Arg Lys Thr Val Ser Glu Pro Asn Leu Lys
   145                 150                 155                 160
   Leu Arg Tyr Lys Pro Lys Lys Ser Leu Glu Arg Arg Lys Asn Pro Leu
                   165                 170                 175
   Leu Arg Lys Glu Ser Ala Pro Pro Ser Leu Arg Arg Arg Pro Ala Glu
               180                 185                 190
   Thr Leu Gly Asp Ser Ser Pro Ser Ser Ser Ser Thr Pro Ala Ser Gly
           195                 200                 205
   Cys Ser Ser Pro Asn Asp Ser Glu His Gly Pro Asn Pro Ile Leu Gly
       210                 215                 220
   Asp Ser Asp Arg Arg Thr His Pro Thr Leu Gly Pro Arg Gly Pro Ile
   225                 230                 235                 240
   Leu Gly Ser Pro His Thr Pro Leu Phe Leu Pro His Gly Leu Glu Pro
                   245                 250                 255
   Glu Ala Gly Gly Cys Leu Pro Ser Arg Leu Gln Pro Ile Leu Leu Leu
               260                 265                 270
   Asp Pro Ser Gly Ser His Ala Pro Leu Leu Thr Val Pro Gly Leu Gly
           275                 280                 285
   Pro Leu Pro Phe His Phe Ala Gln Ser Ile Met Thr Thr Glu Arg Leu
       290                 295                 300
   Ser Gly Ser Gly Leu His Trp Pro Leu Ser Arg Thr Arg Ser Glu Pro
   305                 310                 315                 320
   Leu Pro Pro Ser Ala Thr Ala Pro Pro Pro Pro Gly Pro Met Gln Pro
                   325                 330                 335
   Arg Leu Glu Gln Leu Lys Thr His Val Gln Val Ile Lys Arg Ser Ala
               340                 345                 350
   Lys Pro Ser Glu Lys Pro Arg Leu Arg Gln Ile Pro Ser Ala Glu Asp
   355 360 365
   Leu Glu Thr Asp Gly Gly Gly Pro Gly Gln Val Val Asp Asp Gly Leu
   370 375 380
   Glu His Arg Glu Leu Gly His Gly Gln Pro Glu Ala Arg Gly Pro Ala
   385 390 395 400
   Pro Leu Gln Gln His Pro Gln Val Ile Ile Trp Glu Gln Gln Arg Leu
   405 410 415
   Ala Gly Arg Leu Pro Arg Gly Ser Thr Gly Asp Cys Val Ile Leu Pro
   420 425 430
   Leu Ala Gln Gly Gly His Arg Pro Leu Ser Arg Ala Gln Ser Ser Pro
   435 440 445
   Ala Ala Pro Ala Ser Ile Ser Ala Pro Glu Pro Ala Ser Gln Ala Arg
   450 455 460
   Val Leu Ser Ser Ser Glu Thr Pro Ala Arg Thr Leu Pro Phe Leu Thr
   465 470 475 480
   Gly Leu Ile Tyr Asp Ser Val Met Leu Lys His Gln Cys Ser Cys Gly
   485 490 495
   Asp Asn Ser Arg His Pro Glu His Ala Gly Arg Ile Gln Ser Ile Trp
   500 505 510
   Ser Arg Leu Gln Glu Arg Gly Leu Arg Ser Gln Cys Glu Cys Leu Arg
   515 520 525
   Gly Arg Lys Ala Ser Ile Glu Glu Leu Gln Ser Val His Ser Glu Arg
   530 535 540
   His Val Leu Leu Tyr Gly Thr Asn Pro Leu Ser Arg Leu Lys Leu Asp
   545 550 555 560
   Asn Gly Lys Leu Ala Gly Ile Ile Ala Gln Arg Met Phe Glu Met Leu
   565 570 575
   Pro Cys Gly Gly Val Gly Val Asp Thr Asp Thr Ile Trp Asn Glu Leu
   580 585 590
   His Ser Ser Asn Ala Ala Arg Trp Ala Ala Gly Ser Val Thr Asp Leu
   595 600 605
   Ala Phe Lys Val Ala Ser Arg Glu Leu Lys Asn Gly Phe Ala Val Val
   610 615 620
   Arg Pro Pro Gly His His Ala Asp His Ser Thr Ala Met Gly Phe Cys
   625 630 635 640
   Phe Phe Asn Ser Val Ala Ile Ala Cys Arg Gln Leu Gln Gln Gln Ser
   645 650 655
   Lys Ala Ser Lys Ala Ser Lys Ile Leu Ile Val Asp Trp Asp Val His
   660 665 670
   His Gly Asn Gly Thr Gln Gln Thr Phe Tyr Gln Asp Pro Ser Val Leu
   675 680 685
   Tyr Ile Ser Leu His Arg His Asp Asp Gly Asn Phe Phe Pro Gly Ser
   690 695 700
   Gly Ala Val Asp Glu Val Gly Ala Gly Ser Gly Glu Gly Phe Asn Val
   705 710 715 720
   Asn Val Ala Trp Ala Gly Gly Leu Asp Pro Pro Met Gly Asp Pro Glu
   725 730 735
   Tyr Leu Ala Ala Phe Arg Ile Val Val Met Pro Ile Ala Arg Glu Phe
   740 745 750
   Ser Pro Asp Leu Val Leu Val Ser Ala Gly Phe Asp Ala Ala Glu Gly
   755 760 765
   His Pro Ala Pro Leu Gly Gly Tyr His Val Ser Ala Lys Cys Phe Gly
   770 775 780
   Tyr Met Thr Gln Gln Leu Met Asn Leu Ala Gly Gly Ala Val Val Leu
   785 790 795 800
   Ala Leu Glu Gly Gly His Asp Leu Thr Ala Ile Cys Asp Ala Ser Glu
                   805                 810                 815
   Ala Cys Val Ala Ala Leu Leu Gly Asn Arg Val Asp Pro Leu Ser Glu
               820                 825                 830
   Glu Gly Trp Lys Gln Lys Pro Gln Pro Gln Cys His Pro Leu Ser Gly
           835                 840                 845
   Gly Arg Asp Pro Gly Ala Gln
       850                 855

<210>  14
<211>  3131
<212>  DNA
<213>  Human

<400>  14
   ataataccta ccttgcagga ccacgacagg attaagtgag gaaaaacccc catgagagtg      60
   ttttgccatt gtcaagtgag cctgagggag gctgaggggg gatcaggctg tatcatgccc     120
   ccgaggacaa actttccagt ttaccctgct ccctctctct gtccctaggc tgccccaggc     180
   cctgcgcaga cacaccaggc cctcagccgc agcccatgga cctgcgggtg ggccagcggc     240
   ccccagtgga gcccccacca gagcccacat tgctggccct gcagcgtccc cagcgcctgc     300
   accaccacct cttcctagca ggcctgcagc agcagcgctc ggtggagccc atgaggctct     360
   ccatggacac gccgacgccc gagttgcagg tgggacccca ggaacaagag ctgcggcagc     420
   ttctccacaa ggacaagagc aagcgaagtg ctgtagccag cagcgtggtc aagcagaagc     480
   tagcggaggt gattctgaaa aaacagcagg cggccctaga aagaacagtc catcccaaca     540
   gccccggcat tccctacaga accccggagc ccctggagac ggaaggagcc acccgctcca     600
   tgctcagcag ccttccgcct cctgctccca gcccgcccag tgacccccca gagcactccc     660
   ctctgcgcaa gacagtctct gagcccaacc tgaagctgcg ccataagccc aagaagtccc     720
   cggagcggag gaagaatcca ctgctccgaa aggagagtgc gccccccagc ccccggcggc     780
   ggcccgcaga gaccctcgga gactcctccc caagtagtag cagcacgccc gcatcagggt     840
   gcagtccccc caatgacagc gagcacggcc ccaatcccat cctgggcgac agtgaccgca     900
   ggacccatcc gactctgggc ccccgggggc caatcctggg gagcccccac actcccctct     960
   tcctgcccca tggcttggag cccgaggctg gggcaccctt gccctcccgc ctgcagccca    1020
   ttcctctcct ggacccctca ggctctcatg ccccgctgct gactgtgccc gggcttgggc 1080
   ccttgccctt ccactttgcc cagtccttaa tgaccaccga gcggctctct gggtcaggcc 1140
   tccactggcc actgagccgg actcgctcag agcccctgcc ccccagtgcc accgctcccc 1200
   caccgccggg ccccatgcag ccccgcctgg agcagctcaa aactcacgtc caggtgatca 1260
   agaggtcagc caagccgagt gagaagcccc ggctgcggca gataccctcg gctgaagacc 1320
   tggagacaga tggcggggga ccgggccagg tggtggacga cggcccggag cacagggagc 1380
   tgggccatgg gcagcccgag gccagaggcc ccgctcctct ccagcagcac cctcaggtgt 1440
   tgctctggga acagcagcga ctggctgggc ggctcccccg gggcagcacc ggggacactg 1500
   tgctgcttcc tctggcccag ggtgggcacc ggcctctgtc ccgggctcag tcttccccag 1560
   ccgcacctgc ctcactgtca gccccagagc ctgccagcca ggcccgagtc ctctccagct 1620
   cagagacccc tgccaggacc ctgcccttca ccacagggct gatctatgac tcggtcatgc 1680
   tgaagcacca gtgctcctgc ggtgacaaca gcaggcaccc ggagcacgcc ggccgcatcc 1740
   agagcatctg gtcccggctg caggagcggg ggcctcggag ccagtgtgag tgtctccgag 1800
   gccggaaggc ctccctggaa gagctgcagt cggtccactc tgagcggcac gtgctcctct 1860
   acggcaccaa cccgctcagc cgcctcaaac tggacaacgg gaagctggca gggctcctgg 1920
   cacagcggat gtttgagatg ctgccctgtg gtggggttgg ggtggacact gacaccatct 1980
   ggaatgagct tcattcctcc aatgcagccc gctgggccgc tggcagtgtc actgacctcg 2040
   ccttcaaagt ggcttctcgt gagctaaaga atggtttcgc tgtggtgcgg cccccaggac 2100
   accatgcaga tcattcaaca gccatgggct tctgcttctt caactcagtg gccatcgcct 2160
   gccggcagct gcaacagcag agcaaggcca gcaaggccag caagatcctc attgtagact 2220
   gggacgtgca ccatggcaac ggcacccagc aaaccttcta ccaagacccc agtgtgctct 2280
   acatctccct gcatcgccat gacgacggca acttcttccc ggggagtggg gctgtggatg 2340
   aggtaggggc tggcagcggt gagggcttca atgtcaatgt ggcctgggct ggaggtctgg 2400
   acccccccat gggggatcct gagtacctgg ctgctttcag gatagtcgtg acgcccatcg 2460
   cccgagagtt ctctccagac ctagtcctgg tgtctgccgg atttgatgct gctgagggtc 2520
   acccggcccc actgggtggc taccatgttt ctgccaaatg ttttggatac atgacgcagc 2580
   aactgatgaa cctggcagga ggcgcagtgg tgctggcctt ggagggtggc catgacctca 2640
   cagccatctg tgacgcctct gaggcctgtg tggctgctct tctgggtaac agggtggatc 2700
   ccctttcaga agaaggctgg aaacagaaac ccaacctcaa tgccactcgc tctctggagg 2760
   gccgtgatcc gggtgcacag taaatactgg ggctgcatgc agcgcctggc ctcctgtcca 2820
   gactcctggg tgcctagagt gccaggggct gacaaagaag aagtggaggc agtgaccgca 2880
   ctggcgtccc tctctgtggg catcctggct gaagataggc cctcggagca gctggtggag 2940
   gaggaagaac ctatgaatct ctaaggctct ggaaccatct gcccgcccac catgcccttg 3000
   ggacctggtt ctcttctaac ccctggcaat agcccccatt cctgggtctt tagagatcct 3060
   gtgggcaagt agttggaacc agagaacagc ctgcctgctt tgacagttat cccagggagc 3120
   gtgagaaaat c 3131

<210> 15
<211> 377
<212> PRT
<213> Human

<400> 15

Met Glu Glu Pro Glu Glu Pro Ala Asp Ser Gly Gln Ser Leu Val Pro
1               5                   10                  15

Val Tyr Ile Tyr Ser Pro Glu Tyr Val Ser Met Cys Asp Ser Leu Ala
            20                  25                  30

Lys Ile Pro Lys Arg Ala Ser Met Val His Ser Leu Ile Glu Ala Tyr
        35                  40                  45

Ala Leu His Lys Gln Met Arg Ile Val Lys Pro Lys Val Ala Ser Met
    50                  55                  60

Glu Glu Met Ala Thr Phe His Thr Asp Ala Tyr Leu Gln His Leu Gln
65                  70                  75                  80

Lys Val Ser Gln Glu Gly Asp Asp Asp His Pro Asp Ser Ile Glu Tyr
                85                  90                  95

Gly Leu Gly Tyr Asp Cys Pro Ala Thr Glu Gly Ile Phe Asp Tyr Ala
            100                 105                 110

Ala Ala Ile Gly Gly Ala Thr Ile Thr Ala Ala Gln Cys Leu Ile Asp
        115                 120                 125

Gly Met Cys Lys Val Ala Ile Asn Trp Ser Gly Gly Trp His His Ala
    130                 135                 140

Lys Lys Asp Glu Ala Ser Gly Phe Cys Tyr Leu Asn Asp Ala Val Leu
145                 150                 155                 160

Gly Ile Leu Arg Leu Arg Arg Lys Phe Glu Arg Ile Leu Tyr Val Asp
                165                 170                 175

Leu Asp Leu His His Gly Asp Gly Val Glu Asp Ala Phe Ser Phe Thr
            180                 185                 190

Ser Lys Val Met Thr Val Ser Leu His Lys Phe Ser Pro Gly Phe Phe
        195                 200                 205

Pro Gly Thr Gly Asp Val Ser Asp Val Gly Leu Gly Lys Gly Arg Tyr
    210                 215                 220

Tyr Ser Val Asn Val Pro Ile Gln Asp Gly Ile Gln Asp Glu Lys Tyr
225                 230                 235                 240

Tyr Gln Ile Cys Glu Ser Val Leu Lys Glu Val Tyr Gln Ala Phe Asn
                245                 250                 255

Pro Lys Ala Val Val Leu Gln Leu Gly Ala Asp Thr Ile Ala Gly Asp
            260                 265                 270

Pro Met Cys Ser Phe Asn Met Thr Pro Val Gly Ile Gly Lys Cys Leu
        275                 280                 285

Lys Tyr Ile Leu Gln Trp Gln Leu Ala Thr Leu Ile Leu Gly Gly Gly
    290                 295                 300

Gly Tyr Asn Leu Ala Asn Thr Ala Arg Cys Trp Thr Tyr Leu Thr Gly
305                 310                 315                 320

Val Ile Leu Gly Lys Thr Leu Ser Ser Glu Ile Pro Asp Asx Glu Phe
                325                 330                 335

Phe Thr Ala Tyr Gly Pro Asp Tyr Val Leu Glu Ile Thr Pro Ser Cys
            340                 345                 350

Arg Pro Asp Arg Asn Glu Pro His Arg Ile Gln Gln Ile Leu Asn Tyr
        355                 360                 365

Ile Lys Gly Asn Leu Lys His Val Val
    370                 375

<210>  16
<211>  1654
<212>  DNA
<213>  Human
<220>
<221>  misc_feature
<222>  (1590)..(1641)
<223>  Nucleotides 1590, 1592, 1600, 1607, 1611, 1630 and 1641 are "n" w
       herein "n" = any nucleotide.

<400>  16
gaaattcggc acgagctcgt gccgaattcg gcacgagaac ggttttaagc ggaagatgga      60

ggagccggag gaaccggcgg acagtgggca gtcgctggtc ccggtttata tctatagtcc     120

cgagtatgtc agtatgtgtg actccctggc caagatcccc aaacgggcca gtatggtgca     180

ttctttgatt gaagcatatg cactgcataa gcaaatgagg atagttaagc ctaaagtggc     240

ctccatggag gagatggcca ccttccacac tgatgcttat ctgcagcatc tccagaaggt     300

cagccaagag ggcgatgatg atcatccgga ctccatagaa tatgggctag gttatgactg     360

cccagccact gaagggatat ttgactatgc agcagctata ggaggggcta cgatcacagc     420

tgcccaatgc ctgattgacg gaatgtgcaa agtagcaatc aactggtctg gagggtggca     480

tcatgcaaag aaagatgaag catctggttt tcgttatctc aatgatgctg tcctgggaat     540

attacgattg cgacggaaat ttgagcgtat tccctacgtg gattcggatc tgcaccatgg     600

agatggtgta gaagacgcat tcagtttcac ctccaaagtc atgaccgtgt ccctgcacaa     660

attctcccca ggattttcc caggaacagg tgacgtgtcc gacgttggcc tagggaaggg     720

acggtactac agtgtaaatg tgcccatcca ggatggcata caagatgaaa aatattacca     780

gatctgcgaa agtgtactaa aggaagtata ccaagccttt aatcccaaag cagtggtctt     840

acagctggga gccgacacaa tagctgggga tcccatgtgc tcctttaaca tgactccagt     900

gggaattggc aagtgtctca agtacatccc tcaatggcag ttggcaacac tcatttcggg     960

aggaggaggc tataaccttg ccaacacggc tcgatgctgg acatacttga ccggggtcat    1020

cctagggaaa acactatcct ctgagatccc agatcatgag tttttcacag catatggtcc    1080

tgattatgtg ctggaaatca cgccaagctg ccggccagac cgcaatgagc cccaccgaat 1140

ccaacaaatc ctcaactaca tcaaagggaa tctgaagcat gtggtctagt tgacagaaag 1200

agatcaggtt tccagagctg aggagtggtg cctataatga agacagcgtg tttatgcaag 1260

cagtttgrgg aatttgtgac tgcagggaaa atttgaaaga aattacttcc tgaaaatttc 1320

caaggggcat caagtggcag ctggcttcct ggggtgaaga ggcaggcacc ccagagtcct 1380

caactggacc taggggaaga aggagatarc ccacatttaa agttcttatt taaaaaaaca 1440

cacacacaca aatgaaattt ttaatctttg aaaattattt ttaagcgaat tggggagggg 1500

agtattttaa tcatcttaaa tgaaacagat cagaagctgg atgagagcag tcaccagttt 1560

gtagggcagg aggcagctga caggcagggn tngggcctcn ggaccancca ngtggagccc 1620

tgggagagan ggtactgatc ngcagactgg gagg 1654

<210> 17
<211> 20
<212> DNA
<213> Human

<400> 17
gaaacgtgag ggactcagca 20

<210> 18
<211> 20
<212> DNA
<213> Human

<400> 18
ggaagccaga gctggagagg 20

<210> 19
<211> 20
<212> DNA
<213> Human

<400> 19
gttaggtgag gcactgagga 20

<210> 20
<211> 20
<212> DNA
<213> Human

<400> 20
gctgagctgt tctgatttgg 20

<210> 21
<211> 20
<212> DNA
<213> Human

<400> 21
cgtgagcact tctcatttcc 20

<210> 22
<211> 20
<212> DNA
<213> Human

<400> 22
cgctttcctt gtcattgaca 20

<210> 23
<211> 20
<212> DNA
<213> Human

<400> 23
gcctttccta ctcattgtgt 20

<210> 24
<211> 20
<212> DNA
<213> Human

<400> 24
gctgcctgcc gtgcccaccc 20

<210> 25
<211> 20
<212> DNA
<213> Human

<400> 25
cgtgcctgcg ctgcccacgg 20

<210> 26

<211>  20
<212>  DNA
<213>  Human

<400>  26
tacagtccat gcaacctcca                                                    20

<210>  27
<211>  20
<212>  DNA
<213>  Human

<400>  27
atcagtccaa ccaacctcgt                                                    20

<210>  28
<211>  20
<212>  DNA
<213>  Human

<400>  28
cttcggtctc acctgcttgg                                                    20

<210>  29
<211>  20
<212>  DNA
<213>  Human

<400>  29
caggctggaa tgagctacag                                                    20

<210>  30
<211>  20
<212>  DNA
<213>  Human

<400>  30
gacgctgcaa tcaggtagac                                                    20

<210>  31
<211>  20
<212>  DNA
<213>  Human

<400>  31
cttcagccag gatgcccaca                                                    20

<210> 32
<211> 20
<212> DNA
<213> Human

<400> 32
ctccggctcc tccatcttcc 20

<210> 33
<211> 20
<212> DNA
<213> Human

<400> 33
agccagctgc cacttgatgc 20

## Description

### BACKGROUND OF THE INVENTION

### Field of the Invention

This invention relates to the fields of inhibition of histone deacetylase expression and enzymatic activity.

### Summary of the Related Art

In eukaryotic cells, nuclear DNA associates with histones to form a compact complex called chromatin. The histones constitute a family of basic proteins which are generally highly conserved across eukaryotic species. The core histones, termed H2A, H2B, H3, and H4, associate to form a protein core. DNA winds around this protein core, with the basic amino acids of the histones interacting with the negatively charged phosphate groups of the DNA. Approximately 146 base pairs of DNA wrap around a histone core to make up a nucleosome particle, the repeating structural motif of chromatin.

Csordas, Biochem. J., 286: 23-38 (1990) teaches that histones are subject to posttranslational acetylation of the epsilon-amino groups of *N-*terminal lysine residues, a reaction that is catalyzed by histone acetyl transferase (HAT1). Acetylation neutralizes the positive charge of the lysine side chain, and is thought to impact chromatin structure. Indeed, Taunton et al., Science, 272: 408-411 (1996), teaches that access of transcription factors to chromatin templates is enhanced by histone hyperacetylation. Taunton *et al.* further teaches that an enrichment in underacetylated histone H4 has been found in transcriptionally silent regions of the genome.

Recently, there has been interest in the role of histone deacetylase (HDAC) in gene expression. Sanches Del Pino et al., Biochem. J.303: 723-729 (1994) discloses a partially purified yeast HDAC activity. Taunton *et al.* (*supra*) discloses a human HDAC that is related to a yeast transcriptional regulator and suggests that this protein may be a key regulator of eukaryotic transcription.

Known inhibitors of mammalian HDAC have been used to probe the role of HDAC in gene regulation. Yoshida et al., J. Biol. Chem265: 17174-17179 (1990) discloses that (R)-Trichostatin A (TSA) is a potent inhibitor of mammalian HDAC. Yoshida et al, Cancer Research47:3688-3691 (1987) discloses that TSA is a potent inducer of differentiation in murine erythroleukemia cells.

More recently, it has been discovered that the HDAC activity is actually provided by a set of discrete HDAC enzyme isoforms. Grozinger et al., Proc. Natl. Acad. Sci. (USA),96: 4868-4873 (1999), teaches that HDACs may be divided into two classes, the first represented by yeast Rpd3-like proteins, and the second represented by yeast Hdal-like proteins. Grozinger *et al.* also teaches that the human HDAC1, HDAC2, and HDAC3 proteins are members of the first class of HDACs, and discloses new proteins, named HDAC4, HDAC5, and HDAC6, which are members of the second class of HDACs. Kao et al., Gene & Development 14: 55-66 (2000), discloses an additional member of this second class, called HDAC-7. More recently, Hu, E. et al. J. Bio. Chem.275:15254-13264 (2000) disclosed the newest member of the first class of histone deacetylases, HDAC-8. It has been unclear what roles these individual HDAC enzymes play.

The known inhibitors of histone deacetylase are all small molecules that inhibit histone deacetylase activity at the protein level. Moreover, all of the known histone deacetylase inhibitors are non-specific for a particular histone deacetylase isoform, and more or less inhibit all members of both the histone deacetylase families equally.

Therefore, there remains a need to develop reagents for inhibiting specific histone deacetylase isoforms. There is also a need for the development of methods for using these reagents to identify and inhibit specific histone deacetylase isoforms involved in tumorigenesis.

### BRIEF SUMMARY OF THE INVENTION

The invention provides methods and reagents for inhibiting specific histone deacetylase (HDAC) isoforms by inhibiting expression at the nucleic acid level or enzymatic activity at the protein level. The invention allows the identification of and specific inhibition of specific histone deacetylase isoforms involved in tumorigenesis and thus provides a treatment for cancer. The invention further allows identification of and specific inhibition of specific HDAC isoforms involved in cell proliferation and/or differentiation and thus provides a treatment for cell proliferative and/or differentiation disorders.

The inventors have discovered new agents that inhibit specific HDAC isoforms. Accordingly, in a first aspect, the invention provides agents that inhibit one or more specific histone deacetylase isoforms but less than all histone deacetylase isoforms. Such specific HDAC isoforms include without limitation, HDAC-1, HDAC-2, HDAC-3, HDAC-4, HDAC-5, HDAC-6, HDAC-7 and HDAC-8. Non-limiting examples of the new agents include antisense oligonucleotides (oligos) and small molecule inhibitors specific for one or more HDAC isoforms but less than all HDAC isoforms.

The present inventors have surprisingly discovered that specific inhibition of HDAC-1 reverses the tumorigenic state of a transformed cell. The inventors have also surprisingly discovered that the inhibition of the HDAC-4 isoform dramatically induces growth and apoptosis arrest in cancerous cells. Thus, in certain embodiments of this aspect of the invention, the histone deacetylase isoform that is inhibited is HDAC-1 and/or HDAC-4.

In certain preferred embodiments, the agent that inhibits the specific HDAC isoform is an oligonucleotide that inhibits expression of a nucleic acid molecule encoding that histone deacetylase isoform. The nucleic acid molecule may be genomic DNA (*e.g.,* a gene), cDNA, or RNA. In some embodiments, the oligonucleotide inhibits transcription of mRNA encoding the HDAC isoform. In other embodiments, the oligonucleotide inhibits translation of the histone deacetylase isoform. In certain embodiments the oligonucleotide causes the degradation of the nucleic acid molecule. Particularly preferred embodiments include antisense oligonucleotides directed to HDAC-1 and/or HDAC-4.

In yet other embodiments of the first aspect, the agent that inhibits a specific HDAC isoform is a small molecule inhibitor that inhibits the activity of one or more specific histone deacetylase isoforms but less than all histone deacetylase isoforms.

In a second aspect, the invention provides a method for inhibiting one or more, but less than all, histone deacetylase isoforms in a cell, comprising contacting the cell with an agent of the first aspect of the invention. In other preferred embodiments, the agent is an antisense oligonucleotide. In certain preferred embodiments, the agent is a small molecule inhibitor. In other certain preferred embodiments of the second aspect of the invention, cell proliferation is inhibited in the contacted cell. In preferred embodiments, the cell is a neoplastic cell which may be in an animal, including a human, and which may be in a neoplastic growth. In certain preferred embodiments, the method of the second aspect of the invention further comprises contacting the cell with a histone deacetylase small molecule inhibitor that interacts with and reduces the enzymatic activity of one or more specific histone deacetylase isoforms. In still yet other preferred embodiments of the second aspect of the invention, the method comprises an agent of the first aspect of the invention which is a combination of one or more antisense oligonucleotides and/or one or more small molecule inhibitors of the first aspect of the invention. In certain preferred embodiments, the histone deacetylase isoform is HDAC-1, HDAC-2, HDAC-3, HDAC-4, HDAC-5, HDAC-6, HDAC-7, or HDAC-8. In other certain preferred embodiments, the histone deacetylase isoform is HDAC-1 and/or HDAC-4. In some embodiments, the histone deacetylase small molecule inhibitor is operably associated with the antisense oligonucleotide.

In a third aspect, the invention provides a method for inhibiting neoplastic cell proliferation in an animal comprising administering to an animal having at least one neoplastic cell present in its body a therapeutically effective amount of an agent of the first aspect of the invention. In certain preferred embodiments, the agent is an antisense oligonucleotide which is combined with a pharmaceutically acceptable carrier and administered for a therapeutically effective period of time. In certain preferred embodiments, the agent is a small molecule inhibitor which is combined with a pharmaceutically acceptable carrier and administered for a therapeutically effective period of time. In certain preferred embodiments of the this aspect of the invention, cell proliferation is inhibited in the contacted cell. In preferred embodiments, the cell is a neoplastic cell which may be in an animal, including a human, and which may be in a neoplastic growth. In other certain embodiments, the agent is a small molecule inhibitor of the first aspect of the invention which is combined with a pharmaceutically acceptable carrier and administered for a therapeutically effective period of time. In still yet other preferred embodiments of the third aspect of the invention, the method comprises an agent of the first aspect of the invention which is a combination of one or more antisense oligonucleotides and/or one or more small molecule inhibitors of the first aspect of the invention. In certain preferred embodiments, the histone deacetylase isoform is HDAC-1, HDAC-2, HDAC-3, HDAC-4, HDAC-5, HDAC-6, HDAC-7, or HDAC-8. In other certain preferred embodiments, the histone deacetylase isoform is HDAC-1 and/or HDAC-4.

In a fourth aspect, the invention provides a method for identifying a specific histone deacetylase isoform that is required for induction of cell proliferation comprising contacting a cell with an agent of the first aspect of the invention. In certain preferred embodiments, the agent is an antisense oligonucleotide that inhibits the expression of a histone deacetylase isoform, wherein the antisense oligonucleotide is specific for a particular HDAC isoform, and thus inhibition of cell proliferation in the contacted cell identifies the histone deacetylase isoform as a histone deacetylase isoform that is required for induction of cell proliferation. In other certain embodiments, the agent is a small molecule inhibitor that inhibits the activity of a histone deacetylase isoform, wherein the small molecule inhibitor is specific for a particular HDAC isoform, and thus inhibition of cell proliferation in the contacted cell identifies the histone deacetylase isoform as a histone deacetylase isoform that is required for induction of cell proliferation. In certain preferred embodiments, the cell is a neoplastic cell, and the induction of cell proliferation is tumorigenesis. In still yet other preferred embodiments of the fourth aspect of the invention, the method comprises an agent of the first aspect of the invention which is a combination of one or more antisense oligonucleotides and/or one or more small molecule inhibitors of the first aspect of the invention. In certain preferred embodiments, the histone deacetylase isoform is HDAC-1, HDAC-2, HDAC-3, HDAC-4, HDAC-5, HDAC-6, HDAC-7, or HDAC-8. In other certain preferred embodiments, the histone deacetylase isoform is HDAC-1 and/or HDAC-4.

In an fifth aspect, the invention provides a method for identifying a histone deacetylase isoform that is involved in induction of cell differentiation, comprising contacting a cell with an agent that inhibits the expression of a histone deacetylase isoform, wherein induction of differentiation in the contacted cell identifies the histone deacetylase isoform as a histone deacetylase isoform that is involved in induction of cell differentiation. In certain preferred embodiments, the agent is an antisense oligonucleotide of the first aspect of the invention. In other certain preferred embodiments, the agent is an small molecule inhibitor of the first aspect of the invention. In still other certain embodiments, the cell is a neoplastic cell. In still yet other preferred embodiments of the fifth aspect of the invention, the method comprises an agent of the first aspect of the invention which is a combination of one or more antisense oligonucleotides and/or one or more small molecule inhibitors of the first aspect of the invention. In certain preferred embodiments, the histone deacetylase isoform is HDAC-1, HDAC-2, HDAC-3, HDAC-4, HDAC-5, HDAC-6, HDAC-7, or HDAC-8. In other certain preferred embodiments, the histone deacetylase isoform is HDAC-1 and/or HDAC-4.

In a sixth aspect, the invention provides a method for inhibiting neoplastic cell growth in an animal comprising administering to an animal having at least one neoplastic cell present in its body a therapeutically effective amount of an agent of the first aspect of the invention. In certain embodiments thereof, the agent is an antisense oligonucleotide, which is combined with a pharmaceutically acceptable carrier and administered for a therapeutically effective period of time.

In an seventh aspect, the invention provides a method for identifying a histone deacetylase isoform that is involved in induction of cell differentiation, comprising contacting a cell with an antisense oligonucleotide that inhibits the expression of a histone deacetylase isoform, wherein induction of differentiation in the contacted cell identifies the histone deacetylase isoform as a histone deacetylase isoform that is involved in induction of cell differentiation. Preferably, the cell is a neoplastic cell. In certain preferred embodiments, the histone deacetylase isoform is HDAC-1, HDAC-2, HDAC-3, HDAC-4, HDAC-5, HDAC-6, HDAC-7, or HDAC-8. In other certain preferred embodiments, the histone deacetylase isoform is HDAC-1 and/or HDAC-4.

In an eighth aspect, the invention provides a method for inhibiting cell proliferation in a cell comprising contacting a cell with at least two reagents selected from the group consisting of an antisense oligonucleotide from the first aspect of the invention that inhibits expression of a specific histone deacetylase isoform, a small molecule inhibitor from the first aspect of the invention that inhibits a specific histone deacetylase isoform, an antisense oligonucleotide that inhibits a DNA methyltransferase, and a small molecule that inhibits a DNA methyltransferase. In one embodiment, the inhibition of cell growth of the contacted cell is greater than the inhibition of cell growth of a cell contacted with only one of the reagents. In certain embodiments, each of the reagents selected from the group is substantially pure. In preferred embodiments, the cell is a neoplastic cell. In yet additional preferred embodiments, the reagents selected from the group are operably associated. In certain preferred embodiments, the histone deacetylase isoform is HDAC-1, HDAC-2, HDAC-3, HDAC-4, HDAC-5, HDAC-6, HDAC-7, or HDAC-8. In other certain preferred embodiments, the histone deacetylase isoform is HDAC-1 and/or HDAC-4.

In a ninth aspect, the invention provides a method for modulating cell proliferation or differentiation, comprising contacting a cell with an agent of the first aspect of the invention, wherein one or more, but less than all, HDAC isoforms are inhibited, which results in a modulation of proliferation or differentiation. In certain embodiments, the agent is an antisense oligonucleotide of the first aspect of the invention. In other certain preferred embodiments, the agent is a small molecule inhibitor of the first aspect of the invention. In preferred embodiments, the cell proliferation is neoplasia. In still yet other preferred embodiments of the this aspect of the invention, the method comprises an agent of the first aspect of the invention which is a combination of one or more antisense oligonucleotides and/or one or more small molecule inhibitors of the first aspect of the invention. In certain preferred embodiments, the histone deacetylase isoform is HDAC-1, HDAC-2, HDAC-3, HDAC-4, HDAC-5, HDAC-6, HDAC-7, or HDAC-8. In other certain preferred embodiments, the histone deacetylase isoform is HDAC-1 and/or HDAC-4.

### BRIEF DESCRIPTION OF THE DRAWINGS

Figure 1A is a schematic diagram providing the amino acid sequence of HDAC-1, as provided in GenBank Accession No. AAC50475 (SEQ ID NO:1).
Figure 1B is a schematic diagram providing the nucleic acid sequence of HDAC-1, as provided in GenBank Accession No. U50079 (SEQ ID NO:2).
Figure 2A is a schematic diagram providing the amino acid sequence of HDAC-2, as provided in GenBank Accession No. AAC50814 (SEQ ID NO:3).
Figure 2B is a schematic diagram providing the nucleic acid sequence of HDAC-2, as provided in GenBank Accession No. U31814 (SEQ ID NO:4).
Figure 3A is a schematic diagram providing the amino acid sequence of HDAC-3, as provided in GenBank Accession No. AAB88241 (SEQ ID NO:5).
Figure 3B is a schematic diagram providing the nucleic acid sequence of HDAC-3, as provided in GenBank Accession No. U75697 (SEQ ID NO:6).
Figure 4A is a schematic diagram providing the amino acid sequence of HDAC-4, as provided in GenBank Accession No. BAA22957 (SEQ ID NO:7).
Figure 4B is a schematic diagram providing the nucleic acid sequence of HDAC-4, as provided in GenBank Accession No. AB006626 (SEQ ID NO.8).
Figure 5A is a schematic diagram providing the amino acid sequence of HDAC-5, as provided in GenBank Accession No. BAA25526 (SEQ ID NO:9).
Figure 5B is a schematic diagram providing the nucleic acid sequence of HDAC-5 as provided in GenBank Accession No. AB011172 (SEQ ID NO:10).
Figure 6A is a schematic diagram providing the amino acid sequence of human HDAC-6, as provided in GenBank Accession No. AAD29048 (SEQ ID NO:11).
Figure 6B is a schematic diagram providing the nucleic acid sequence of human HDAC-6, as provided in GenBank Accession No. AJ011972 (SEQ ID NO:12).
Figure 7A is a schematic diagram providing the amino acid sequence of human HDAC-7, as provided in GenBank Accession No. AAF63491.1 (SEQ ID NO:13).
Figure 7B is a schematic diagram providing the nucleic acid sequence of human HDAC-7, as provided in GenBank Accession No. AF239243 (SEQ ID NO:14).
Figure 8A is a schematic diagram providing the amino acid sequence of human HDAC-8, as provided in GenBank Accession No. AAF73076.1 (SEQ ID NO:15).
Figure 8B is a schematic diagram providing the nucleic acid sequence of human HDAC-8, as provided in GenBank Accession No. AF230097 (SEQ ID NO:16).
Figure 9A is a representation of a Northern blot demonstrating the effect of HDAC-1 AS1 antisense oligonucleotide on HDAC-1 mRNA expression in human A549 cells.

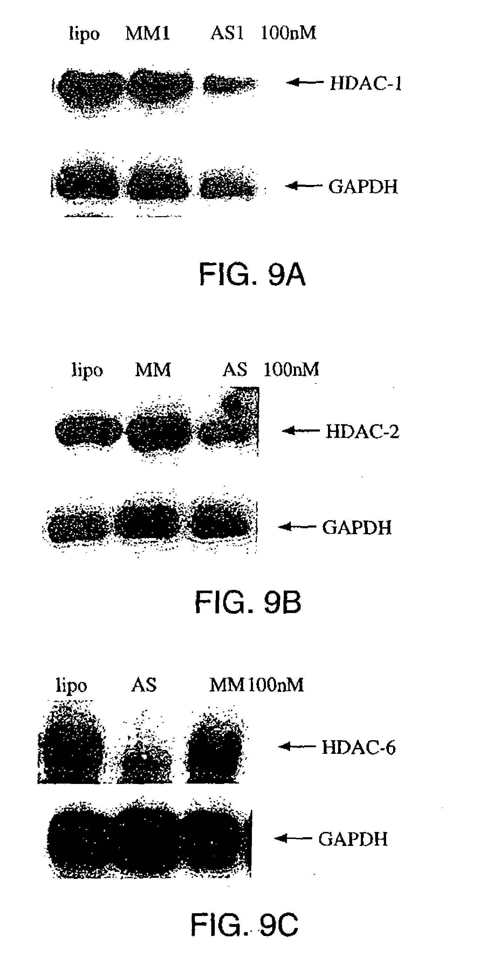

Figure 9A is a representation of a Northern blot demonstrating the effect of HDAC-2 AS antisense oligonucleotide on HDAC-2 mRNA expression in human A549 cells.
Figure 9C is a representation of a Northern blot demonstrating the effect of HDAC-6 AS antisense oligonucleotide on HDAC-6 mRNA expression in human A549 cells.

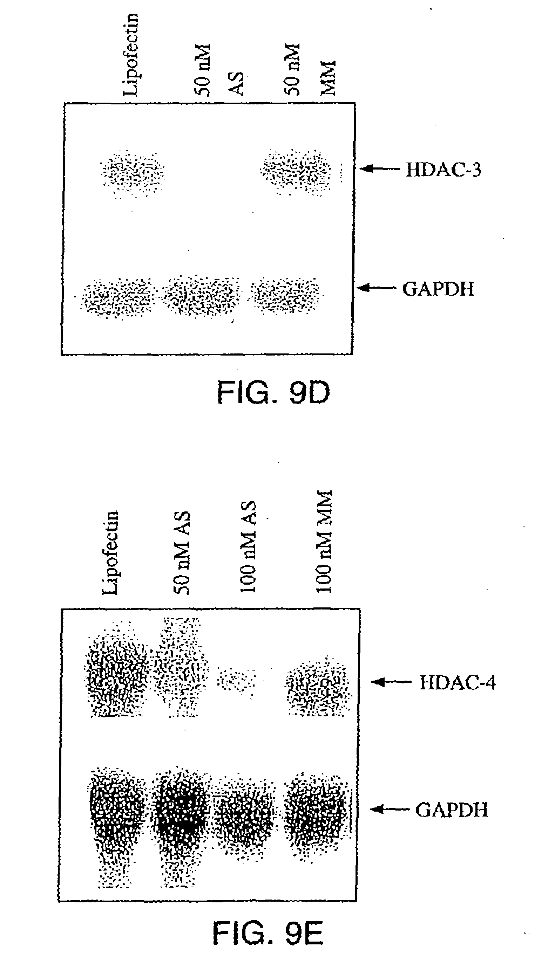

Figure 9D is a representation of a Northern blot demonstrating the effect of HDAC-3 AS antisense oligonucleotide on HDAC-3 mRNA expression in human A549 cells.
Figure 9E is a representation of a Northern blot demonstrating the effect of an HDAC-4 antisense oligonucleotide (AS1) on HDAC-4 mRNA expression in human A549 cells.

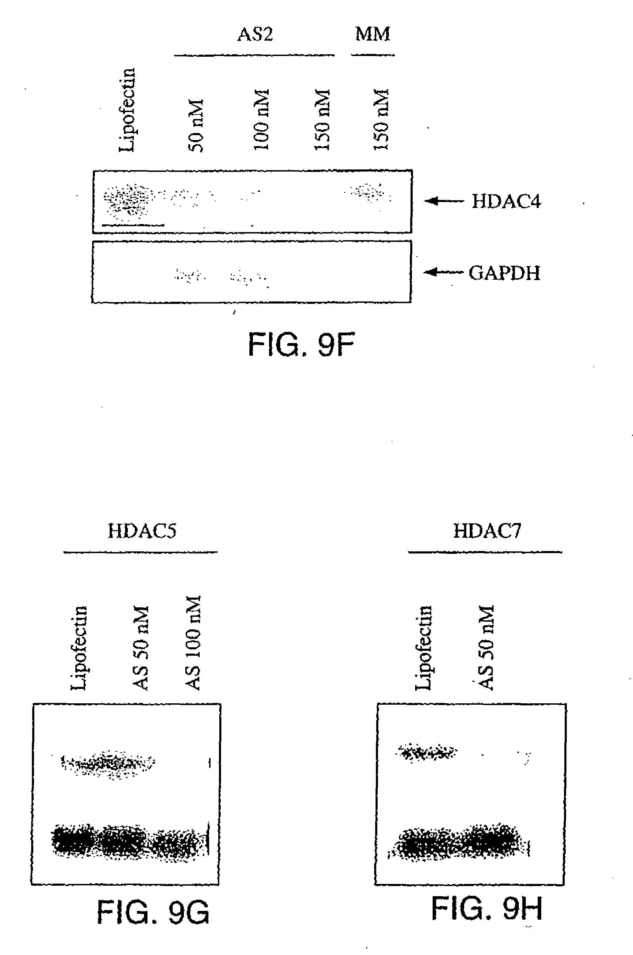

Figure 9F is a representation of a Northern blot demonstrating the dose-dependent effect of an HDAC-4 antisense oligonucleotide (AS2) on HDAC-4 mRNA expression in human A549 cells.
Figure 9G is a representation of a Northern blot demonstrating the effect of an HDAC-5 antisense oligonucleotide (AS) on HDAC-5 mRNA expression in human A549 cells.
Figure 9H is a representation of a Northern blot demonstrating the effect of an HDAC-7 antisense oligonucleotide (AS) on HDAC-7 mRNA expression in human A549 cells.

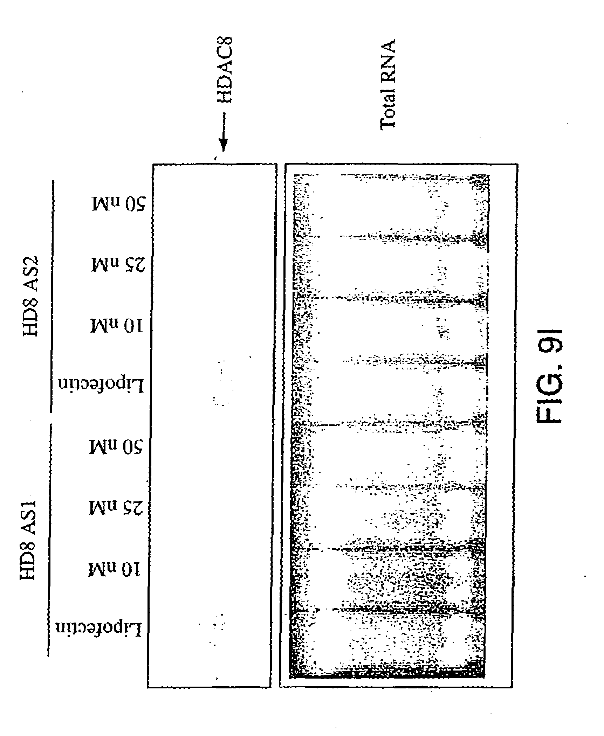

Figure 9I is a representation of a Northern blot demonstrating the dose-dependent effect of HDAC-8 antisense oligonucleotides (AS1 and AS2) on HDAC-8 mRNA expression in human A549 cells.

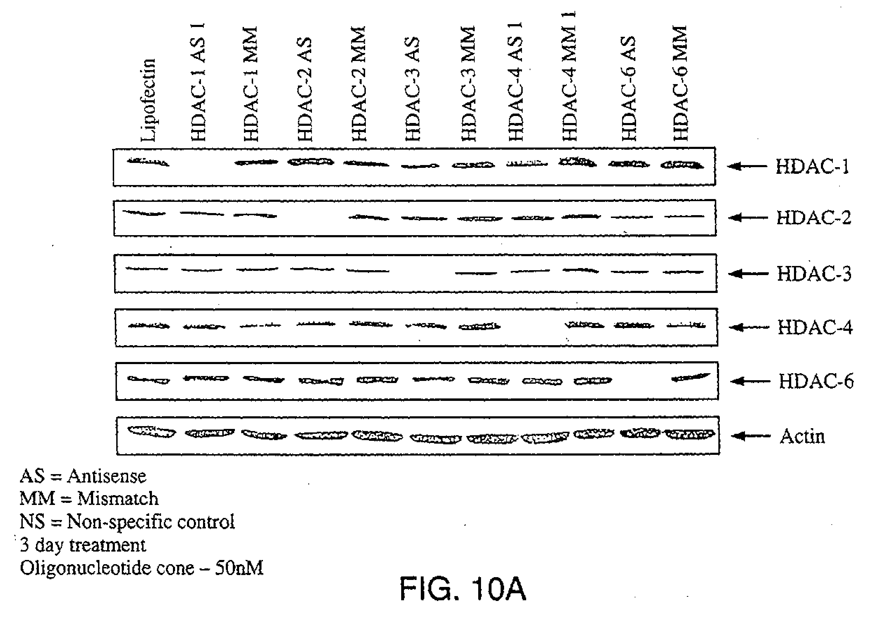

Figure 10A is a representation of a Western blot demonstrating the effect of HDAC isotype-specific antisense oligos on HDAC isotype protein expression in human A549 cells.

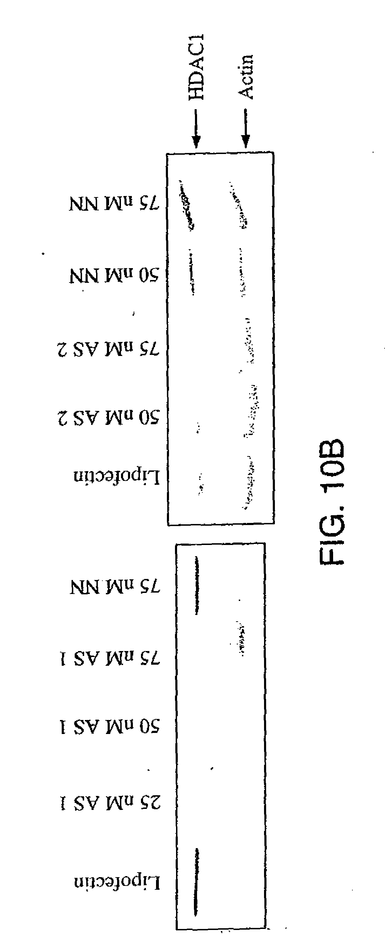

Figure 10B is a representation of a Western blot demonstrating the dose-dependent effect of the HDAC-1 isotype-specific antisense oligo (AS1 and AS2) on HDAC isotype protein expression in human A549 cells.
Figure 10C is a representation of a Western blot demonstrating the effect of HDAC-4 isotype-specific antisense oligonucleotide (AS2) on HDAC isotype protein expression in human A549 cells.

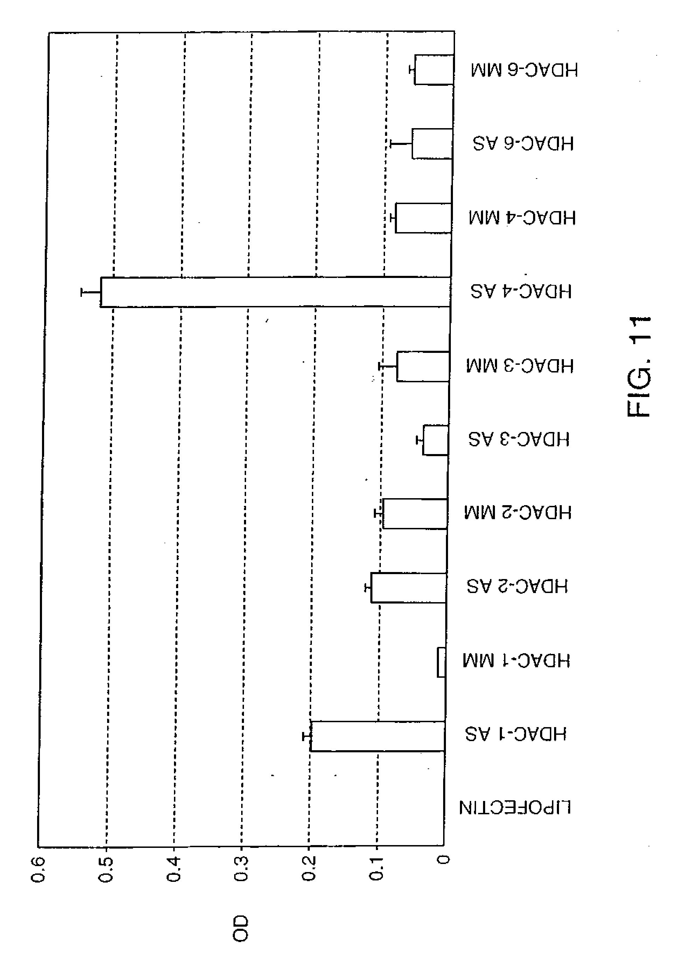

Figure 11A is a graphic representation demonstrating the apoptotic effect of HDAC isotype-specific antisense oligos on human A549 cancer cells.

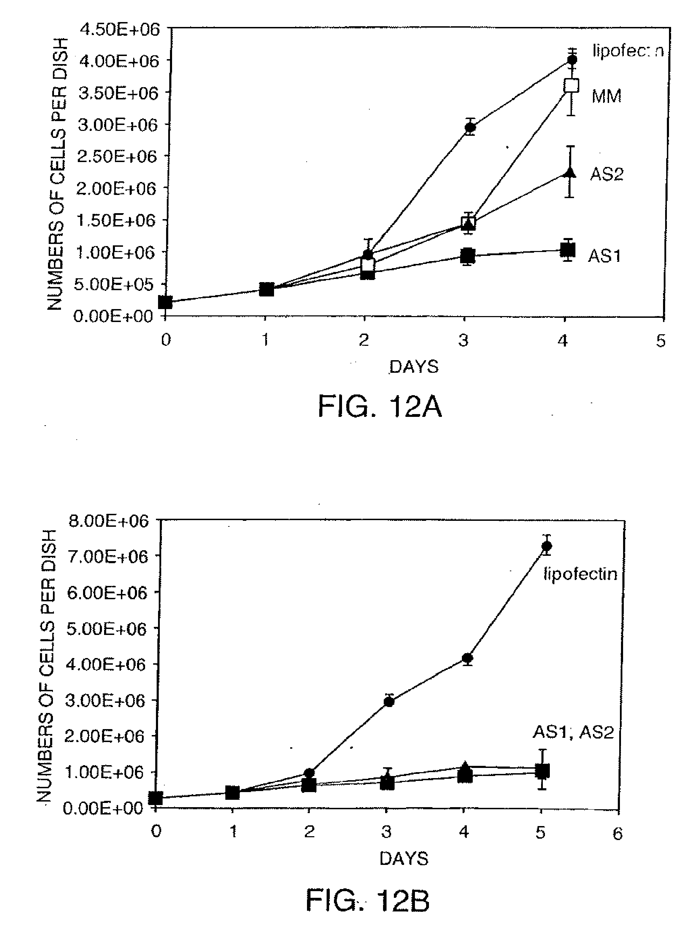

Figure 12A is a graphic representation demonstrating the effect of HDAC-1 AS1 and AS2 antisense oligonucleotides on the proliferation of human A549 cancer cells.
Figure 12B is a graphic representation demonstrating the effect of HDAC-8 specific AS1 and AS2 antisense oligonucleotides on the proliferation of human A549 cancer cells.

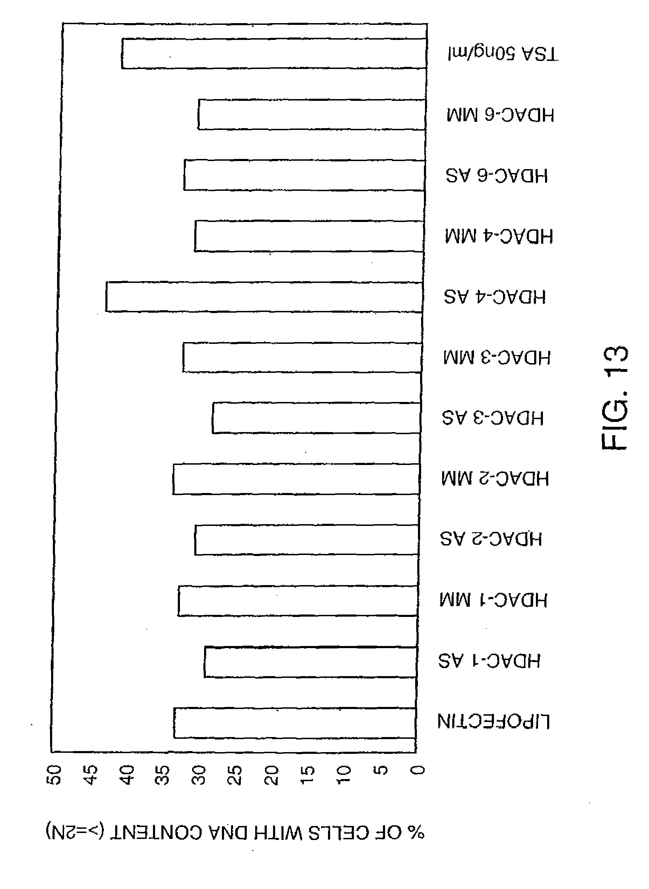

Figure 13 is a a graphic representation demonstrating the cell cycle blocking effect of HDAC specific antisense oligonucleotides on human A549 cancer cells.

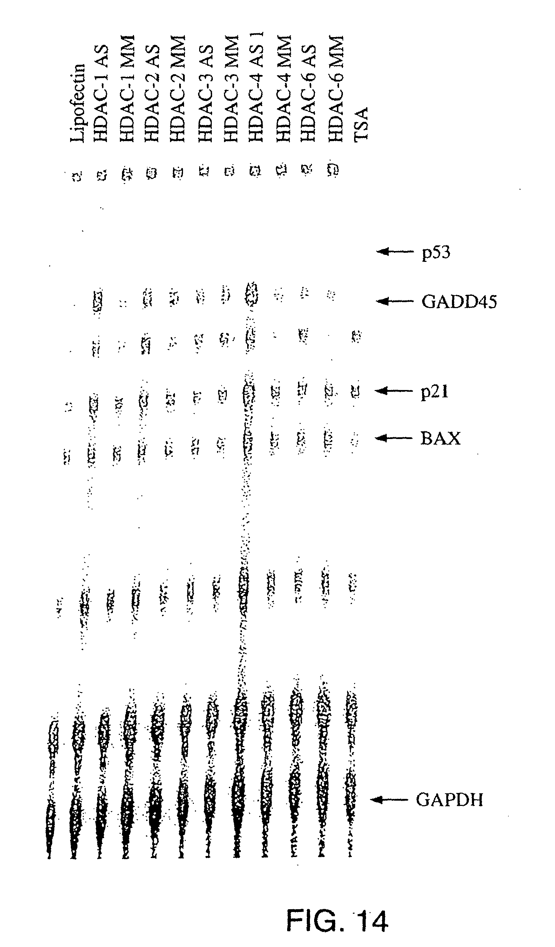

Figure 14 is a representation of an RNAse protection assay demonstrating the effect of HDAC isotype-specific antisense oligonucleotides on HDAC isotype mRNA expression in human A549 cells.

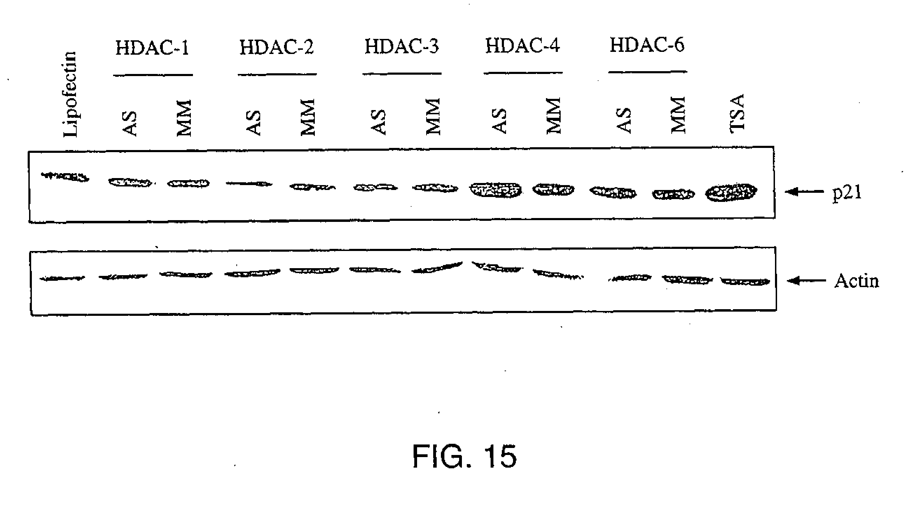

Figure 15 is a representation of a Western blot demonstrating that treatment of human A549 cells with HDAC-4 AS1 antisense oligonucleotide induces the expression of the p21 protein.

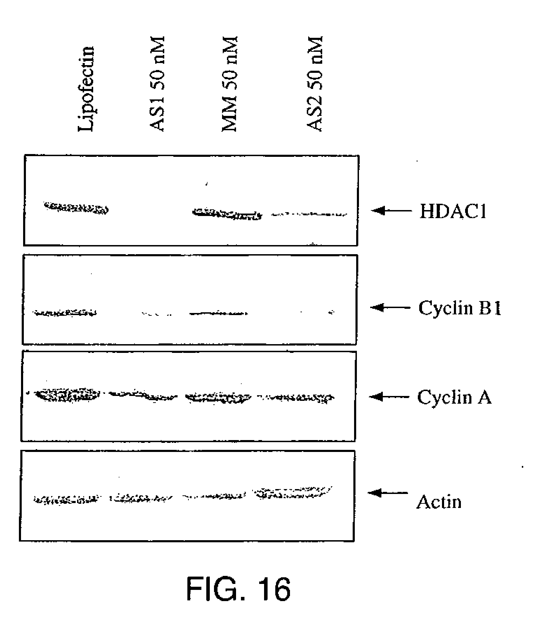

Figure 16 is a representation of a Western blot demonstrating that treatment of human A549 cells with HDAC-1 antisense oligonucleotides (AS1 and AS2) represses the expression of the cyclin B1 and cyclin A genes.

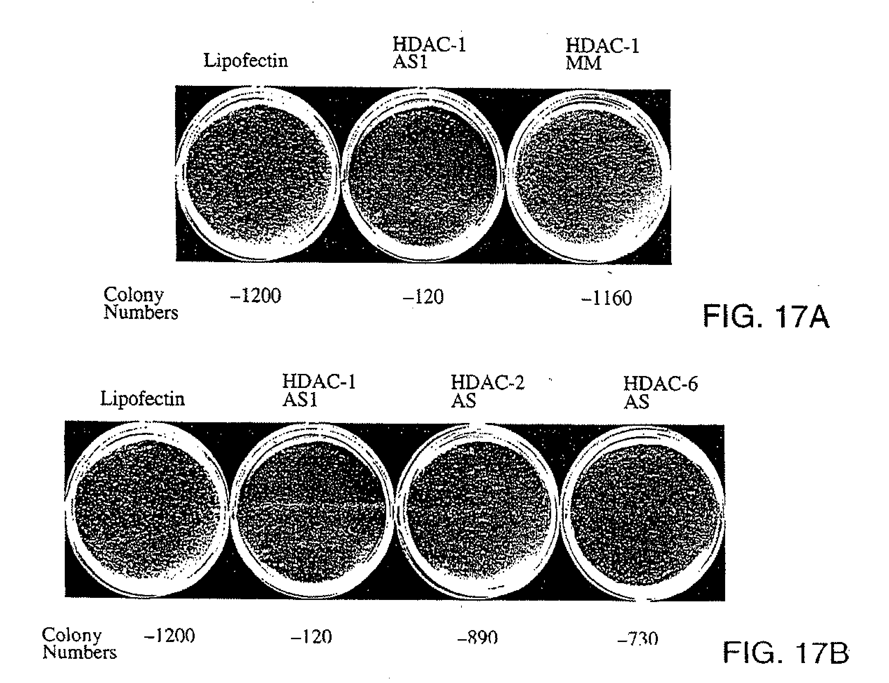

Figure 17 shows plating data demonstrating the ability of antisense oligonucleotides complementary to HDAC-1 to inhibit growth in soft agar of A549 cells far more than can antisense oligonucleotides complementary to HDAC-2, HDAC-6 or mismatched controls.
Figure 18 is a representation of a Western blot demonstrating that treatment of human A549 cells with the small molecule inhibitor Compound 3 (Table 2) induces the expression of the p21 protein and represses the expression of the cyclin B1 and cyclin A genes.

### DETAILED DESCRIPTION OF THE PREFERRED EMBODIMENTS

The invention provides methods and reagents for inhibiting specific histone deacetylase isoforms (HDAC) by inhibiting expression at the nucleic acid level or protein activity at the enzymatic level. The invention allows the identification of and specific inhibition of specific histone deacetylase isoforms involved in tumorigenesis and thus provides a treatment for cancer. The invention further allows identification of and specific inhibition of specific HDAC isoforms involved in cell proliferation and/or differentiation and thus provides a treatment for cell proliferative and/or differentiation disorders.

The patent and scientific literature referred to herein establishes knowledge that is available to those with skill in the art. The issued patents, applications, and references, including GenBank database sequences, that are cited herein are hereby incorporated by reference to the same extent as if each was specifically and individually indicated to be incorporated by reference.

In a first aspect, the invention provides agents that inhibit one or more histone deacetylase isoform, but less than all specific histone deacetylase isoforms. As used herein interchangeably, the terms "histone deacetylase", "HDAC", "histone deacetylase isoform", "HDAC isoform" and similar terms are intended to refer to any one of a family of enzymes that remove acetyl groups from the epsilon-amino groups of lysine residues at the N-terminus of a histone. Unless otherwise indicated by context, the term "histone" is meant to refer to any histone protein, including H1, H2A, H2B, H3, and H4, from any species. Preferred histone deacetylase isoforms include class I and class II enzymes. Specific HDACs include without limitation, HDAC-1, HDAC-2, HDAC-3, HDAC-4, HDAC-5, HDAC-6, HDAC-7 and HDAC-8. By way of non-limiting example, useful agents that inhibit one or more histone deacetylase isoforms, but less than all specific histone deacetylase isoforms, include antisense oligonucleotides and small molecule inhibitors.

The present inventors have surprisingly discovered that specific inhibition of HDAC-1 reverses the tumorigenic state of a transformed cell. The inventors have also surprisingly discovered that the inhibition of the HDAC-4 isoform dramatically induces growth and apoptosis arrest in cancerous cells. Thus, in certain embodiments of this aspect of the invention, the histone deacetylase isoform that is inhibited is HDAC-1 and/or HDAC-4.

Preferred agents that inhibit HDAC-1 and/or HDAC-4 dramatically inhibit growth of human cancer cells, independent of p53 status. These agents significantly induce apoptosis in the cancer cells and cause dramatic growth arrest. They also can induce transcription of tumor suppressor genes, such as p21^{WAF1}, p57^{KIP2}, GADD153 and GADD45. Finally, they exhibit both *in vitro* and *in vivo* anti-tumor activity. Inhibitory agents that achieve one or more of these results are considered within the scope of this aspect of the invention. By way of non-limiting example, antisense oligonucleotides and/or small molecule inhibitors of HDAC-1 and/or HDAC-4 are useful for the invention.

In certain preferred embodiments, the agent that inhibits the specific HDAC isoform is an oligonucleotide that inhibits expression of a nucleic acid molecule encoding a specific histone deacetylase isoform. The nucleic acid molecule may be genomic DNA (e.g., a gene), cDNA, or RNA. In other embodiments, the oligonucleotide ultimately inhibits translation of the histone deacetylase. In certain embodiments the oligonucleotide causes the degradation of the nucleic acid molecule. Preferred antisense oligonucleotides have potent and specific antisense activity at nanomolar concentrations.

The antisense oligonucleotides according to the invention are complementary to a region of RNA or double-stranded DNA that encodes a portion of one or more histone deacetylase isoform (taking into account that homology between different isoforms may allow a single antisense oligonucleotide to be complementary to a portion of more than one isoform).

For purposes of the invention, the term "complementary" means having the ability to hybridize to a genomic region, a gene, or an RNA transcript thereof under physiological conditions. Such hybridization is ordinarily the result of base-specific hydrogen bonding between complementary strands, preferably to form Watson-Crick or Hoogsteen base pairs, although other modes of hydrogen bonding, as well as base stacking can lead to hybridization. As a practical matter, such hybridization can be inferred from the observation of specific gene expression inhibition, which may be at the level of transcription or translation (or both).

For purposes of the invention, the term "oligonucleotide" includes polymers of two or more deoxyribonucleosides, ribonucleosides, or 2'-O-substituted ribonucleoside residues, or any combination thereof. Preferably, such oligonucleotides have from about 8 to about 50 nucleoside residues, and most preferably from about 12 to about 30 nucleoside residues. The nucleoside residues may be coupled to each other by any of the numerous known internucleoside linkages. Such internucleoside linkages include without limitation phosphorothioate, phosphorodithioate, alkylphosphonate, alkylphosphonothioate, phosphotriester, phosphoramidate, siloxane, carbonate, carboxymethylester, acetamidate, carbamate, thioether, bridged phosphoramidate, bridged methylene phosphonate, bridged phosphorothioate, and sulfone internucleotide linkages. In certain preferred embodiments, these internucleoside linkages may be phosphodiester, phosphotriester, phosphorothioate, or phosphoramidate linkages, or combinations thereof. The term oligonucleotide also encompasses such polymers having chemically modified bases or sugars and/or having additional substituents, including without limitation lipophilic groups, intercalating agents, diamines, and adamantane. The term oligonucleotide also encompasses such polymers as PNA and LNA. For purposes of the invention the term "2'-O-substituted" means substitution of the 2' position of the pentose moiety with an -O-lower alkyl group containing 1-6 saturated or unsaturated carbon atoms, or with an -O-aryl or allyl group having 2-6 carbon atoms, wherein such alkyl, aryl, or allyl group may be unsubstituted or may be substituted, *e.g.,* with halo, hydroxy, trifluoromethyl, cyano, nitro, acyl, acyloxy, alkoxy, carboxyl, carbalkoxyl, or amino groups; or such 2' substitution may be with a hydroxy group (to produce a ribonucleoside), an amino or a halo group, but not with a 2'-H group.

Particularly preferred antisense oligonucleotides utilized in this aspect of the invention include chimeric oligonucleotides and hybrid oligonucleotides.

For purposes of the invention, a "chimeric oligonucleotide" refers to an oligonucleotide having more than one type of internucleoside linkage. One preferred embodiment of such a chimeric oligonucleotide is a chimeric oligonucleotide comprising a phosphorothioate, phosphodiester or phosphorodithioate region, preferably comprising from about 2 to about 12 nucleotides, and an alkylphosphonate or alkylphosphonothioate region (see *e.g.,* Pederson et al. U.S. Patent Nos. 5,635,377 and 5,366,878). Preferably, such chimeric oligonucleotides contain at least three consecutive internucleoside linkages selected from phosphodiester and phosphorothioate linkages, or combinations thereof.

For purposes of the invention, a "hybrid oligonucleotide" refers to an oligonucleotide having more than one type of nucleoside. One preferred embodiment of such a hybrid oligonucleotide comprises a ribonucleotide or 2'-O-substituted ribonucleotide region, preferably comprising from about 2 to about 12 2'-O-substituted nucleotides, and a deoxyribonucleotide region. Preferably, such a hybrid oligonucleotide will contain at least three consecutive deoxyribonucleosides and will also contain ribonucleosides, 2'-O-substituted ribonucleosides, or combinations thereof (see *e*.*g*., Metelev and Agrawal, U.S. Patents Nos. 5,652,355 and 5,652,356).

The exact nucleotide sequence and chemical structure of an antisense oligonucleotide utilized in the invention can be varied, so long as the oligonucleotide retains its ability to inhibit expression of a specific histone deacetylase isoform or inhibit one or more histone deacetylase isoforms, but less than all specific histone deacetylase isoforms. This is readily determined by testing whether the particular antisense oligonucleotide is active by quantitating the amount of mRNA encoding a specific histone deacetylase isoform, quantitating the amount of histone deacetylase isoform protein, quantitating the histone deacetylase isoform enzymatic activity, or quantitating the ability of the histone deacetylase isoform to inhibit cell growth in a an *in vitro* or *in vivo* cell growth assay, all of which are described in detail in this specification. The term "inhibit expression" and similar terms used herein are intended to encompass any one or more of these parameters.

Antisense oligonucleotides utilized in the invention may conveniently be synthesized on a suitable solid support using well-known chemical approaches, including H-phosphonate chemistry, phosphoramidite chemistry, or a combination of H-phosphonate chemistry and phosphoramidite chemistry (*i.e.,* H-phosphonate chemistry for some cycles and phosphoramidite chemistry for other cycles). Suitable solid supports include any of the standard solid supports used for solid phase oligonucleotide synthesis, such as controlled-pore glass (CPG) (see, *e*.*g*., Pon, R. T., Methods in Molec. Biol. 20: 465-496, 1993).

Antisense oligonucleotides according to the invention are useful for a variety of purposes. For example, they can be used as "probes" of the physiological function of specific histone deacetylase isoforms by being used to inhibit the activity of specific histone deacetylase isoforms in an experimental cell culture or animal system and to evaluate the effect of inhibiting such specific histone deacetylase isoform activity. This is accomplished by administering to a cell or an animal an antisense oligonucleotide that inhibits one or more histone deacetylase isoform expression according to the invention and observing any phenotypic effects. In this use, the antisense oligonucleotides according to the invention is preferable to traditional "gene knockout" approaches because it is easier to use, and can be used to inhibit specific histone deacetylase isoform activity at selected stages of development or differentiation.

Preferred antisense oligonucleotides of the invention inhibit either the transcription of a nucleic acid molecule encoding the histone deacetylase isoform, and/or the translation of a nucleic acid molecule encoding the histone deacetylase isoform, and/or lead to the degradation of such nucleic acid. Histone deacetylase-encoding nucleic acids may be RNA or double stranded DNA regions and include, without limitation, intronic sequences, untranslated 5' and 3' regions, intron-exon boundaries as well as coding sequences from a histone deacetylase family member gene. For human sequences, see e.g., Yang et al., Proc. Natl. Acad. Sci. (USA) 93(23): 12845-12850, 1996; Furukawa et al., Cytogenet. Cell Genet. 73(1-2): 130-133, 1996; Yang et al., J. Biol. Chem. 272(44): 28001-28007,1997; Betz et al., Genomics 52(2): 245-246,1998; Taunton et al., Science 272(5260): 408-411, 1996; and Dangond et al., Biochem, Biophys. Res. Commun. 242(3): 648-652, 1998).

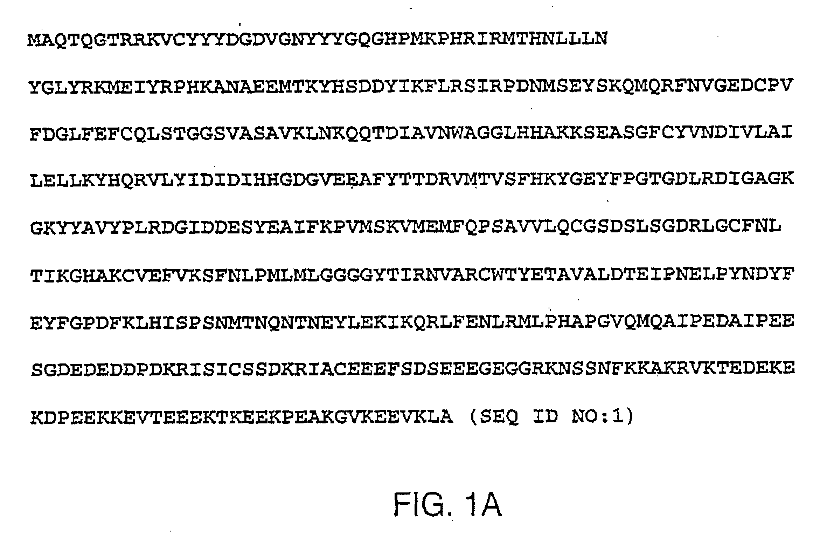

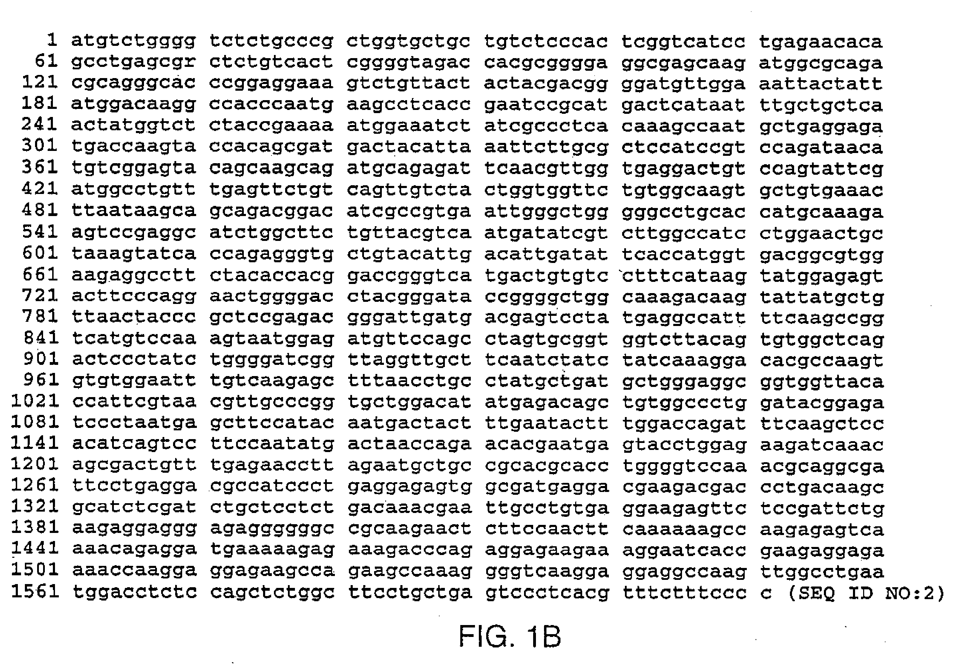

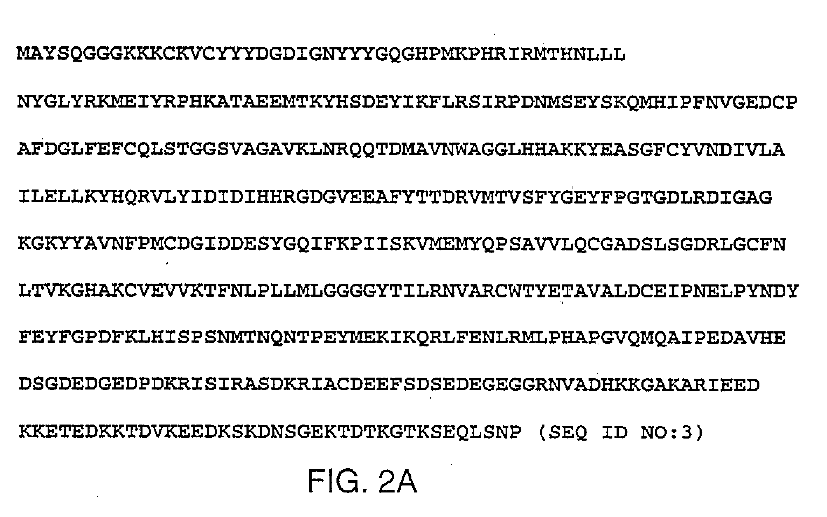

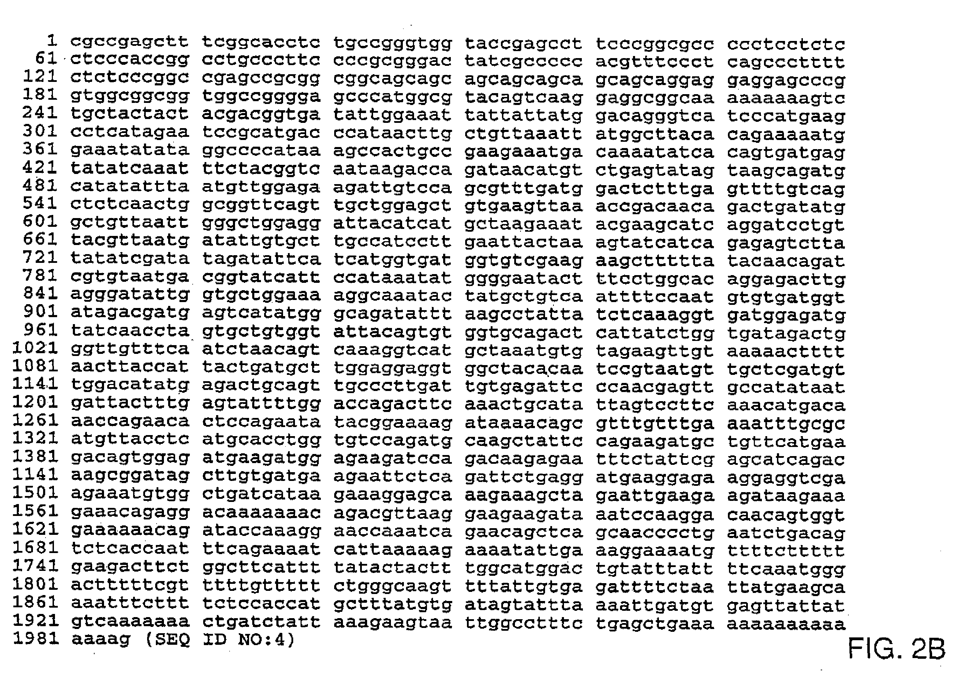

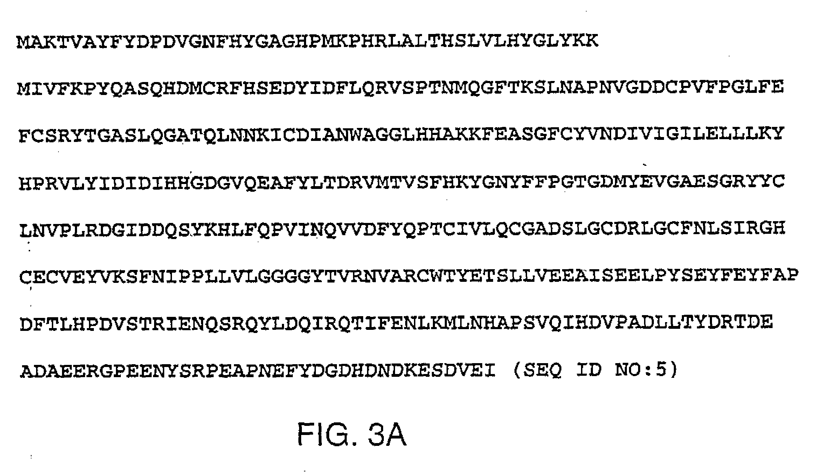

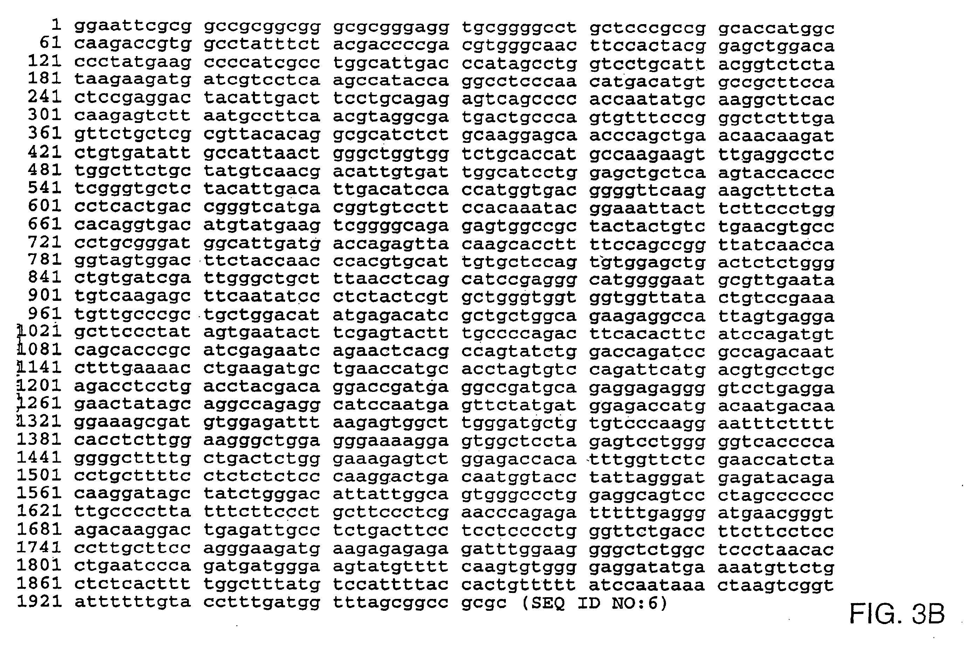

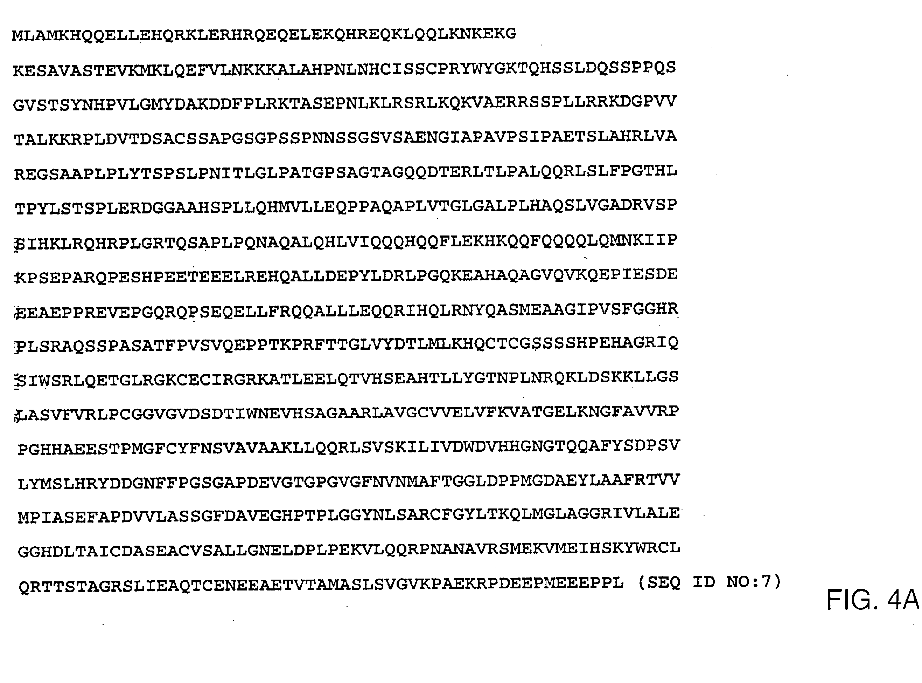

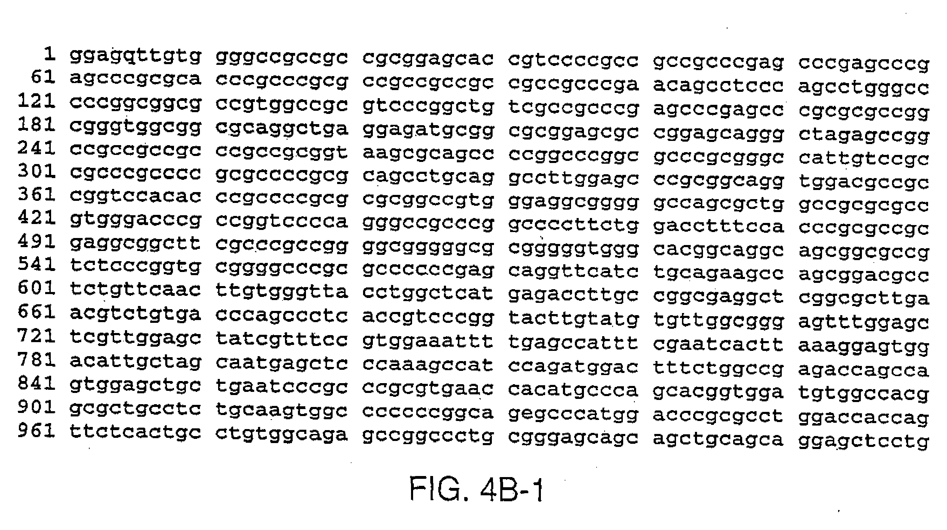

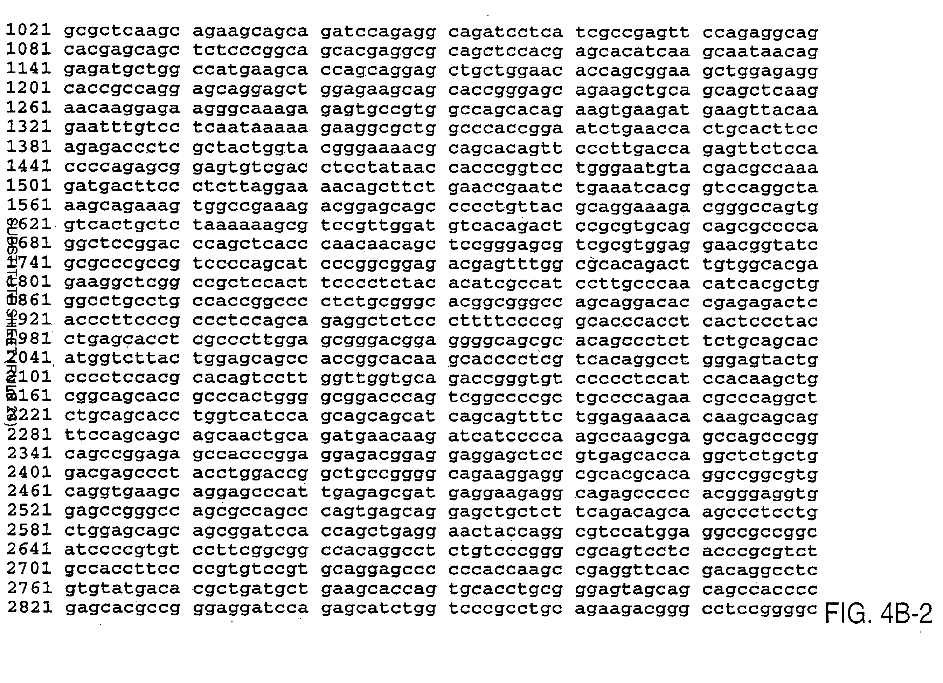

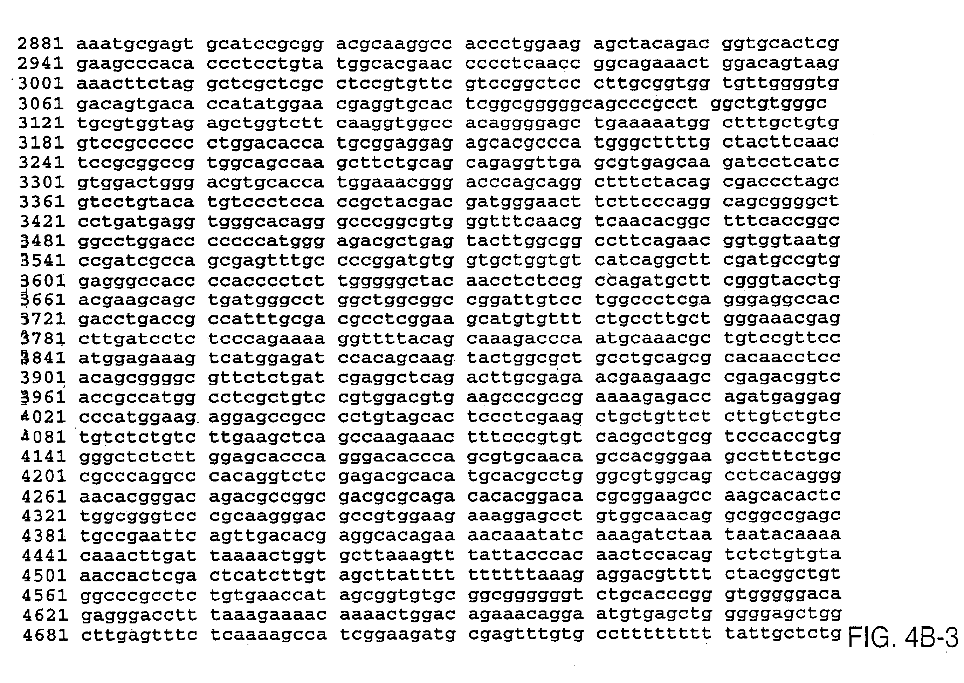

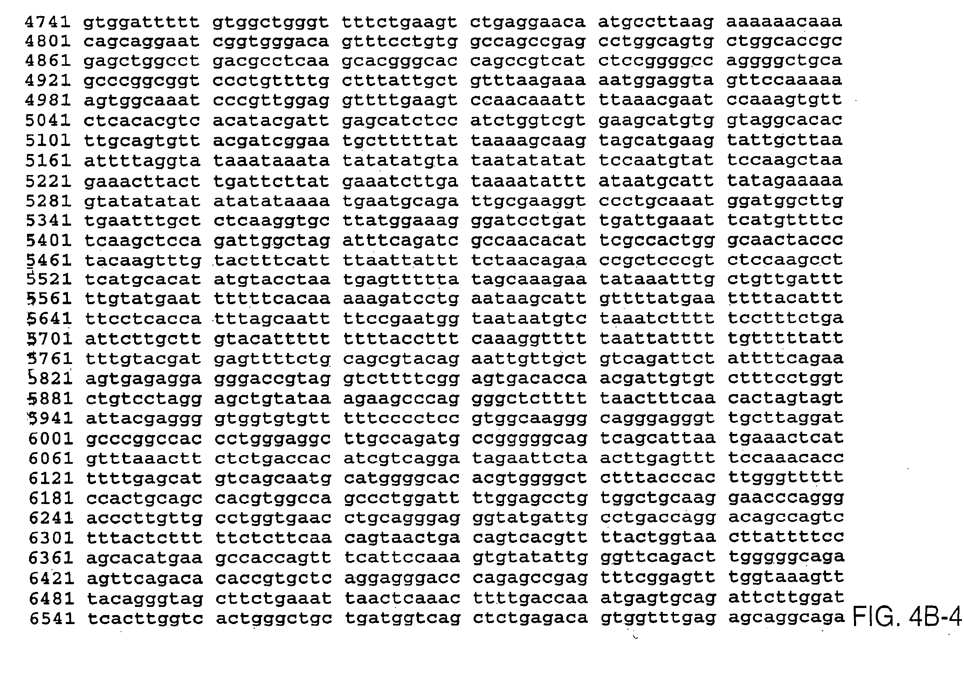

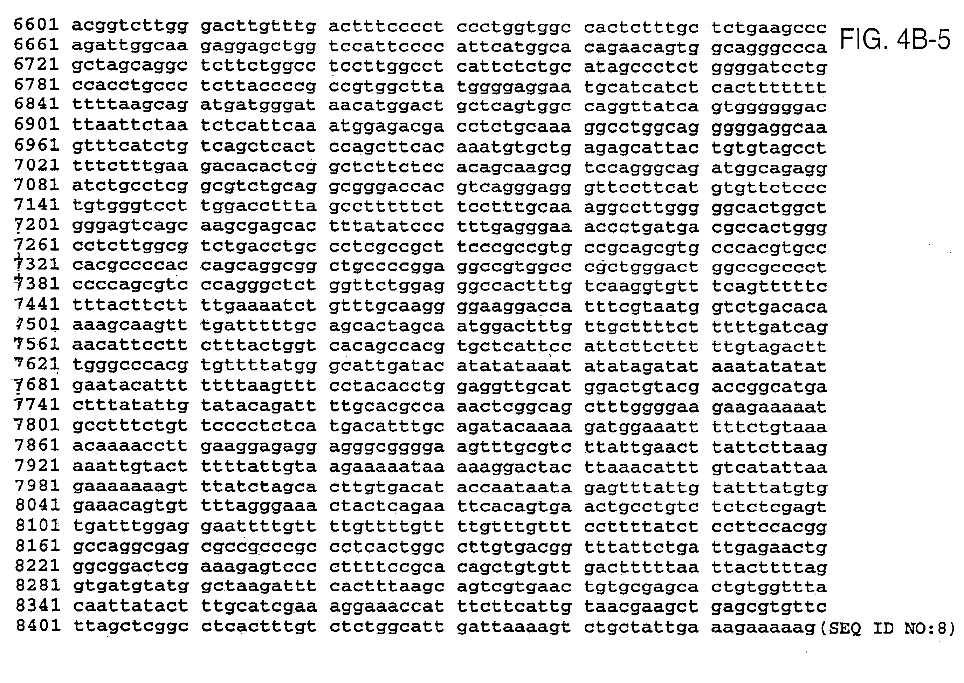

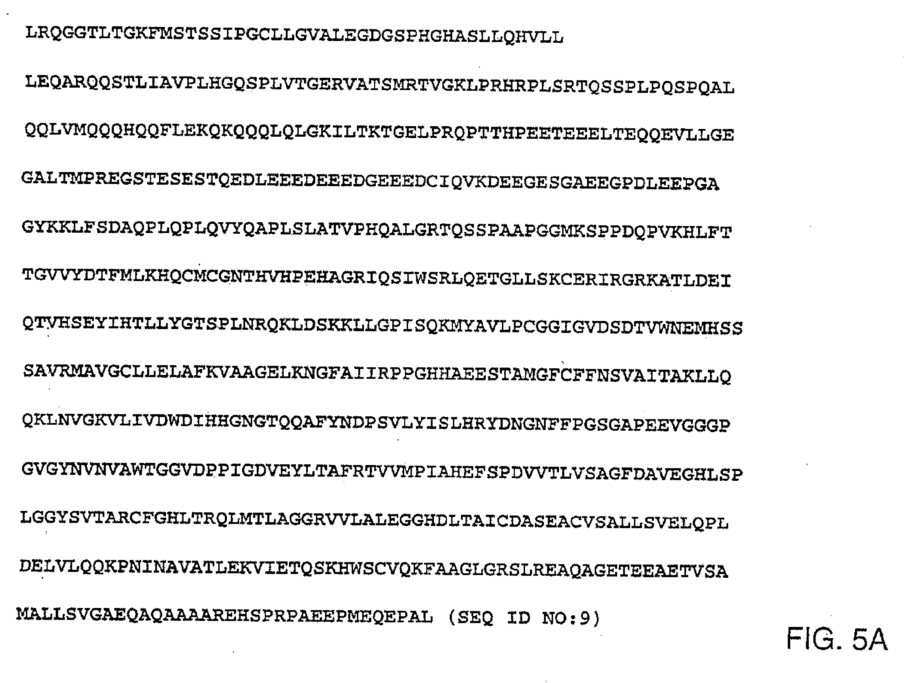

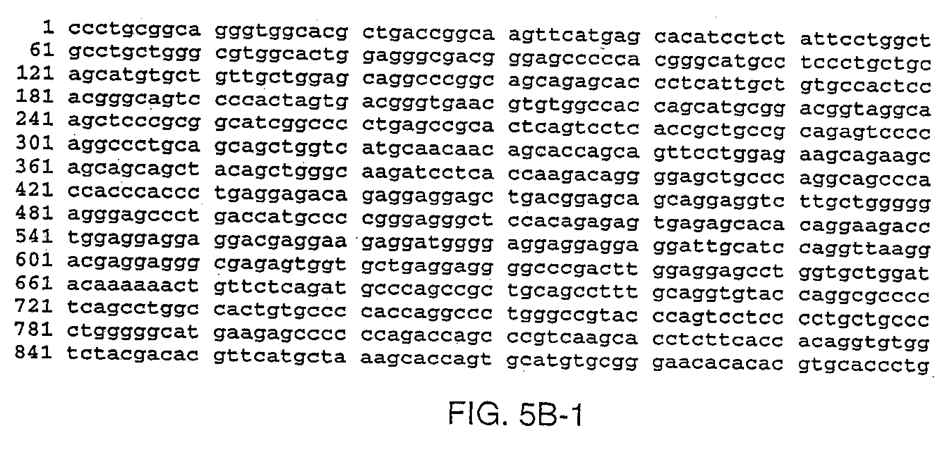

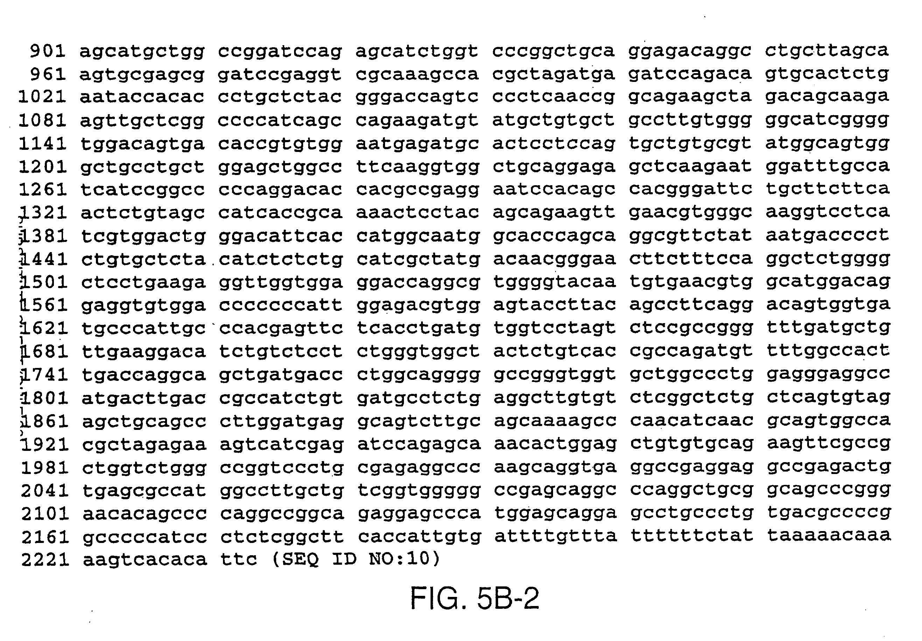

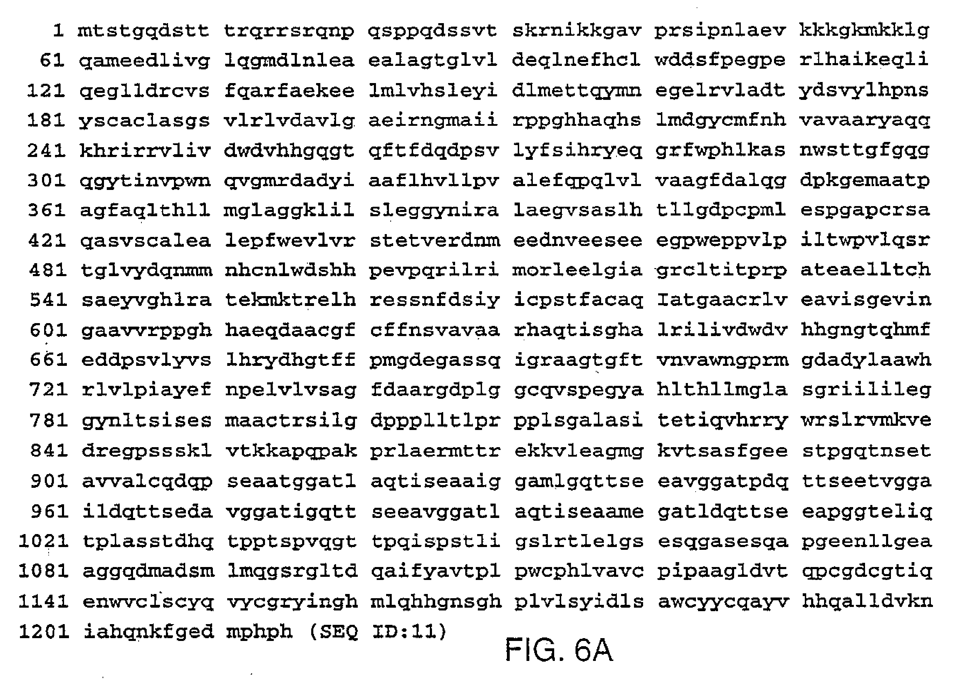

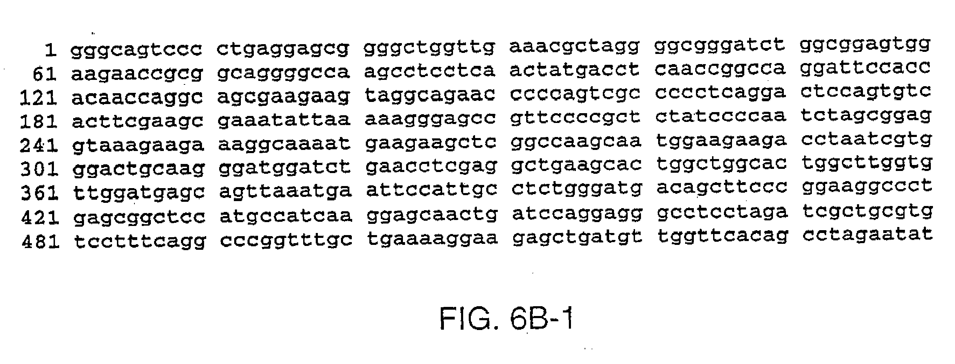

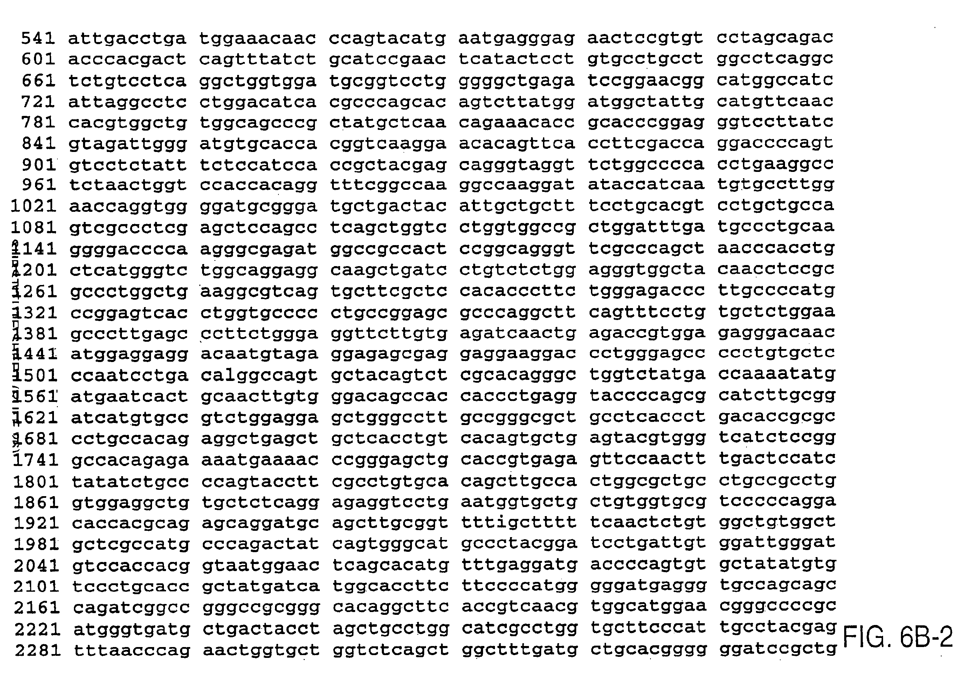

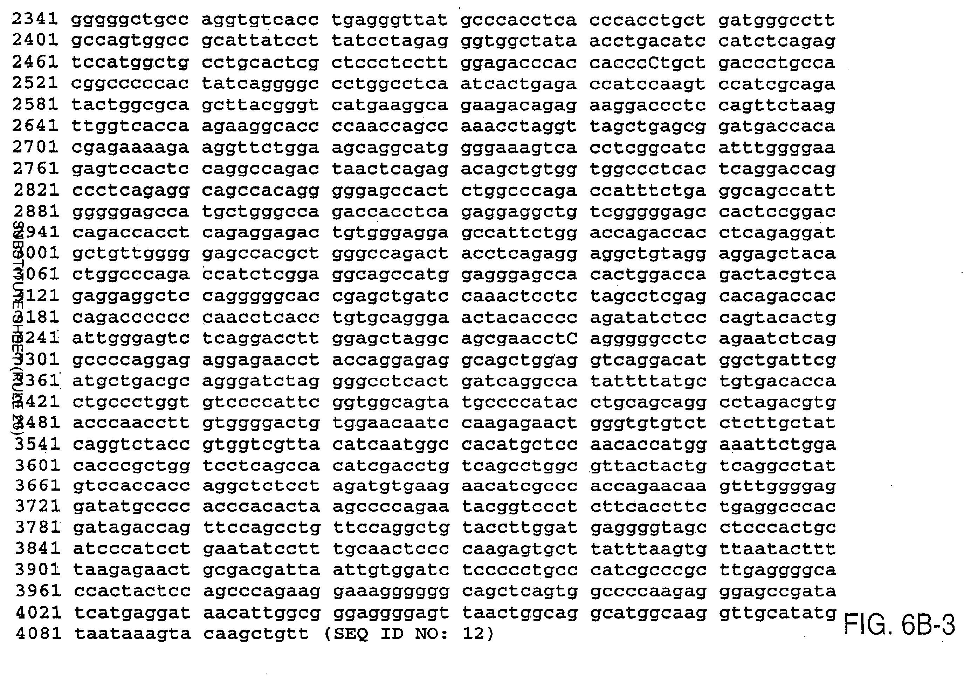

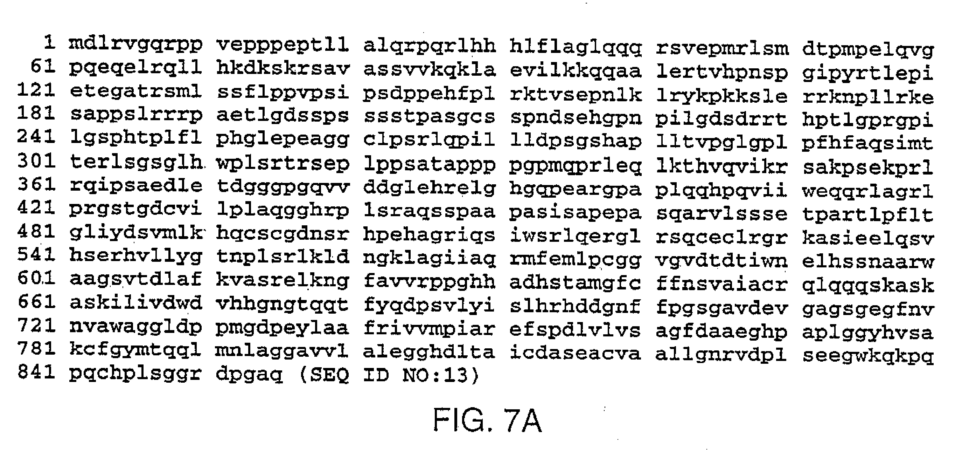

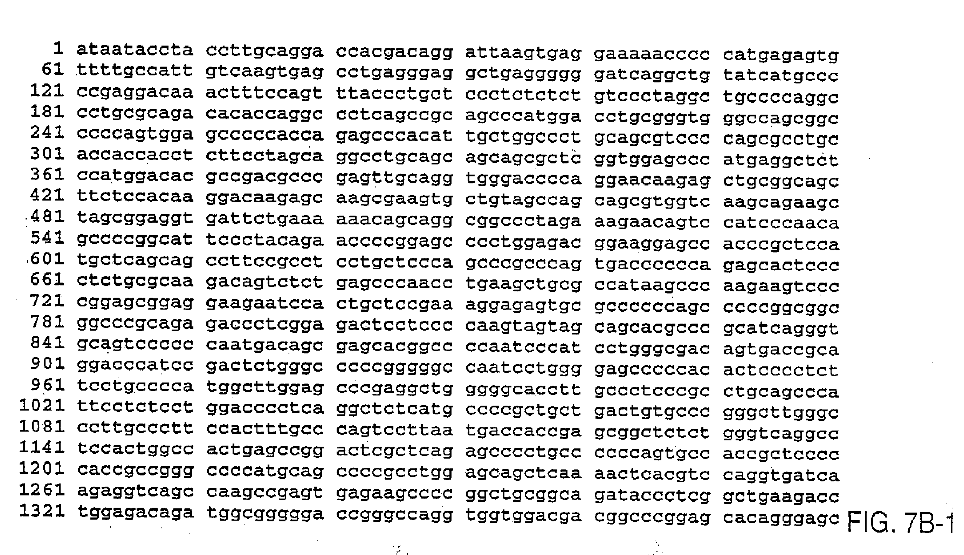

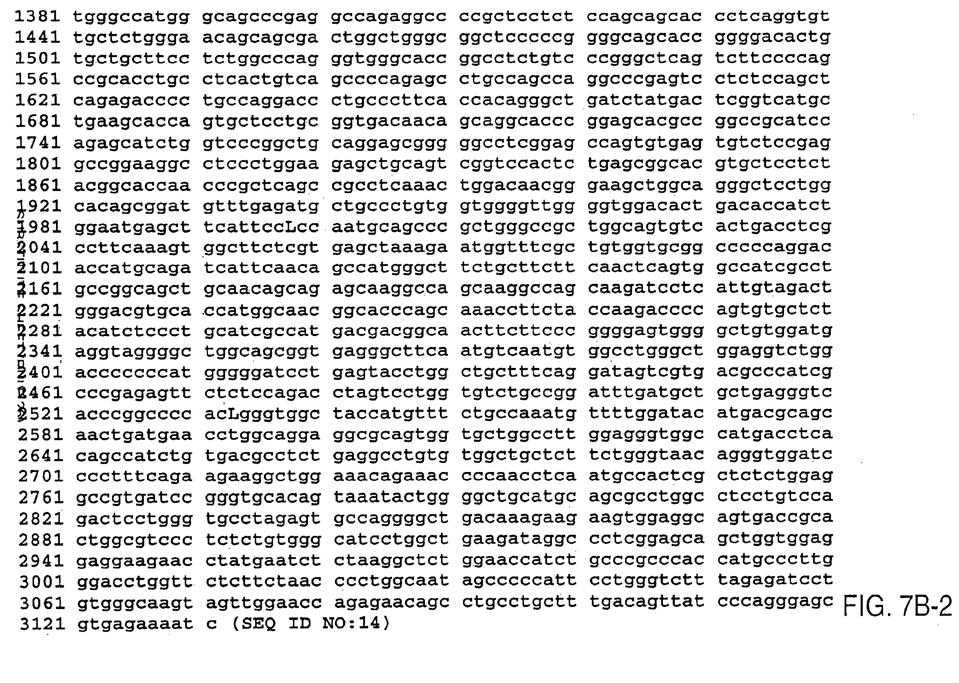

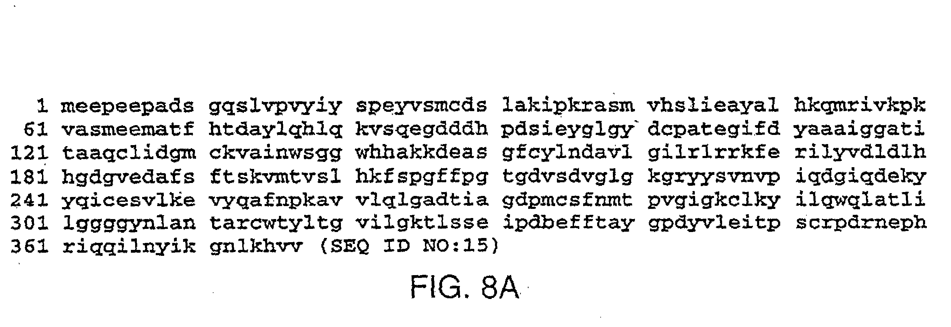

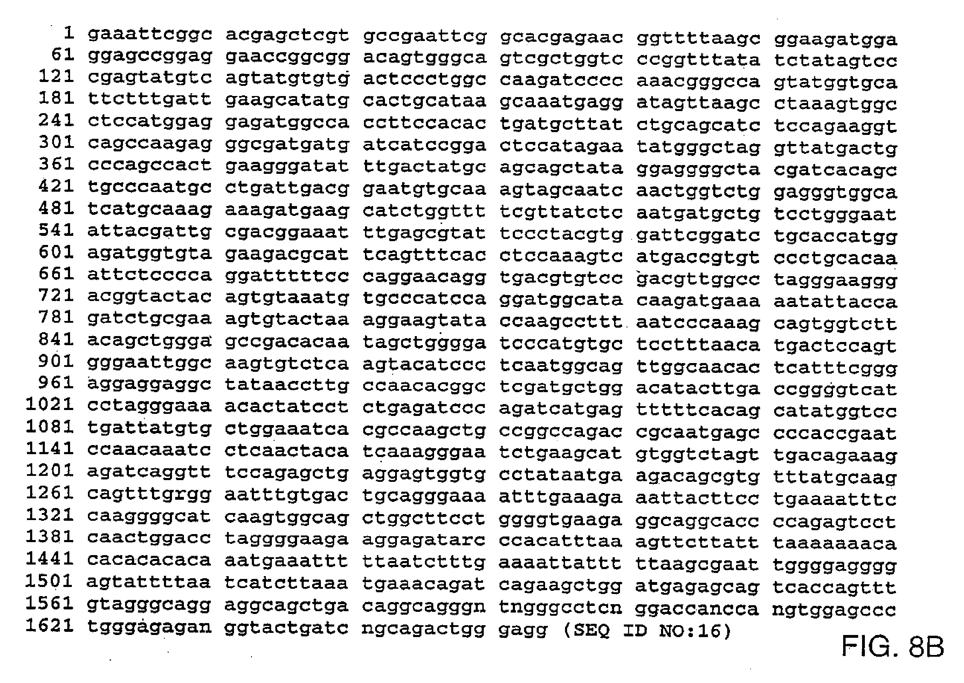

Particularly preferred non-limiting examples of antisense oligonucleotides of the invention are complementary to regions of RNA or double-stranded DNA encoding a histone deacetylase isoform (e.g., HDAC-1, HDAC-2, HDAC-3, HDAC-4, HDAC-5, HDAC-6, HDAC-7, or HDAC-8). (see *e.g.*, GenBank Accession No. U50079 for human HDAC-1 (Fig. 1B); GenBank Accession No. U31814 for human HDAC-2; (Fig. 2B) GenBank Accession No. U75697 for human HDAC-3 (Fig. 3B; GenBank Accession No. AB006626 for human HDAC-4 (Fig. 4B); GenBank Accession No. AB011172 for human HDAC-5 (Fig. 5B); GenBank Accession No. AJ011972 for human HDAC-6 (Fig. 6B); GenBank Accession No. AF239243 for human HDAC-7 (Fig. 7B); and GenBank Accession No. AF230097 for human HDAC-8 (Fig. 8B)).

The sequences encoding histone deacetylases from many non-human animal species are also known (see, for example, GenBank Accession Numbers X98207 (murine HDAC-1); NM_008229 (murine HDAC-2); NM_010411 (murine HDAC-3); NM_006037 (murine HDAC-4); NM_010412 (murine HDAC-5); NM_010413 (murine HDAC-6); and AF207749 (murine HDAC-7)). Accordingly, the antisense oligonucleotides of the invention may also be complementary to regions of RNA or double-stranded DNA that encode histone deacetylases from non-human animals. Antisense oligonucleotides according to these embodiments are useful as tools in animal models for studying the role of specific histone deacetylase isoforms.

Particularly, preferred oligonucleotides have nucleotide sequences of from about 13 to about 35 nucleotides which include the nucleotide sequences shown in Table I. Yet additional particularly preferred oligonucleotides have nucleotide sequences of from about 15 to about 26 nucleotides of the nucleotide sequences shown below. Most preferably, the oligonucleotides shown below have phosphorothioate backbones, are 20-26 nucleotides in length, and are modified such that the terminal four nucleotides at the 5' end of the oligonucleotide and the terminal four nucleotides at the 3' end of the oligonucleotide each have 2'-O- methyl groups attached to their sugar residues.

Antisense oligonucleotides used in the present study are shown in Table I.

**Table 1**

| **Sequences of Human Isotype-Specific Antisense (AS) Oligonucleotides and Their Mismatch (MM) Oligonucleotides** | | | | | |
|---|---|---|---|---|---|
| Oligo | Target | Accession Number | Nucleotide Position | Sequence | Gene Position |
| HDACI ASl | Human HDACl | U50079 | 1585-1604 | 5'-GAAACGTGAGGGACTCAGCA-3' (SEQ ID NO:17) | 3'-UTR |
| HDACI AS2 | Human HDACl | U50079 | 1565-1584 | 5'GGAAGCCAGAGCTGGAGAGG-3'(SEQ ID NO:18) | 3'-UTR |
| HDACI MM | Human HDACl | U50079 | 1585-1604 | 5'-GTTAGGTGAGGCACTGAGGA-3'(SEQ ID NO:19) | 3'-UTR |
| HDAC2 AS | Human HDAC2 | U31814 | 1643-1622 | 5'-GCTGAGCTGTTCTGATTTGG-3'(SEQ ID NO:20) | 3'-UTR |
| HDAC2 MM | Human HPAC2 | U31814 | 1643-1622 | 5'CGTGAGCACTTCTCATTTCC3'(SEQ ID NO:21 | 3'-UTA |
| HDAC3 AS | Human HDAC3 | AF039703 | 1276-1295 | 5'-CGCTTTCCTTGTCATTGACA-3'(SEQ ID NO:22) | 3'-UTR |
| HDAC3 MM | Human HDAC3 | AF039703 | 1276-1295 | 5'-GCCTTTCCTACTCATTGTGT-3'(SEQ ID NO:23) | 3'-UTR |
| HDAC4 AS1 | Human HDAC4 | AB006626 | 514-33 | 5-GCTGCCTGCCGTGCCCACCC-3'(SEQ ID NO:24) | 5'-UTR |
| HDAC4 MM1 | Human HDAC4 | AB006626 | 514-33 | 5'-CGTGCCTGCGCTGCCCACGG-3'(SEQ ID NO:25) | 5'-UTR |
| HDAC4 AS2 | Human HDAC4 | AB006626 | 7710-29 | 5'-TACAGTCCATCCAACCTCCA-3'(SEQ ID NO:26) | 3'-UTR |
| HDAC4 MM4 | Human HDAC4 | AB006626 | 7710-29 | 5'-ATCAGTCCAACCAACCTCGT-3'(SEQ ID NO:27) | 3'-UTR |
| HDAC5 AS | Human HDAC5 | AF039691 | 2663-2682 | 5'-CTTCGGTCTCACCTGCTTGG-3'(SEQ ID NQ:28) | 3'-UTR |
| HDAC6 AS | Human HDAC6 | AJ011972 | 3791-3810 | 5'-CAGGCTGOAATCAGCTACAG-3'(SEQ ID NO:29) | 3'-UTR |
| HDAC6 MM | Human HDAC6 | AJ011972 | 3791-3810 | 5'-GACGCTGCAATCAGGTAGAC-3'(SEQ ID NO:30) | 3'-UTR |
| HDAC7 AS | Human HDAC7 | AF239243 | 2896-2915 | 5'-CTTCAGCCAGCATGCCCACA-3'(SEQ ID NO:31) | 3'-UTR |
| HDAC8 AS1 | Human HDAC8 | AF230097 | 51-70 | 5'-CTCCGGCTCCTCCATCTTCC-3'(SEQ ID NO:32) | 5'-UTR |
| HDAC8 AS2 | Human HDAC8 | AF230097 | 1328-1347 | 5'-AGCCAGCTGCCACTTGATGC-3'(SEQ ID NO:33) | 3'-UTR |

The antisense oligonucleotides according to the invention may optionally be formulated with any of the well known pharmaceutically acceptable carriers or diluents (see preparation of pharmaceutically acceptable formulations in, *e.g.,* Remington's Pharmaceutical Sciences, 18th Edition, ed. A. Gennaro, Mack Publishing Co., Easton, PA, 1990), with the proviso that such carriers or diluents not affect their ability to modulate HDAC activity.

By way of non-limiting example, the agent of the first aspect of the invention may also be a small molecule inhibitor. The term "small molecule" as used in reference to the inhibition of histone deacetylase is used to identify a compound having a molecular weight preferably less than 1000 Da, more preferably less than 800 Da, and most preferably less than 600 Da, which is capable of interacting with a histone deacetylase and inhibiting the expression of a nucleic acid molecule encoding an HDAC isoform or activity of an HDAC protein. Inhibiting histone deacetylase enzymatic activity means reducing the ability of a histone deacetylase to remove an acetyl group from a histone. In some preferred embodiments, such reduction of histone deacetylase activity is at least about 50%, more preferably at least about 75%, and still more preferably at least about 90%. In other preferred embodiments, histone deacetylase activity is reduced by at least 95% and more preferably by at least 99%. In one certain embodiment, the small molecule inhibitor is an inhibitor of one or more but less than all HDAC isoforms. By "all HDAC isoforms" is meant all proteins that specifically remove an epsilon acetyl group from an N-terminal lysine of a histone, and includes, without limitation, HDAC-1, HDAC-2, HDAC-3, HDAC-4, HDAC-5, HDAC-6, HDAC-7, or HDAC-8, all of which are considered "related proteins," as used herein.

Most preferably, a histone deacetylase small molecule inhibitor interacts with and reduces the activity of one or more histone deacetylase isoforms (e.g., HDAC-1 and/or HDAC-4), but does not interact with or reduce the activities of all of the other histone deacetylase isoforms (*e.g.,* HDAC-2 and HDAC-6). As discussed below, a preferred histone deacetylase small molecule inhibitor is one that interacts with and reduces the enzymatic activity of a histone deacetylase isoform that is involved in tumorigenesis.

Non-limiting examples of small molecule inhibitors useful for the invention are presented in Table 2.

**Table 2**

| Small Molecule HDAC Inhibitors [µM] and Their Antitumor Activities *In Vivo* | | | | | | | | | | | | |
|---|---|---|---|---|---|---|---|---|---|---|---|---|
| | | Enzyme IC50 (uM) | | | | | | % inhibitor of tumor formation in vivo | | | | |
| | Inhibitor | | | | | | | | | | | |
| Cpd | Structure | HDAC1 | HDAC2 | HDAC3 | HDAC4 | HDAC6 | R4-Ac | MTT | Cell Cycle Arrest EC | colon | lung | prostate |
| 1 | 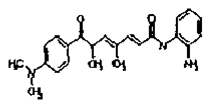 | 3 | 25 | 21 | 23 | >50 | I | 3 | 2 | | | |
| 2 | 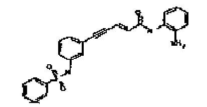 | 3 | 31 | 30 | 35 | >30 | 5 | 4 | 8 | 53 (40.po ) | 54 (50.ip) | |
| 3 | 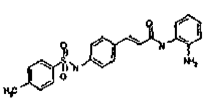 | 3 | 22 | 45 | 28 | >50 | 5 | 4 | 2 | 55 (40.ip) | | |
| note: for *in vivo* antitumor studies, numbers outside brackets indicate % of inhibition of tumor growth in vivo; | | | | | | | | | | | | |
| numbers In brockets indicate daily dose of inhibitor used (mg/kg body weight/day); | | | | | | | | | | | | |
| oral (PO) or intraperitoneal (IP) administration is indicated in brackets. | | | | | | | | | | | | |

The reagents according to the invention are useful as analytical tools and as therapeutic tools, including as gene therapy tools. The invention also provides methods and compositions which may be manipulated and fine-tuned to fit the condition(s) to be treated while producing fewer side effects.

In a second aspect, the invention provides a method for inhibiting one or more, but less than all, histone deacetylase isoforms in a cell comprising contacting the cell with an agent of the first aspect of the invention. By way of non-limiting example, the agent may be an antisense oligonucleotide or a small molecule inhibitor that inhibits the expression of one or more, but less than all, specific histone deacetylase isoforms in the cell.

In one certain embodiment, the invention provides a method comprising contacting a cell with an antisense oligonucleotide that inhibits one or more but less than all histone deacetylase isoforms in the cell. Preferably, cell proliferation is inhibited in the contacted cell. Thus, the antisense oligonucleotides according to the invention are useful in therapeutic approaches to human diseases including benign and malignant neoplasms by inhibiting cell proliferation in cells contacted with the antisense oligonucleotides. The phrase "inhibiting cell proliferation" is used to denote an ability of a histone deacetylase antisense oligonucleotide or a small molecule histone deacetylase inhibitor (or combination thereof) to retard the growth of cells contacted with the oligonucleotide or small molecule inhibitor, as compared to cells not contacted. Such an assessment of cell proliferation can be made by counting contacted and non-contacted cells using a Coulter Cell Counter (Coulter, Miami, FL) or a hemacytometer. Where the cells are in a solid growth (*e.g.,* a solid tumor or organ), such an assessment of cell proliferation can be made by measuring the growth with calipers, and comparing the size of the growth of contacted cells with non-contacted cells. Preferably, the term includes a retardation of cell proliferation that is at least 50% of non-contacted cells. More preferably, the term includes a retardation of cell proliferation that is 100% of non-contacted cells (*i.e.,* the contacted cells do not increase in number or size). Most preferably, the term includes a reduction in the number or size of contacted cells, as compared to non-contacted cells. Thus, a histone deacetylase antisense oligonucleotide or a histone deacetylase small molecule inhibitor that inhibits cell proliferation in a contacted cell may induce the contacted cell to undergo growth retardation, to undergo growth arrest, to undergo programmed cell death (*i.e.,* to apoptose), or to undergo necrotic cell death.

Conversely, the phrase "inducing cell proliferation" and similar terms are used to denote the requirement of the presence or enzymatic activity of a specific histone deacetylase isoform for cell proliferation in a normal (*i.e.,* non-neoplastic) cell. Hence, over-expression of a specific histone deacetylase isoform that induces cell proliferation may or may not lead to increased cell proliferation; however, inhibition of a specific histone deacetylase isoform that induces cell proliferation will lead to inhibition of cell proliferation.

The cell proliferation inhibiting ability of the antisense oligonucleotides according to the invention allows the synchronization of a population of a-synchronously growing cells. For example, the antisense oligonucleotides of the invention may be used to arrest a population of non-neoplastic cells grown *in vitro* in the G1 or G2 phase of the cell cycle. Such synchronization allows, for example, the identification of gene and/or gene products expressed during the G1 or G2 phase of the cell cycle. Such a synchronization of cultured cells may also be useful for testing the efficacy of a new transfection protocol, where transfection efficiency varies and is dependent upon the particular cell cycle phase of the cell to be transfected. Use of the antisense oligonucleotides of the invention allows the synchronization of a population of cells, thereby aiding detection of enhanced transfection efficiency.

The anti-neoplastic utility of the antisense oligonucleotides according to the invention is described in detail elsewhere in this specification.

In yet other preferred embodiments, the cell contacted with a histone deacetylase antisense oligonucleotide is also contacted with a histone deacetylase small molecule inhibitor.

In a few preferred embodiments, the histone deacetylase small molecule inhibitor is operably associated with the antisense oligonucleotide. As mentioned above, the antisense oligonucleotides according to the invention may optionally be formulated well known pharmaceutically acceptable carriers or diluents. This formulation may further contain one or more one or more additional histone deacetylase antisense oligonucleotide(s), and/or one or more histone deacetylase small molecule inhibitor(s), or it may contain any other pharmacologically active agent.

In a particularly preferred embodiment of the invention, the antisense oligonucleotide is in operable association with a histone deacetylase small molecule inhibitor. The term "operable association" includes any association between the antisense oligonucleotide and the histone deacetylase small molecule inhibitor which allows an antisense oligonucleotide to inhibit one or more specific histone deacetylase isoform-encoding nucleic acid expression and allows the histone deacetylase small molecule inhibitor to inhibit specific histone deacetylase isoform enzymatic activity. One or more antisense oligonucleotide of the invention may be operably associated with one or more histone deacetylase small molecule inhibitor. In some preferred embodiments, an antisense oligonucleotide of the invention that targets one particular histone deacetylase isoform (e.g., HDAC-1) is operably associated with a histone deacetylase small molecule inhibitor which targets the same histone deacetylase isoform. A preferred operable association is a hydrolyzable. Preferably, the hydrolyzable association is a covalent linkage between the antisense oligonucleotide and the histone deacetylase small molecule inhibitor. Preferably, such covalent linkage is hydrolyzable by esterases and/or amidases. Examples of such hydrolyzable associations are well known in the art. Phosphate esters are particularly preferred.

In certain preferred embodiments, the covalent linkage may be directly between the antisense oligonucleotide and the histone deacetylase small molecule inhibitor so as to integrate the histone deacetylase small molecule inhibitor into the backbone. Alternatively, the covalent linkage may be through an extended structure and may be formed by covalently linking the antisense oligonucleotide to the histone deacetylase small molecule inhibitor through coupling of both the antisense oligonucleotide and the histone deacetylase small molecule inhibitor to a carrier molecule such as a carbohydrate, a peptide or a lipid or a glycolipid. Other preferred operable associations include lipophilic association, such as formation of a liposome containing an antisense oligonucleotide and the histone deacetylase small molecule inhibitor covalently linked to a lipophilic molecule and thus associated with the liposome. Such lipophilic molecules include without limitation phosphotidylcholine, cholesterol, phosphatidylethanolamine, and synthetic neoglycolipids, such as syalyllacNAc-HDPE. In certain preferred embodiments, the operable association may not be a physical association, but simply a simultaneous existence in the body, for example, when the antisense oligonucleotide is associated with one liposome and the small molecule inhibitor is associated with another liposome.

In a third aspect, the invention provides a method for inhibiting neoplastic cell proliferation in an animal comprising administering to an animal having at least one neoplastic cell present in its body a therapeutically effective amount of an agent of the first aspect of the invention. In one certain embodiment, the agent is an antisense oligonucleotide of the first aspect of the invention, and the method further comprises a pharmaceutically acceptable carrier. The antisense oligonucleotide and the pharmaceutically acceptable carrier are administered for a therapeutically effective period of time. Preferably, the animal is a mammal, particularly a domesticated mammal. Most preferably, the animal is a human.

The term "neoplastic cell" is used to denote a cell that shows aberrant cell growth. Preferably, the aberrant cell growth of a neoplastic cell is increased cell growth. A neoplastic cell may be a hyperplastic cell, a cell that shows a lack of contact inhibition of growth *in vitro,* a benign tumor cell that is incapable of metastasis *in vivo,* or a cancer cell that is capable of metastases *in vivo* and that may recur after attempted removal. The term "tumorigenesis" is used to denote the induction of cell proliferation that leads to the development of a neoplastic growth.

The terms "therapeutically effective amount" and "therapeutically effective period of time" are used to denote known treatments at dosages and for periods of time effective to reduce neoplastic cell growth. Preferably, such administration should be parenteral, oral, sublingual, transdermal, topical, intranasal, or intrarectal. When administered systemically the therapeutic composition is preferably administered at a sufficient dosage to attain a blood level of antisense oligonucleotide from about 0.1 µM to about 10 µM. For localized administration, much lower concentrations than this may be effective, and much higher concentrations may be tolerated. One of skill in the art will appreciate that such therapeutic effect resulting in a lower effective concentration of the histone deacetylase inhibitor may vary considerably depending on the tissue, organ, or the particular animal or patient to be treated according to the invention.

In a preferred embodiment, the therapeutic composition of the invention is administered systemically at a sufficient dosage to attain a blood level of antisense oligonucleotide from about 0.01 µM to about 20 µM. In a particularly preferred embodiment, the therapeutic composition is administered at a sufficient dosage to attain a blood level of antisense oligonucleotide from about 0.05 µM to about 15 µM. In a more preferred embodiment, the blood level of antisense oligonucleotide is from about 0.1 µM to about 10 µM.

For localized administration, much lower concentrations than this may be therapeutically effective. Preferably, a total dosage of antisense oligonucleotide will range from about 0.1 mg to about 200 mg oligonucleotide per kg body weight per day. In a more preferred embodiment, a total dosage of antisense oligonucleotide will range from about 1 mg to about 20 mg oligonucleotide per kg body weight per day. In a most preferred embodiment, a total dosage of antisense oligonucleotide will range from about 1 mg to about 10 mg oligonucleotide per kg body weight per day. In a particularly preferred embodiment, the therapeutically effective amount of a histone deacetylase antisense oligonucleotide is about 5 mg oligonucleotide per kg body weight per day.

In certain preferred embodiments of the third aspect of the invention, the method further comprises administering to the animal a therapeutically effective amount of a histone deacetylase small molecule inhibitor with a pharmaceutically acceptable carrier for a therapeutically effective period of time. In some preferred embodiments, the histone deacetylase small molecule inhibitor is operably associated with the antisense oligonucleotide, as described *supra.*

The histone deacetylase small molecule inhibitor-containing therapeutic composition of the invention is administered systemically at a sufficient dosage to attain a blood level histone deacetylase small molecule inhibitor from about 0.01µM to about 10µM. In a particularly preferred embodiment, the therapeutic composition is administered at a sufficient dosage to attain a blood level of histone deacetylase small molecule inhibitor from about 0.05µM to about 10µM. In a more preferred embodiment, the blood level of histone deacetylase small molecule inhibitor is from about 0.1µM to about 5µM. For localized administration, much lower concentrations than this may be effective. Preferably, a total dosage of histone deacetylase small molecule inhibitor will range from about 0.01 mg to about 100 mg protein effector per kg body weight per day. In a more preferred embodiment, a total dosage of histone deacetylase small molecule inhibitor will range from about 0.1 mg to about 50 mg protein effector per kg body weight per day. In a most preferred embodiment, a total dosage of histone deacetylase small molecule inhibitor will range from about 0.1 mg to about 10 mg protein effector per kg body weight per day. In a particularly preferred embodiment, the therapeutically effective synergistic amount of histone deacetylase small molecule inhibitor (when administered with an antisense oligonucleotide) is about 5 mg per kg body weight per day.

Certain preferred embodiments of this aspect of the invention result in an improved inhibitory effect, thereby reducing the therapeutically effective concentrations of either or both of the nucleic acid level inhibitor (*i.e.,* antisense oligonucleotide) and the protein level inhibitor (*i.e.,*histane deacetylase small molecule inhibitor) required to obtain a given inhibitory effect as compared to those necessary when either is used individually.

Furthermore, one of skill will appreciate that the therapeutically effective synergistic amount of either the antisense oligonucleotide or the histone deacetylase inhibitor may be lowered or increased by fine tuning and altering the amount of the other component. The invention therefore provides a method to tailor the administration/treatment to the particular exigencies specific to a given animal species or particular patient. Therapeutically effective ranges may be easily determined for example empirically by starting at relatively low amounts and by step-wise increments with concurrent evaluation of inhibition.

In a fourth aspect, the invention provides a method for identifying a specific histone deacetylase isoform that is required for induction of cell proliferation comprising contacting a cell with an agent of the first aspect of the invention. In certain preferred embodiments, the agent is an antisense oligonucleotide that inhibits the expression of a histone deacetylase isoform, wherein the antisense oligonucleotide is specific for a particular HDAC isoform, and thus inhibition of cell proliferation in the contacted cell identifies the histone deacetylase isoform as a histone deacetylase isoform that is required for induction of cell proliferation. In other certain embodiments, the agent is a small molecule inhibitor that inhibits the activity of a histone deacetylase isoform, wherein the small molecule inhibitor is specific for a particular HDAC isoform, and thus inhibition of cell proliferation in the contacted cell identifies the histone deacetylase isoform as a histone deacetylase isoform that is required for induction of cell proliferation. In certain preferred embodiments, the cell is a neoplastic cell, and the induction of cell proliferation is tumorigenesis. In still yet other preferred embodiments of the fourth aspect of the invention, the method comprises an agent of the first aspect of the invention which is a combination of one or more antisense oligonucleotides and/or one or more small molecule inhibitors of the first aspect of the invention. In certain preferred embodiments, the histone deacetylase isoform is HDAC-1, HDAC-2, HDAC-3, HDAC-4, HDAC-5, HDAC-6, HDAC-7, or HDAC-8. In other certain preferred embodiments, the histone deacetylase isoform is HDAC-1 and/or HDAC-4.

In an fifth aspect, the invention provides a method for identifying a histone deacetylase isoform that is involved in induction of cell differentiation comprising contacting a cell with an agent that inhibits the expression of a histone deacetylase isoform, wherein induction of differentiation in the contacted cell identifies the histone deacetylase isoform as a histone deacetylase isoform that is involved in induction of cell differentiation. In certain preferred embodiments, the agent is an antisense oligonucleotide of the first aspect of the invention. In other certain preferred embodiments, the agent is an small molecule inhibitor of the first aspect of the invention. In still other certain embodiments, the cell is a neoplastic cell. In still yet other preferred embodiments of the fifth aspect of the invention, the method comprises an agent of the first aspect of the invention which is a combination of one or more antisense oligonucleotides and/or one or more small molecule inhibitors of the first aspect of the invention. In certain preferred embodiments, the histone deacetylase isoform is HDAC-1, HDAC-2, HDAC-3, HDAC-4, HDAC-5, HDAC-6, HDAC-7, or HDAC-8. In other certain preferred embodiments, the histone deacetylase isoform is HDAC-1 and/or HDAC-4.

In a sixth aspect, the invention provides a method for inhibiting neoplastic cell growth in an animal comprising administering to an animal having at least one neoplastic cell present in its body a therapeutically effective amount of an agent of the first aspect of the invention. In certain embodiments thereof, the agent is an antisense oligonucleotide, which is combined with a pharmaceutically acceptable carrier and administered for a therapeutically effective period of time.

In certain embodiments where the agent of the first aspect of the invention is a histone deacetylase small molecule inhibitor, therapeutic compositions of the invention comprising said small molecule inhibitor(s) are administered systemically at a sufficient dosage to attain a blood level histone deacetylase small molecule inhibitor from about 0.01 µM to about 10 µM. In a particularly preferred embodiment, the therapeutic composition is administered at a sufficient dosage to attain a blood level of histone deacetylase small molecule inhibitor from about 0.05 µM to about 1.0 µM. In a more preferred embodiment, the blood level of histone deacetylase small molecule inhibitor is from about 0.1 µM to about 5 µM. For localized administration, much lower concentrations than this may be effective. Preferably, a total dosage of histone deacetylase small molecule inhibitor will range from about 0.01 mg to about 100 mg protein effector per kg body weight per day. In a more preferred embodiment, a total dosage of histone deacetylase small molecule inhibitor will range from about 0.1 mg to about 50 mg protein effector per kg body weight per day. In a most preferred embodiment, a total dosage of histone deacetylase small molecule inhibitor will range from about 0.1 mg to about 10 mg protein effector per kg body weight per day.

In a sixth aspect, the invention provides a method for investigating the role of a particular histone deacetylase isoform in cellular proliferation, including the proliferation of neoplastic cells. In this method, the cell type of interest is contacted with an amount of an antisense oligonucleotide that inhibits the expression of one or more specific histone deacetylase isoform, as described for the first aspect according to the invention, resulting in inhibition of expression of the histone deacetylase isoform(s) in the cell. If the contacted cell with inhibited expression of the histone deacetylase isoform(s) also shows an inhibition in cell proliferation, then the histone deacetylase isoform(s) is required for the induction of cell proliferation. In this scenario, if the contacted cell is a neoplastic cell, and the contacted neoplastic cell shows an inhibition of cell proliferation, then the histone deacetylase isoform whose expression was inhibited is a histone deacetylase isoform that is required for tumorigenesis. In certain preferred embodiments, the histone deacetylase isoform is HDAC-1, HDAC-2, HDAC-3, HDAC-4, HDAC-5, HDAC-6, HDAC-7, or HDAC-8. In certain preferred embodiments, the histone deacetylase isoform is HDAC-1 and/or HDAC-4.

Thus, by identifying a particular histone deacetylase isoform that is required for in the induction of cell proliferation, only that particular histone deacetylase isoform need be targeted with an antisense oligonucleotide to inhibit cell proliferation or induce differentiation. Consequently, a lower therapeutically effective dose of antisense oligonucleotide may be able to effectively inhibit cell proliferation. Moreover, undesirable side effects of inhibiting all histone deacetylase isoforms may be avoided by specifically inhibiting the one (or more) histone deacetylase isoform(s) required for inducing cell proliferation.

As previously indicated, the agent of the first aspect includes, but is not limited to, oligonucleotides and small molecule inhibitors that inhibit the activity of one or more, but less than all, HDAC isoforms. The measurement of the enzymatic activity of a histone deacetylase isoform can be achieved using known methodologies. For example, Yoshida et al. (J. Biol. Chem.265: 17174-17179,1990) describe the assessment of histone deacetylase enzymatic activity by the detection of acetylated histones in trichostatin A treated cells. Taunton et al. (Science272: 408-411, 1996) similarly describes methods to measure histone deacetylase enzymatic activity using endogenous and recombinant HDAC. Both Yoshida et al. (J. Biol. Chem. 265: 17174-17179, 1990) and Taunton et al. (Science272: 408-411, 1996) are hereby incorporated by reference.

Preferably, the histone deacetylase small molecule inhibitor(s) of the invention that inhibits a histone deacetylase isoform that is required for induction of cell proliferation is a histone deacetylase small molecule inhibitor that interacts with and reduces the enzymatic activity of fewer than all histone deacetylase isoforms.

In an seventh aspect, the invention provides a method for identifying a histone deacetylase isoform that is involved in induction of cell differentiation, comprising contacting a cell with an antisense oligonucleotide that inhibits the expression of a histone deacetylase isoform, wherein induction of differentiation in the contacted cell identifies the histone deacetylase isoform as a histone deacetylase isoform that is involved in induction of cell differentiation. Preferably, the cell is a neoplastic cell. In certain preferred embodiments, the histone deacetylase isoform is HDAC-1, HDAC-2, HDAC-3, HDAC-4, HDAC-5, HDAC-6, HDAC-7, or HDAC-8.

The phrase "inducing cell differentiation" and similar terms are used to denote the ability of a histone deacetylase antisense oligonucleotide or histone deacetylase small molecule inhibitor (or combination thereof) to induce differentiation in a contacted cell as compared to a cell that is not contacted. Thus, a neoplastic cell, when contacted with a histone deacetylase antisense oligonucleotide or histone deacetylase small molecule inhibitor (or both) of the invention, may be induced to differentiate, resulting in the production of a daughter cell that is phylogenetically more advanced than the contacted cell.

In an eighth aspect, the invention provides a method for inhibiting cell proliferation in a cell, comprising contacting a cell with at least two of the reagents selected from the group consisting of an antisense oligonucleotide that inhibits a specific histone deacetylase isoform, a histone deacetylase small molecule inhibitor, an antisense oligonucleotide that inhibits a DNA methyltransferase, and a DNA methyltransferase small molecule inhibitor. In one embodiment, the inhibition of cell growth of the contacted cell is greater than the inhibition of cell growth of a cell contacted with only one of the reagents. In certain preferred embodiments, each of the reagents selected from the group is substantially pure. In preferred embodiments, the cell is a neoplastic cell. In yet additional preferred embodiments, the reagents selected from the group are operably associated.

Antisense oligonucleotides that inhibit DNA methyltransferase are described in Szyf and von Hofe, U.S. Patent No. 5,578,716, the entire contents of which are incorporated by reference. DNA methyltransferase small molecule inhibitors include, without limitation, 5-aza-2'-deoxycytidine (5-aza-dC), 5-fluoro-2'-deoxycytidine, 5-aza-cytidine (5-aza-C), or 5,6-dihydro-5-aza-cytidine.

In a ninth aspect, the invention provides a method for modulating cell proliferation or differentiation comprising contacting a cell with an agent of the first aspect of the invention, wherein one or more, but less than all, HDAC isoforms are inhibited, which results in a modulation of proliferation or differentiation. In preferred embodiments, the cell proliferation is neoplasia.

For purposes of this aspect, it is unimportant how the specific HDAC isoform is inhibited. The present invention has provided the discovery that specific individual HDACs are involved in cell proliferation or differentiation, whereas others are not. As demonstrated in this specification, this is true regardless of how the particular HDAC isoform(s) is/are inhibited.

By the term "modulating" proliferation or differentiation is meant altering by increasing or decreasing the relative amount of proliferation or differentiation when compared to a control cell not contacted with an agent of the first aspect of the invention. Preferably, there is an increase or decrease of about 10% to 100%. More preferably, there is an increase or decrease of about 25% to 100%. Most preferably, there is an increase or decrease of about 50% to 100%. The term "about" is used herein to indicate a variance of as much as 20% over or below the stated numerical values.

In certain preferred embodiments, the histone deacetylase isoform is selected from HDAC-1, HDAC-2, HDAC-3, HDAC-4, HDAC-5, HDAC-6, HDAC-7 and HDAC-8. In certain preferred embodiments, the histone deacetylase isoform is HDAC-1.

The following examples are intended to further illustrate certain preferred embodiments of the invention and are not limiting in nature. Those skilled in the art will recognize, or be able to ascertain, using no more than routine experimentation, numerous equivalents to the specific substances and procedures described herein. Such equivalents are considered to be within the scope of this invention, and are covered by the appended claims.

### EXAMPLES

### Example 1

### Synthesis and Identification of Antisense Oligonucleotides

Antisense (AS) and mismatch (MM) oligodeoxynucleotides (oligos) were designed to be directed against the 5'- or 3'-untranslated region (UTR) of the targeted gene. Oligos were synthesized with the phosphorothioate backbone and the 4X4 nucleotides 2'-O-methyl modification on an automated synthesizer and purified by preparative reverse-phase HPLC. All oligos used were 20'base pairs in length.

To identify antisense oligodeoxynucleotide (ODN) capable of inhibiting HDAC-1 expression in human cancer cells, eleven phosphorothioate ODNs containing sequences complementary to the 5' or 3' UTR of the human HDAC-1 gene (GenBank Accession No. U50079) were initially screened in T24 cells at 100 nM. Cells were harvested after 24 hours of treatment, and HDAC-1 RNA expression was analyzed by Northern blot analysis. This screen identified HDAC-1 AS1 and AS2 as ODNs with antisense activity to human HDAC-1. HDAC-1 MM oligo was created as a control; compared to the antisense oligo, it has a 6-base mismatch.

Twenty-four phosphorothioate ODNs containing sequences complementary to the 5' or 3' UTR of the human HDAC-2 gene (GenBank Accession No. U31814) were screened as above. HDAC-2 AS was identified as an ODN with antisense activity to human HDAC-2. HDAC-2 MM was created as a control; compared to the antisense oligo, it contains a 7-base mismatch.

Twenty-one phosphorothioate ODNs containing sequences complementary to the 5' or 3' UTR of the human HDAC-3 gene (GenBank Accession No. AF039703) were screened as above. HDAC-3 AS was identified as an ODN with antisense activity to human HDAC-3. HDAC-3 MM oligonucleotide was created as a control; compared to the antisense oligonucleotide, it contains a 6-base mismatch.

Seventeen phosphorothioate ODNs containing sequences complementary to the 5' or 3' UTR of the human HDAC-4 gene (GenBank Accession No. AB006626) were screened as above. HDAC-4 AS1 and AS2 were identified as ODNs with antisense activity to human HDAC-4. HDAC-4 MM1 and MM2 oligonucleotides were created as controls; compared to the antisense oligonucleotides, they each contain a 6-base mismatch.

Thirteen phosphorothioate ODNs containing sequences complementary to the 5' or 3' untranslated regions of the human HDAC-5 gene (GenBank Accession No. AF039691) were screened as above. HDAC-5 AS was identified as an ODN with antisense activity to human HDAC-5.

Thirteen phosphorothioate ODNs containing sequences complementary to the 5' or 3' untranslated regions of the human HDAC-6 gene (GenBank Accession No. AJ011972) were screened as above. HDAC-6 AS was identified as an ODN with antisense activity to human HDAC-6. HDAC-6 MM oligo was created as a control; compared to the antisense oligo, it contains a 7-base mismatch.

Eighteen phosphorothioate ODNs containing sequences complementary to the 5' or 3' untranslated regions of the human HDAC-7 gene (GenBank Accession No. AF239243) were screened as above. HDAC-7 AS was identified as an ODN with antisense activity to human HDAC-7.

Fourteen phosphorothioate ODNs containing sequences complementary to the 5' or 3' untranslated regions of the human HDAC-8 gene (GenBank Accession No. AF230097) were screened as above. HDAC-8 AS was identified as an ODN with antisense activity to human HDAC-8.

### Example 2

### HDAC AS ODNs Specifically Inhibit Expression at the mRNA Level

In order to determine whether AS ODN treatment reduced HDAC expression at the mRNA level, human A549 cells were treated with 50 nM of antisense (AS) oligonucleotide directed against human HDAC-3 or its corresponding mismatch (MM) oligo for 48 hours, and A549 cells were treated with 50 nM or 100 nM of AS oligonucleotide directed against human HDAC-1, HDAC-2, HDAC-4, HDAC-5, HDAC-6 or HDAC-7 or the appropriate MM oligonucleotide (100 nM) for 24 hours.

Briefly, human A549 and/or T24 human bladder carcinoma cells were seeded in 10 cm tissue culture dishes one day prior to oligonucleotide treatment. The cell lines were obtained from the American Type Culture Collection (ATCC) (Manassas, VA) and were grown under the recommended culture conditions. Before the addition of the oligonucleotides, cells were washed with PBS (phosphate buffered saline). Next, lipofectin transfection reagent (GIBCO BRL Mississauga, Ontario, CA), at a concentration of 6.25 µg/ml, was added to serum free OPTIMEM medium (GIBCO BRL, Rockville, MD), which was then added to the cells. The oligonucleotides to be screened were then added directly to the cells (*i.e*., one oligonucleotide per plate of cells). Mismatched oligonucleotides were used as controls. The same concentration of oligonucleotide (*e.g.,* 50 nM) was used per plate of cells for each oligonucleotide tested.

Cells were harvested, and total RNAs were analyzed by Northern blot analysis. Briefly, total RNA was extracted using RNeasy miniprep columns (QIAGEN). Ten to twenty µg of total RNA was run on a formaldehyde-containing 1% agarose gel with 0.5 M sodium phosphate (pH 7.0) as the buffer system. RNAs were then transferred to nitrocellulose membranes and hybridized with the indicated radiolabeled DNA probes. Autoradiography was performed using conventional procedures.

Figures 9A-9I present results of experiments conducted with HDAC-1 (Figure 9A), HDAC-2 (Figure 9B), HDAC-6 (Figure 9C), HDAC-3 (Figure 9D), HDAC-4 (Figures 9E and 9F), HDAC-5 (Figure 9G), HDAC-7 (Figure 9H), and HDAC-8 (Figure 9I) AS ODNs.

Treatment of cells with the respective HDAC AS ODN significantly inhibits the expression of the targeted HDAC mRNA in human A549 cells.

### Example 3

### HDAC OSDNs Inhibit HDAC Protein Expression

In order to determine whether treatment with HDAC OSDNs would inhibit HDAC protein expression, human A549 cancer cells were treated with 50 nM of paired antisense or its mismatch oligos directed against human HDAC-1, HDAC-2, HDAC-3, HDAC-4 or HDAC-6 for 48 hours. OSDN treatment conditions were as previously described.

Cells were lysed in buffer containing 1% Triton X- 100, 0.5 % sodium deoxycholate, 5 mM EDTA, 25 mM Tris-HCl, pH 7.5, plus protease inhibitors. Total protein was quantified by the protein assay reagent from Bio-Rad (Hercules, CA). 100 ug of total protein was analyzed by SDSPAGE. Next, total protein was transferred onto a PVDF membrane and probed with various HDAC-specific primary antibodies. Rabbit anti-HDAC-1 (H-51), anti-HDAC-2 (H-54) antibodies (Santa Cruz Biotechnologies, Santa Cruz, CA) were used at 1:500 dilution. Rabbit anti-HDAC-3 antibody (Sigma, St. Louis, MO) was used at a dilution of 1:1000. Anti-HDAC-4 antibody was prepared as previously described (Wang, S.H. et al., (1999) Mol. Cell. Biol. 19:7816-27), and was used at a dilution of 1:1000. Anti-HDAC-6 antibody was raised by immunizing rabbits with a GST fusion protein containing a fragment of HDAC-6 protein (amino acid #990 to #1216, GenBank Accession No. AAD29048). Rabbit antiserum was tested and found only to react specifically to the human HDAC-6 isoform. HDAC-6 antiserum was used at 1:500 dilution in Western blots to detect HDAC-6 in total cell lysates. Horse Radish Peroxidase conjugated secondary antibody was used at a dilution of 1:5000 to detect primary antibody binding. The secondary antibody binding was visualized by use of the Enhanced chemiluminescence (ECL) detection kit (Amersham-Pharmacia Biotech., Inc., Piscataway, NJ).

As shown in Figure 10A, the treatment of cells with HDAC-1, HDAC-2, HDAC-3, HDAC-4 or HDAC-6 ODNs for 48 hours specifically inhibits the expression of the respective HDAC isotype protein. Figure 10B presents dose dependent response for the inhibited expression of HDAC-1 protein in cells treated with two HDAC-1 AS ODNs. As predicted, treatment of cells with the respective mismatch (MM) control oligonucleotide does not result in a significant decrease in HDAC-1 protein expression in the treated cells.

In order to demonstrate that the level of HDAC protein expression is an important factor in the cancer cell phenotype, experiments were done to determine the level of HDAC isotype expression in normal and cancer cells. Western blot analysis was performed as described above.

The results are presented in Table 3 clearly demonstrate that HDAC-1, HDAC-2, HDAC-3, HDAC-4, and HDAC-6, isotype proteins are overexpressed in cancer cell lines.

**Table 3**

| **Expression Level of HDAC Isotypes in Human Normal and Cancer Cells** | | | | | | | |
|---|---|---|---|---|---|---|---|
| States of Cell | Tissue Type | Cell Designation | HDAC-1 | HDAC-2 | HDAC-3 | HDAC-4 | HDAC-6 |
| Normal | Breast Epithelial | HMEC | - | + | ++ | + | + |
| Normal | Foreskin Fibroblasts | MRHF | - | + | + | ++ | + |
| Cancer | Bladder | T24 | +++ | ++ | +++ | ++ | +++ |
| Cancer | Lung | A549 | ++ | +++ | +++ | +++ | ++ |
| Cancer | Colon | SW48 | +++ | +++ | +++ | +++ | +++ |
| Cancer | Colon | HCT116 | ++++ | +++ | +++ | ++++ | +++ |
| Cancer | Colon | HT29 | +++ | +++ | +++ | +++ | +++ |
| Cancer | Colon | NCI-H446 | ++ | ++++ | +++ | ++++ | ++ |
| Cancer | Cervix | Hela | +++ | ++++ | +++ | +++ | +++ |
| Cancer | Prostate | DU145 | +++ | +++ | +++ | ++++ | +++ |
| Cancer | Breast | MDA-MB-231 | ++ | +++ | +++ | +++ | ++++ |
| Cancer | Breast | MCF-7 | +++ | +++ | +++ | ++ | ++ |
| Cancer | Breast | T47D | +++ | +++ | +++ | ++ | +++ |
| Cancer | Kidney | 293T | +++ | ++++ | ++++ | ++ | ++ |
| Cancer | Leukemia | K562 | +++ | ++++ | ++++ | ++++ | ++++ |
| Cander | Leukemia | Jurkat T | +++ | ++ | ++++ | ++ | ++ |

| | | | | | | | |
|---|---|---|---|---|---|---|---|
| (-): not detectable; (+): detectable; (++): 2X over (+); (+++): 5X over (+); (++++); 10X over (+) | | | | | | | |

### Example 4

### Effect of HDAC Isotype Specific OSDNs on Cell Growth and Apoptosis

In order to determine the effect of HDAC OSDNs on cell growth and cell death through apoptosis, A549 or T24 cells, MDAmb231 cells, and HMEC cells (ATCC, Manassas, VA) were treated with HDAC OSDNs as previously described.

For the apoptosis study, cells were analyzed using the Cell Death Detection ELISA Plus kit (Roche Diagnostic GmBH, Mannheim, Germany) according to the manufacturer's directions. Typically, 10,000 cells were plated in 96-well tissue culture dishes for 2 hours before harvest and lysis. Each sample was analyzed in duplicate. ELISA reading was done using a MR700 plate reader (DYNEX Technology, Ashford, Middlesex, England) at 410 nm. The reference was set at 490 nm.

For the cell growth analysis, human cancer or normal cells were treated with 50 nM of paired AS or MM oligos directed against human HDAC-1, HDAC-2, HDAC-3, HDAC-4 or HDAC-6 for 72 hours. Cells were harvested and cell numbers counted by trypan blue exclusion using a hemocytometer. Percentage of inhibition was calculated as (100 - AS cell numbers/control cell numbers)%.

Results of the study are shown in Figures 11-13, and in Table 4 and Table 5. Treatment of human cancer cells by HDAC-4 AS, and to a lesser extent, HDAC1 AS, induces growth arrest and apoptosis of various human cancer. The corresponding mismatches have no effect. The effects of HDAC-4 AS or HDAC-1 AS on growth inhibition and apoptosis are significantly reduced in human normal cells. In contrast to the effects of HDAC-4 or HDAC-1 AS oligos, treatment with human HDAC-3 and HDAC-6 OSDNs has no effect on cancer cell growth or apoptosis, and treatment with human HDAC-2 OSDN has a minimal effect on cancer cell growth inhibition. Since T24 cells are p53 null and A549 cells have functional p53 protein, this induction of apoptosis is independent of p53 activity.

**Table 4**

| **Effect of HDAC Isotype-Specific OSDNs on Human Normal and Cancer Cells Growth Inhibition (AS vs. MM)** | | | | |
|---|---|---|---|---|
| | Cancer Cells | Normal Cells | | |
| | A549 | T24 | MDAmb231 | HMEC |
| HDAC-1 AS1 | ++(+) | +(+) | +/- | +/- |
| HDAC-2 AS | +(+) | +/- | - | +/- |
| HDAC-3 AS | - | - | - | - |
| HDAC-4 AS1 | +++ | ++ | ++ | +/- |
| HDAC-6 AS | - | - | +/- | - |

| | | | | |
|---|---|---|---|---|
| "-": no inhibition, "+": <50% inhibition, "++": 50-75% inhibition, "+++": >75% inhibition | | | | |

**Table 5**

| **Effect of HDAC Isotype-Specific OSDNs on Human Normal and Cancer Cells Apoptosis After 48 Hour Treatment** | | | | |
|---|---|---|---|---|
| | A549 | T24 | MDAmb231 | HMEC |
| HDAC-1 AS1 | + | - | | - |
| HDAC-2 AS | - | - | - | - |
| HDAC-3 AS | - | - | - | - |
| HDAC-4 AS1 | +++ | + | ++ | - |
| HDAC-6 AS | - | - | - | - |
| TSA (100ng/ml) | ++ | ++ | ++ | + |

| | | | | |
|---|---|---|---|---|
| "-": < = 2x fold over non-specific background; "+": 2-3X fold; "++": 3-5X fold; "+++": 5-8X fold; "++++": 8X fold | | | | |

### Example 5

### Inhibition of HDAC Isotypes Induces the Expression of Growth Regulatory Genes

In order to understand the mechanism of growth arrest and apoptosis of cancer cells induced by HDAC-1 or HDAC-4 AS treatment, RNase protection assays were used to analyze the mRNA expression of cell growth regulators (p21 and *GADD45*) and proapoptotic gene *Bax*.

Briefly, human cancer A549 or T24 cells were treated with HDAC isotype-specific antisense oligonucleotides (each 50 nM) for 48 hours. Total RNAs were extracted and RNase protection assays were performed to analyzed the mRNA expression level of p21 and *GADD45.* As a control, A549 cells were treated by lipofectin with or without TSA (250 ng/ml) treatment for 16 hours. These RNase protection assays were done according to the following procedure. Total RNA from cells was prepared using "RNeasy miniprep kit" from QIAGEN following the manufacturer's manual. Labeled probes used in the protection assays were synthesized using "hStress-1 multiple-probe template sets" from Pharmingen (San Diego, California, U.S.A.) according to the manufacturer's instructions. Protection procedures were performed using "RPA II™ Ribonuclease Protection Assay Kit" from Ambion, (Austin, Tx) following the manufacturer's instructions. Quantitation of the bands from autoradiograms was done by using Cyclone™ Phosphor System (Packard Instruments Co. Inc., Meriden, CT). The results are shown in Figures 14,15 and Table 6.

**Table 6**

| **Up-Regulation of p21, *GADD45* and *Bax* After Cell Treatment with Human HDAC Isotype-Specific Antisenses** | | | | | | |
|---|---|---|---|---|---|---|
| | A549 | | | T24 | | |
| | p21 | *GADD45* | *Bax* | p21 | GADD45 | *Bax* |
| HDAC-1 | 1.7 | 5.0 | 0.8 | 2.4 | 3.4 | 0.9 |
| HDAC-2 | 1.1 | 1.2 | 1.0 | 1.0 | 1.0 | 0.9 |
| HDAC-3 | 0.7 | 0.9 | 1.0 | 0.9 | 1.0 | 1.0 |
| HDAC-4 | 3.1 | 5.7 | 2.6 | 2.8 | 2.7 | 1.9 |
| HDAC-6 | 1.0 | 1.0 | 1.0 | 1.0 | 0.8 | 1.1 |
| TSA vs lipofectin | 2.8 | 0.6 | 0.8 | | | |

| | | | | | | |
|---|---|---|---|---|---|---|
| Values indicate the fold induction of transcription as measured by RNase protection analysis for the respective AS vs. MM HDAC isotype-specific oligos. | | | | | | |

Results of the experiments are presented in Table 6. The inhibition of HDAC-4 in both A549 and T24 cancer cells dramatically up-regulates both p21 and *GADD45* expression. Inhibition of HDAC-1 by antisense oligonucleotides induces p21 expression but more greatly induces *GADD45* expression. Inhibition of HDAC-4, upregulates *Bax* expression in both A549 and T24 cells. The effect of HDAC-4 AS treatment (50 nM, 48 hrs) on p21 induction in A549 cells is comparable to that of TSA (0.3 to 0.8 uM, 16 hrs).

Experiments were also conducted to examine the affect of HDAC antisense oligonucleotides on HDAC protein expression. In A549 cells, treatment with HDAC-4 antisene oligonucleotides results in a dramatic increase in the level of p21 protein (Figure 15).

### Example 6

### Cyclin Gene Expression Is Repressed by HDAC-1 AS Treatment

Human cancer A549 cells were treated with AS1, AS2 or MM oligo directed human HDAC1 for 48 hours. Total cell lysates were harvested and analyzed by Western blot using antibodies against human HDAC1, cyclin B1, cyclin A and actin (all from Santa Cruz Biotechnology, Inc., Santa Cruz, California). AS1 or AS2 both repress expression of cyclin B1 and A. Downregulation of cyclin A and B1 expression by AS1 and AS2 correlates well with their ability to inhibit cancer cell growth. (Figure 16)

### Example 7

### Inhibition of Growth in Soft Agar

1.3 g granulated agar (DIDFCO) was added to 100 ml deionized water and boiled in a microwave to sterilize. The boiled agar was held at 55•C until further use. Iscove's Modified Dulbecco's Medium (GIBCO/BRL), 100x Penicillin-Streptomycin-Glutamine (GIBCO/BRL) and fetal bovine serum (medicorp) were pre-warmed at 37•C. To 50 ml sterile tubes was added 9 ml Isove's medium, 2 ml fetal bovine serum and 0.2 ml 100x Pen-Strep-Gln. Then 9 ml 55•C 1.3% agar was added to each tube. The tube contents were mixed immediately, avoiding air bubbles, and 2.5 ml of the mixture was poured into each sterile 6 cm petri dish to form a polymerized bottom layer. Dishes with polymerized bottom layers were then put in a CO2 incubator at 37•C until further use. In 50 ml sterile tubes were prewarmed at 37•C for each 4 cell lines/samples, 20 ml Iscove's medium, 0.4 ml 100x Pen-Strp-Gln and 8 ml fetal bovine serum. Cells were trypsinized and counted by trypan blue staining and 20,000 cells were aliquotted into a sterile 15 ml tube. To the tube was then added DMEM with low glucose (GIBCO/BRL) + 10% fetal bovine serum + Pen-Strep-Gln to a final volume of 1 ml. To the prewarmed 37•C mix in the 50 ml tube was quickly added 8 ml 55•C 1.3% agar, which was then mixed well. Nine ml of this mixture was then aliquotted to each ml cells in the 15 ml tube which is then mixed and 5 ml aliquotted onto the ploymerized bottom layer of the 6 cm culture plates and allowed to polymerize at room temperature. After polymerization, 2.5 ml bottom layer mix was gently added over the cell layer. Plates were wrapped up in foil paper and incubated in a CO2 incubator at 37°•C for three weeks, at which time colonies in agar are counted. The results are shown in Figure 17.

These results demonstrate that an antisense oligonucleotide complementary to HDAC-1 inhibits growth of A549 cells in soft agar, but antisense oligonucleotides complementary to HDAC-2 or HDAC-6, or mismatch controls, do not.

### Example 8

### Inhibition of HDAC Isotypes by Small Molecules

In order to demonstrate the identification of HDAC small molecule inhibitors, HDAC small molecule inhibitors were screened in histone deacetylase enzyme assays using various human histone deacetylase isotypic enzymes (*i.e.,* HDAC-1, HDAC-3, HDAC-4 and HDAC-6). Cloned recombinant human HDAC-1, HDAC-3 and HDAC-6 enzymes, which were tagged with the Flag epitope (Grozinger, C.M., et al., Proc. Natl. Acad. Sci. U.S.A.96:4868-4873 (1999)) in their C-termini, were produced by a baculovirus expression system in insect cells.

Flag-tagged human HDAC-4 enzyme was produced in human embronic kidney 293 cells after transformation by the calcium phosphate precipitation method. Briefly, 293 cells were cultured in Dulbecco's Modified Eagle Medium (DMEM) containing 10% fetal bovine serum and antibiotics. Plasmid DNA encoding Flag-tagged human HDAC-4 was precipitated by ethanol and resuspend in sterile water. DNA-calcium precipitates, formed by mixing DNA, calcium choloride and 2XHEPES-buffered saline solution, were left on 293 cells for 12-16 hours. Cells were return to serum-contained DMEM medium and harvested at 48 hour post transfection for purification of Flag-tagged HDAC-4 enzyme.

HDAC-1 and HDAC-6 were purified on a Q-Sepharose column, followed by an anti-Flag epitope affinity column. The other HDAC isotypes, HDAC-3 and HDAC-4, were purified directly on an anti-Flag affinity column.

For the deacetylase assay, 20,000 cpm of an [³H]-metabalically-labeled acetylated histone was used as a substrate. Histones were incubated with cloned recombinant human HDAC enzymes at 37°C. For the HDAC-1 asasy, the incubation time was 10 minutes, and for the HDAC-3, HDAC-4 and HDAC-6 assays, the incubation time was 2 hours. All assay conditions were pre-determined to be certain that each reaction was linear. Reactions were stopped by adding acetic acid (0.04 M, final concentration) and HCl (250 mM, final concentration). The mixture was extracted with ethyl acetate, and the released [³H]-acetic acid was quantified by liquid scintillation counting. For the inhibition studies, HDAC enzyme was preincubated with test compounds for 30 minutes at 4°C prior to the start of the enzymatic assay. IC₅₀ values for HDAC enzyme inhibitors were identified with dose response curves for each individual compound and, thereby, obtaining a value for the concentration of inhibitor that produced fifty percent of the maximal inhibition.

### Example 9

### Inhibition of HDAC Activity in Whole Cells by Small Molecules

T24 human bladder cancer cells (ATCC, Manassas, VA) growing in culture were incubated with test compounds for 16 hours. Histones were extracted from the cells by standard procedures (see *e.g*. Yoshida *et al., supra*) after the culture period. Twenty µg total core histone protein was loaded onto SDS/PAGE and transferred to nitrocellulose membranes, which were then reacted with polyclonal antibody specific for acetylated histone H-4 (Upstate Biotech Inc., Lake Placid, NY). Horse Radish Peroxidase conjugated secondary antibody was used at a dilution of 1:5000 to detect primary antibody binding. The secondary antibody binding was visualized by use of the Enhanced chemiluminescence (ECL) detection kit (Amersham-Pharmacia Biotech., Inc., Piscataway, NJ]. After exposure to film, acetylated H-4 signal was quantitated by densitometry.

The results, shown in Table 2 above, demonstrate that small molecule inhibitors selective for HDAC-1 and/or HDAC-4 can inhibit histone deacetylation in whole cells.

### Example 10

### Inhibition of Cancer Cell Growth by HDAC Small Molecule Inhibitors

Two thousand (2,000) human colon cancer HCT116 cells (ATCC, Manassas, VA) were used in an MTT (3-[4,5-dimethylthiazol-2-yl]-2,5 diphenyl tetrazolium bromide) assay to quantitatively determine cell proliferation and cytotoxicity. Typically, HCT116 cells were plated into each well of the 96-well tissue culture plate and left overnight to attach to the plate. Compounds at various concentrations were added into the culture media (final DMSO concentration 1%) and incubated for 72 hours. MTT solution (obtained from Sigma as powder) was added and incubated with the cells for 4 hours at 37°C in incubator with 5% CO₂, During the incubation, viable cells convert MTT to a water-insoluble formazan dye. Solubilizing buffer (50% N,N-dimethylformamide, 20% SDS, pH 4.7) was added to cells and incubated for overnight at 37C in incubator with 5% CO₂. Solubilized dye was quantitated by colorimetric reading at 570 nM using a reference of 630 nM. Optical density values were converted to cell number values by comparison to a standard growth curve for each cell line. The concentration test compound that reduces the total cell number to 50% that of the control treatment, *i.e.,* 1% DMSO, is taken as the EC₅₀ value.

The results, shown in Table 2 above, demonstrate that small molecule inhibitors selective for HDAC-1 and/or HDAC-4 can affect cell proliferation.

### Example 11

### Inhibition by Small Molecules of Tumor Growth in a Mouse Model

Female BALB/c nude mice were obtained from Charles River Laboratories (Charles River, NY) and used at age 8-10 weeks. Human prostate tumor cells (DU145,2 x 10⁶) or human colon cancer cells (HCT116; 2 x 10⁶) or small lung core A549 2 x 10⁶ were injected subcutaneously in the animal's flank and allowed to form solid tumors. Tumor fragments were serially passaged a minimum of three times, then approximately 30 mg tumor fragments were implanted subcutaneously through a small surgical incision under general anaesthesia. Small molecule inhibitor administration by intraperotineal or oral administration was initiated when the tumors reached a volume of 100 mm³. For intraperotineal administration, small molecule inhibitors of HDAC (40-50 mg/kg body weight/day) were dissolved in 100% DMSO and administered daily intraperitoneally by injection. For oral administration, small molecule inhibitors of HDAC (40-50 mg/kg body weight/days) were dissolved in a solution containing 65% polyethylene glycol 400 (PEG 400 (Sigma-Aldridge, Mississauga, Ontario, CA, Catalogue No. P-3265), 5% ethanol, and 30% water. Tumor volumes were monitored twice weekly up to 20 days. Each experimental group contained at least 6-8 animals. Percentage inhibition was calculated using volume of tumor from vehicle-treated mice as controls.

The results, shown in Table 2 above, demonstrate that small molecule inhibitors selective for HDAC-1 and/or HDAC-4 can inhibit the growth of tumor cells *in vivo.*

### Example 12

### Upregulation of p21 Expression and Down regulation of Cyclin Gene Expression Following Treatment with Small Molecule Inhibitor

Sulfonamide aniline (compound 3, Table 2) is a small molecule HDAC1 specific inhibitor. Human HCT116 cells were treated with escalating doses of compound 3 for 16 hours. Total cell lysates were harvested and expression of p21 ^{WAF1}, cyclin B1, cyclin A and actin was analyzed by Western blot. Ariti-p21 ^{WAF1} antibody was purchased from BD Transduction Laboratories (BD Pharmingen Canada, Missasagua, Ontario). Compound 3 clearty upregulates expression of p21 ^{WAF1} and represses the expression of cyclin A and B1. The expression profile of these cell cycle regulators correlates well with the ability of compound 3 to inhibit HCT116 proliferation in MTT assays (see Table 2),

### Example 13

### Cell Cycle Arrest Induced by HDAC Small Molecule Inhibtiors

Human cancer HCT116 cells were plated at 2X10⁵ per 10-cm dish and were left to attach to the dish overnight in the incubator. Cells were treated with small molecule inhibitors at various concentrations (1 uM and 10 uM, typically, dissolved in DMSO) for 16 hours. Cells were harvested by trypsinization and washed once in 1X PBS (phosphate buffered saline). The cells were resuspended in about 200ul 1X PBS and were fixed by slowly adding 1 ml 70% ethanol at -20° C and were left at least overnight at -20° C. Fixed cells were centrifuged at low speed (1,000 rpm) for 5 minutes, and the cell pellets were washed again with 1X PBS. Nucleic acids from fixed cells were incubated in a staining solution (0.1*%* (w/v) glucose in 1X PBS containing 50 ug/ml propidium iodide) (Sigma-Aldridge, Mississauga, Ontario, CA) and RNase A (final 100 units/ml, (Sigma-Aldridge, Mississauga, Ontario, CA) for at least 30 minutes in the dark at 25° C. DNA content was measured by using a fluorescence-activated cell sorter (FACS) machine. Treatment of cells with all HDAC small molecule inhibitors in Table 2 results in a significant accumulation of cancer cell in G2/M phase of the cell cycle and concomitantly reduce the accumulation of cancer cells in S phase of the cell cycle. The ratio of cells in G2/M phase vs. cells in the S phase was determined. The Effective concentration (EC) of a small molecule inhibitor to induce a (G2+M)/S ratio of 2.5 is calculated, as shown in Table 2.

### Example: 14

### Synthesis of Small Molecule Compound No. 2

The following provides a synthesis scheme for small molecule Compound No. 2 from Table 2.

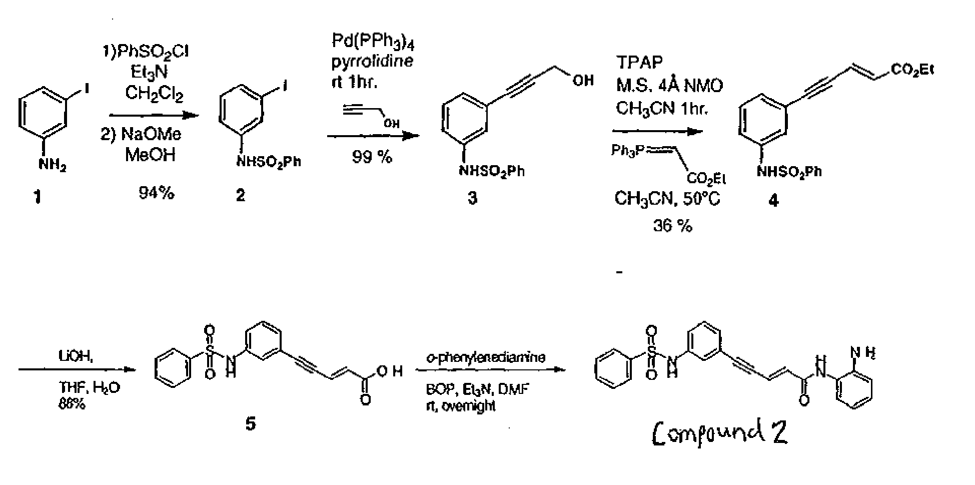

### Step 1: 3-(benzenesulfonylamino)-phenyl iodide (2)

To a solution of 3-iodoaniline (5 g, 22.8 mmol), in CH₂Cl₂ (100 mL), were added at room temperature Et₃N (6.97 mL) followed by benzenesulfonyl chloride (5.84 mL). The mixture was stirred 4 h then a white precipitate was formed. A saturated aqueous solution of NaHCO₃ was added and the phases were separated. The aqueous layer was extracted several times with CH₂Cl₂ and the combined extracts were dried over (MgSO₄) then evaporated. The crude mixture was dissolved in MeOH (100 mL) and NaOMe (6 g), was added and the mixture was heated 1 h at 60°C. The solution became clear with time and HCl (1N) was added. The solvent was evaporated under reduced pressure then the aqueous phase was extracted several times with CH₂Cl₂. The combined organic extracts were dried over (MgSO₄) and evaporated. The crude material was purified by flash chromatography using (100% CH₂Cl₂) as solvent yielding the title compound 21 (7.68g, 94 %) as yellow solid.
¹H NMR: (300 MHz, CDCl₃): δ 7.82-7.78 (m, 2H), 7.60-7.55 (m, 1H), 7.50-7.42 (m, 4H), 7.10-7.06 (m, 1H), 6.96 (t, J = 8Hz, 1H), 6.87 (broad s, 1H).

### Step 2:3-(benzenesulfonylamino)-phenyl-propargylic alcohol (3)

To a solution of 2 (500 mg, 1.39 mmol) in pyrrolidine (5 mL) at room temperature was added Pd(PPh₃)₄ (80 mg, 0.069 mmol), followed by CuI (26 mg, 0.139 mmol). The mixture was stirred until complete dissolution. Propargylic alcohol (162 •L, 2.78 mmol) was added and stirred 6 h at room temperature. Then the solution was treated with a saturated aqueous solution of NH₄Cl and extracted several times with AcOEt. The combined organic extracts were dried over (MgSO₄) then evaporated. The residue was purified by flash chromatography using hexane/AcOEt (1:1) as solvent mixture yielding 3 (395 mg, 99 %) as yellow solid.
¹H NMR: (300 MHz, CDCl₃): δ 7.79-7.76 (m, 2H), 7.55-7.52 (m,1H), 7.45 (t, J = 8Hz, 2H), 7.19-7.15 (m, 3H), 7.07-7.03 (m, 1H), 4.47 (s, 2H).

### Step 3:5-[3-(benzenesulfonylamino)-phenyl]-4-yn-2-pentenoate (4)

To a solution of 3 (2.75 g, 9.58 mmol) in CH₃CN (150 mL) at room temperature were added 4-methylmorpholine N-oxide (NMO, 1.68 g, 14.37 mmol) followed by tetrapropylammonium perruthenate (TPAP, 336 mg, .958 mmol). The mixture was stirred at room temperature 3 h, and then filtrated through a Celite pad with a fritted glass funnel. To the filtrate carbethoxymethylenetriphenyl-phosphorane (6.66 g, 19.16 mmol) was added and the resulting solution was stirred 3 h at room temperature. The solvent was evaporated and the residue was dissolved in CH₂Cl₂ and washed with a saturated aqueous solution of NH₄Cl. The aqueous layer was extracted several times with CH₂Cl₂ then the combined organic extract were dried over (MgSO₄) and evaporated. The crude material was purified by flash chromatography using hexane/AcOEt (1:1) as solvent mixture giving 4 (1.21 g, 36%) as yellow oil.
¹H NMR: (300 MHz, CDCl₃): δ 7.81 (d, J = 8Hz, 2H), 7.56-7.43 (m, 3H), 7.26-7.21 (m, 3H), 7.13-7.11 (m, 1H), 6.93 (d, J = 16 Hz, 1H), 6.29 (d, J = 16Hz, 1H), 4.24 (q, J = 7 Hz, 2H), 1.31 (t, J = 7Hz, 3H).

### Step 4:5-[3-(benzenesulfonylamino)-phenyl]-4-yn-2-pentenic acid (5)

To a solution of 4 (888 mg, 2.50 mmol) in a solvent mixture of THF (10 mL) and water (10 mL) at room temperature was added LiOH (1.04 g, 25.01 mmol). The resulting mixture was heated 2 h at 60°C and treated with HCl (1N) until pH 2. The phases were separated and the aqueous layer was extracted several times with AcOEt. The combined organic extracts were dried over (MgSO₄) then evaporated. The crude residue was purified by flash chromatography using CH₂Cl₂/MeOH (9:1) as solvent mixture yielding 5 (712 mg, 88 %), as white solid.
¹H NMR: (300 MHz, DMSO-*d*₆): δ 7.78-7.76 (m, 2H), 7.75-7.53 (m, 3H), 7.33-7.27 (m, 1H), 7.19-7.16 (m, 3H), 6.89 (d, J = 16 Hz, 1H), 6.33 (d, J = 16 Hz, 1H).

### Step 5: Compound 2

Coupling of 5 with o-phenylenediamine in the presence of benzotriazol-1-yloxytris(dimethylamino)phosphonium hexafluorophosphate (BOP) afforded the anilide Compound 2.
¹H NMR: (300 MHz, DMSO *d*₆): δ 7.77 (broad s, 4H); 7.57 (d, 1H, J=15.7Hz); 7.35 (d, 1H, J=6.9Hz); 7.03-6.94 (m, 6H); 6.76 (d, 1H, J=7.1 Hz); 6.59 (d, 1H, J=6.9Hz); 4.98 (broad s, 2H); 2.19 (s, 3H).
¹³C NMR: (75 MHz, DMSO *d*₆): δ 162.9; 141.6; 139.8; 139.0; 137.6; 134.8; 133.6; 129.6; 128.1; 127.3; 125.9; 125.4; 124.7; 123.2; 120.7; 116.2; 115.9; 20.3.

### Example: 15

### Synthesis of Small Molecule Compound No. 3

The following provides a synthesis scheme for Compound No. 3 from Table 2.

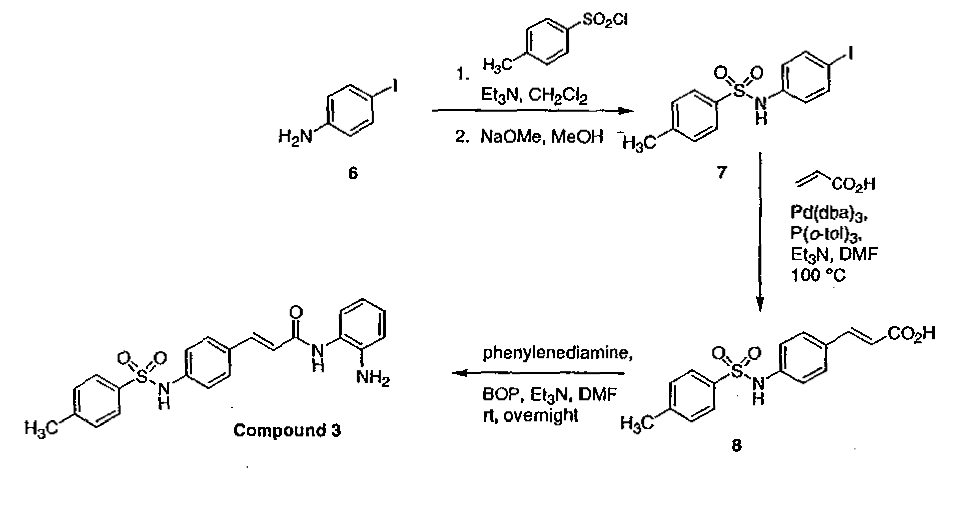

### Step 1: 3-[4-(toluenesulfonylamino)-phenyl]-2-propenoic acid (8)

To a solution of 7 (1.39 mmol), in DMF (10 mL) at room temperature were added tris(dibenzylideneacetone)dipalladium(0) (Pd₂(dba)₃; 1.67 mmol), tri-*o*-tolylphosphine (P(*o*-tol)₃, 0.83 mmol), Et₃N (3.48 mmol) and finally acrylic acid (1.67 mmol). The resulting solution was degassed and purged several times with N₂ then heated overnight at 100 °C. The solution was filtrated through a Celite pad with a fritted glass funnel then the filtrate was evaporated. The residue was purified by flash chromatography using CH₂Cl₂/MeOH (95:5) as solvent mixture yielding the title compound 8.

### Step 2: N-Hydroxy-3-[4-(benzenesulfonylamino)-phenyl]-2-propenamide

### (Compound 3)

The acid 8 was coupled with o-phenylenediamine in the presence of benzotriazol-1-yloxytris(dimethylamino)phosphonium hexafluorophosphate (BOP) to afford the anilide **Compound 3.** ¹H NMR: (300 MHz, DMSO *d*₆): δ 7.77 (broad s, 4H); 7.57 (d, 1H, J=15.7Hz); 7.35 (d, 1H, J=6.9Hz); 7.03-6.94 (m, 6H); 6.76 (d, 1H, J=7.1 Hz); 6.59 (d, 1H, J=6.9Hz); 4.98 (broad s, 2H); 2.19 (s, 3H).
¹³C NMR: (75 MHz, DMSO *d*₆): δ 162.9; 141.6; 139.8; 139.0; 137.6; 134.8; 133.6; 129.6; 128.1; 127.3; 125.9; 125.4; 124.7; 123.2; 120.7; 116.2; 115.9; 20.3.

### Example : 16

### Synthesis of Small Molecule No. Compound 1

The following provides a synthesis scheme for small molecule Compound No. 1 from Table 2.

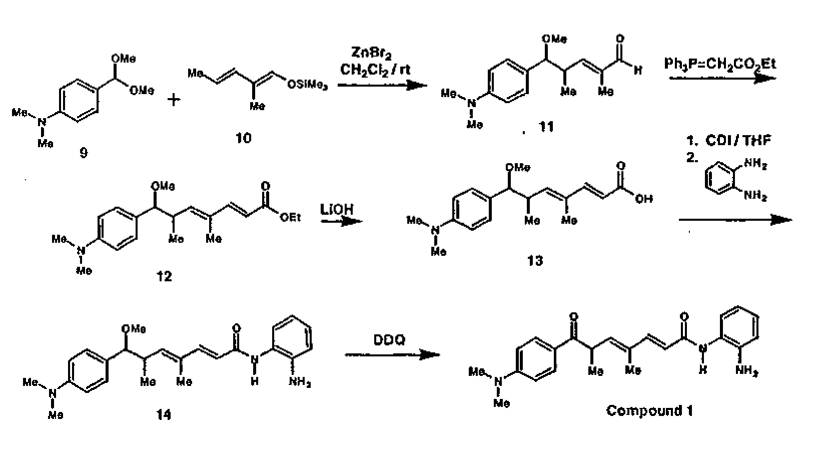

### Step 1: (11)

To a stirred solution of *p*-anisaldehyde dimethyl acetal (9) (10 mmol) in dry CH₂Cl₂ (60 mL) at rt was added 2-methyl-1-trimethylsilyloxypenta-1,3-diene (10) (Tetrahedron, 39: 881 (1983)) (10 mmol) followed by catalytic amount of anhydrous ZnBr₂ (25 mg). After being stirred for 5 h at rt, the reaction was quenched with water (20 mL). The two phases were separated and the aqueous layer was extracted with CH₂Cl₂ (2 x 25 mL). The combined organic layers were washed with brine, dried over magnesium sulfate, filtered, and concentrated under reduced pressure. Purification of the crude product by flash silica gel chromatography (25% ethyl acetate in hexane) afforded the desired aldehyde **11** in 68% yield as a mixture of two isomers in a ca. 2.5:1 patio: major isomer: ¹H NMR (300 MHz, CDCl₃) • 9.29 (s, 1H), 7.08 (d, J = 8.4 Hz, 2H), 6.67 (d, J =8.4 Hz, 2H), 6.29 (dq, J = 9.9, 1.2 Hz, 1H), 3.96 (d, J = 6.6 Hz, 1H), 3.20 (s, 3H), 3.05 (m, 1H), 2.94 (s, 6H), 1.60 (d, J = 0.9 Hz, 3H), 1.12 (d, J = 6.9 Hz, 3H).

### Step 2: (12)

A mixture of aldehyde 11 (5.14 mmol) and ethyl (triphenyl-phosphoranylidene)acetate (2.15 g, 6.16 mmol) in toluene (25 mL) was heated at reflux overnight under N₂. After removal of the solvent under reduced pressure, the crude product obtained was purified by flash silica gel chromatography (10% ethyl acetate in hexane) to give the title compound 12 in 96 % yield as a mixture of two isomers in a ca. 2.5 : 1 ratio: major isomer: ¹H NMR (300 MHz, CDCl₃) δ 7.21 (dd, J = 15.6, 0.9 Hz, 1H), 7.06 (d, J = 8.7 Hz, 2H), 6.66 (d, J = 8.7 Hz, 2H), 5.69 (d, J = 15.6Hz, 1H), 5.67 (br. d, J = 9.0 Hz, 1H), 4.17 (q, J = 7.2 Hz, 2H), 3.87 (d, J = 6.9Hz, 1H), 3.18 (s, 3H), 2.93 (s, 6H), 2.81 (m, 1H), 1.59 (d, J = 1.2 Hz, 3H), 1.27 (t, J = 7.2 Hz, 3H), 1.05 (d, 6.6 Hz, 3H).

### Step 3: (13)

To a stirred solution of diene ester 12 (1.24 mmol) in methanol (10 mL) at rt was added aqueous LiOH 0.5 N solution (1.7mmol). After being stirred at 40 °C for 16 h, methanol was removed under reduced pressure and the resulting aqueous solution was acidified with 3N HCl (pH = ca. 4), extracted with ethyl acetate (25 × 3 mL), dried (MgSO₄), and concentrated under reduced pressure to give the desired carboxylic acid 13 in 98 % yield: major isomer: ¹H NMR (300 MHz, CD₃OD) δ 7.21 (d, J = 15.6, 0.6 Hz, 1H), 7.04 (d, J = 8.7 Hz, 2H), 6.70 (d, J = 8.7 Hz, 2H), 5.61 (d, J = 15.6 Hz, 1H), 5.60 (br. d, J =10.0 Hz, 1H), 3.85 (d, J = 7.5 Hz, 1H), 3.13 (s, 3H), 2.87 (s, 6H), 2.81 (m, 1H), 1.52 (d, J = 1.5 Hz, 3H), 1.06 (d, J = 6.6 Hz, 3H).

### Step 4: (14)

To a solution of carboxylic acid 13 (0.753 mmol) in anhydrous THF (10 mL) was added 1,1'-carbonyldiimidazole (0.790 mmol) at rt, and the mixture was stirred overnight. To the resulting solution was added 1,2-phenylenediamine (5.27 mmol), followed by trifluoroacetic acid (52 µl), and the reaction mixture was stirred for 16 h at rt. The reaction mixture was diluted with ethyl acetate (30 mL), washed with saturated NaHCO₃ solution (5 mL) and then water (10 mL), dried (MgSO₄), and concentrated. Purification by flash silica gel chromatography (50% ethyl acetate in toluene) afforded the title compound 14 in 61% yield, as a mixture of two isomers in a ca.3 : 1 ratio: major isomer: ¹H NMR (300 MHz, CD₃OD) δ 7.28-7.02 (m, 5H), 6.79 (m, 2H), 6.68 (d, J = 8.7 Hz, 2H), 5.83 (d, J = 15.0 Hz, 1H), 5.69 (d, J = 9.6 Hz, 1H), 3.87 (d, J = 6.9 Hz, 1H), 3.19 (s, 3H), 2.94 (s, 6H), 2.80 (m, 1H), 1.61 (br. s, 3H), 1.07 (d, J = 6.6 Hz, 3H).

### Step 5: (Compound 1)

To a stirred solution of compound 14 (0.216 mmol) in wet benzene (2 mL, benzene : H₂O = 9 : 1) at room temperature was added 2,3-dichloro-5,6-dicyano-1,4-benzoquinone (DDQ, 0.432 mmol). After being stirred vigorously for 15 min., the mixture was diluted with ethyl acetate (30 mL), washed with water (2 × 5 mL), dried (anhydr.MgSO₄), and concentrated. Purification by flash silica gel chromatography (50% ethyl acetate in hexanes, and then ethyl acetate only) afforded the title compound 35 (6 mg, 7% yield): ¹H NMR (300 MHz, CDCl₃) δ 7.83 (d, J = 9.0, 2H), 7.87 (br. s, 1H), 7.29 (d, J = 15.6 Hz, 1H), 7.27 (d, 7.8 Hz, 1H), 7.00 (m, 1H), 6.72 (m, 2H), 6.62 (d, J = 9.0 Hz, 2H), 5.97 (d, J = 15.6 Hz, 1H), 5.97 (d, J = 9.3Hz, 1H), 4.34 (dq, J = 9.3, 6.9 Hz, 1H), 3.03 (s, 3H), 1.87 (br. s, 3H), 1.29 (d, J = 6.9 Hz, 3H); ¹³C NMR (75 MHz, CDCl₃)
δ 12.6, 17.6, 39.9, 40.8, 110.7, 118.0, 119.0, 119.3, 123.8, 124.4, 125.1, 126.9, 130.6, 132.5, 140.8, 146.2, 153.4, 164.8, 198.6.

### EQUIVALENTS

Those skilled in the art will recognize, or be able to ascertain, using no more than routine experimentation, many equivalents to the specific embodimemts of the invention described herein. Such equivalents are intended to be encompasssed by the following claims.

## Claims

1. A histone deacetylase small molecule inhibitor that inhibits one or more specific histone deacetylase isoforms, but less than all histone deacetylase isoforms.

2. The inhibitor according to claim 1, wherein the histone deacetylase isoform is selected from the group consisting of HDAC-1, HDAC-2, HDAC-3, HDAC-4, HDAC-5, HDAC-6, HDAC-7 and HDAC-8.

3. The inhibitor according to claim 1, wherein the histone deacetylase isoform is HDAC-1 and/or HDAC-4.

4. Use of the inhibitor of any one of claims 1-3, in the manufacture of a medicament for modulating cell proliferation or differenciation.

5. The use accoding to claim 4, wherein the cell proliferation is neoplasia.

6. Use of the inhibitor of any one of claims 1-3, in the manufacture of a medicament for inhibiting neoplastic cell proliferation in an animal having at least one neoplastic cell present in its body.

7. The use according to claim 6, wherein the animal is a human.

8. The use according to claim 6, further comprising using a therapeutically effective amount of a histone deacetylase small molecule inhibitor with a pharmaceutically acceptable carrier.

9. Use of the inhibitor of any one of claims 1-3, in the manufacture of a medicament for inhibiting one or more histone deacetylase isoforms in a cell.

10. A pharmaceutical composition comprising a histone deacetylase small molecule inhibitor according to any one of claims 1-3 and a pharmaceutically acceptable carrier.
